(19) Europäisches Patentamt
European Patent Office
Office européen des brevets

(11) EP 4 083 217 A1

(12) EUROPEAN PATENT APPLICATION

(43) Date of publication:
**02.11.2022 Bulletin 2022/44**

(21) Application number: **21382363.6**

(22) Date of filing: **26.04.2021**

(51) International Patent Classification (IPC):
***C12N 15/867*** *(2006.01)* ***A61K 48/00*** *(2006.01)*
***A61P 1/16*** *(2006.01)* ***C12N 9/10*** *(2006.01)*

(52) Cooperative Patent Classification (CPC):
**C12N 15/86; A61K 48/005; A61K 48/0058; A61P 1/16; C12N 9/1096; C12Y 206/01044; C12Y 206/01051;** A01K 2217/075; A01K 2227/105; A01K 2267/0306; C12N 2740/16043; C12N 2800/22; C12N 2830/008; C12N 2830/48

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Centro de Investigaciones Energéticas, Medioambientales y Tecnológicas O.A., M.P. (CIEMAT)**
  **28040 Madrid (ES)**
- **Consorcio Centro de Investigación Biomédica en Red, M.P. (CIBER)**
  **28029 Madrid (ES)**
- **Fundacion Instituto De Investigacion Sanitaria Fundacion Jimenez Diaz**
  **28040 Madrid (ES)**

(72) Inventors:
- **SEGOVIA SANZ, José Carlos**
  **28040 Madrid (ES)**
- **GARCÍA BRAVO, María**
  **28040 Madrid (ES)**
- **MOLINOS VICENTE, Andrea**
  **28040 Madrid (ES)**
- **GARCÍA TORRALBA, Aida**
  **28040 Madrid (ES)**
- **NIETO ROMERO, Virginia**
  **28040 Madrid (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

# (54) IN VIVO LENTIVIRAL GENE THERAPY FOR THE TREATMENT OF PRIMARY HYPEROXALURIA TYPE 1

(57) The present invention relates to a lentivirus vector (LV) comprising a nucleic acid, wherein the nucleic acid comprises a transcriptional unit which comprises, from 5' to 3', an hepatocyte-specific promoter, preferably a ET promoter, a nucleotide sequence encoding the optimized Alanine-Glyoxylate and Serine-Pyruvate Aminotransferase protein (AGXT-RHEAM), a woodchuck hepatitis virus posttranscriptional regulatory element (WPRE), preferably a mutated WPRE, and at least one copy of at least one miRNA target sequence, preferably miRNA-142. The lentiviral vector of the present invention further comprises the backbone elements needed for the LV to carry out its function, which are at least one of the following: a 5' long terminal repeat (5' LTR), a primer binding site (PBS), a psi packaging signal, the Stem-loop 4 (SL4) region of wild-type HIV virus, a Rev responsive element (RRE), and a DNA flap central polypurine tract (cPPT), and a 3' long terminal repeat (3' LTR), or any combination thereof.

EP 4 083 217 A1

Figure 12

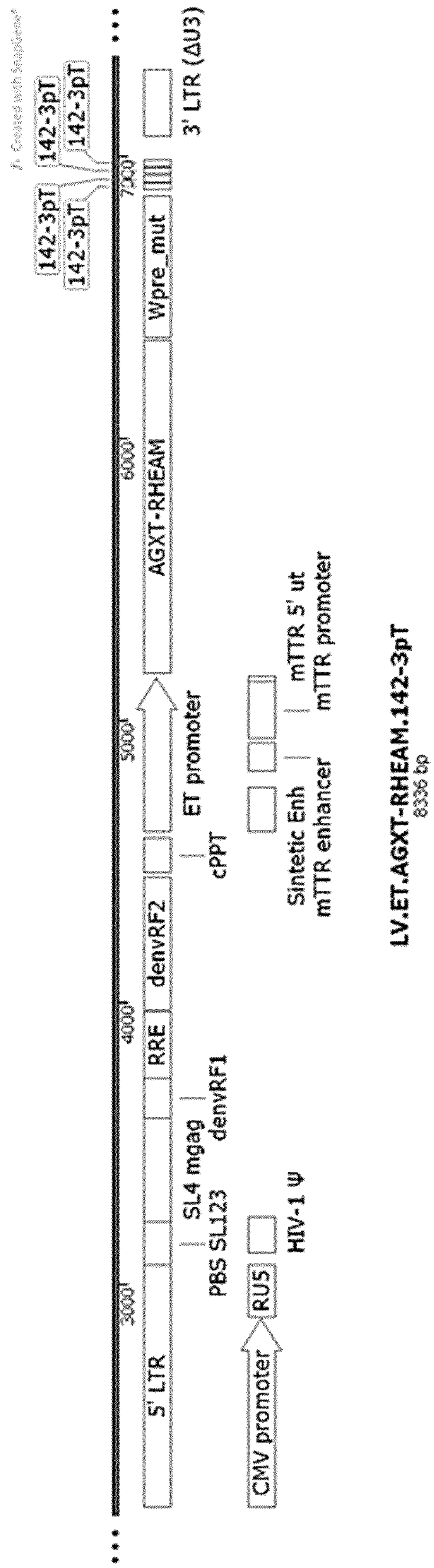
142-3pT
142-3pT
142-3pT
142-3pT
3000
4000
5000
6000
7000
5' LTR
RRE
denvRF2
AGXT-RHEAM
Wpre_mut
3' LTR (ΔU3)
CMV promoter
RU5
PBS SL123
SL4 mgag
denvRF1
HIV-1 Ψ
cPPT
ET promoter
Sintetic Enh
mTTR enhancer
mTTR 5' ut
mTTR promoter
LV.ET.AGXT-RHEAM.142-3pT
8336 bp

## Description

### TECHNICAL FIELD OF THE INVENTION

The present invention can be included in the field of gene therapy, particularly in the treatment of Primary Hyperoxaluria type 1. Specifically, the present invention relates to a lentiviral vector for gene therapy, capable of treating Primary Hyperoxaluria type 1.

### BACKGROUND OF THE INVENTION

Primary Hyperoxaluria Type 1 (PH1) (OMIM#259900) is an inherited rare autosomal recessive metabolic disease. It is caused by a deficiency in the alanine:glyoxylate aminotransferase (AGT; EC 2.6.1.44) enzyme, encoded by the *AGXT* gene. AGT activity is limited to hepatic peroxisomes in humans. This enzyme is only active intracellularly and is not secreted into the extracellular space. PH1 is characterized by an overproduction of oxalate in the liver. Oxalate is an end product of metabolism in mammals that is normally excreted by the urine. When produced in large amounts, the kidney is the first organ affected due to the aggregates of calcium oxalate (CaOx) that are formed in the urinary space (urolithiasis) and renal tissue (nephrocalcinosis), causing interstitial fibrosis and renal failure. As a result of renal damage, the glomerular filtration rate (GFR) decreases and chronic kidney disease rises. Ultimately, around 60% of PH1 patients progress to end-stage renal disease by the age of 40. Subsequent systemic involvement, with widespread CaOx precipitation, called oxalosis, put life at risk for PH1 patients (Cochat et al., 2012; Sas et al., 2020). Current guidelines for patients with PH1 given by the European Consortium of Hyperoxaluria (http://www.oxaleurope.com/) recommend preventive organ transplantation when renal function is already damaged and before systemic oxalosis appears. Thus, there is a need for new therapeutic approaches that, ideally, could be applied as soon as possible, just after the disease is diagnosed. Because of the absence of an accurate test to predict the decline in renal function and the available genetic diagnosis, new treatments should be introduced at an early stage of the disease aiming to reduce oxalate levels and to preserve kidney function.

Gene therapy has the potential to be curative with one-time dosing, by restoring AGT activity through delivery of a functional copy of the *AGXT* gene. Currently, gene therapy for inherited metabolic liver diseases is mostly based on *in vivo* approaches. Liver sinusoidal endothelial cells have a fenestrated structure, facilitating access and gene transfer to hepatocytes (Piccolo & Brunetti-Pierri, 2015). AAV vectors are widely adopted for *in vivo* liver-directed gene therapy, given their remarkable safety and efficacy profile shown in pre-clinical models and clinical trials. AAV-vector based gene transfer to the liver of clotting factors for the treatment of the coagulation disorder hemophilia is among the most successful applications of gene therapy (George et al., 2017; Pasi et al., 2020). In the particular case of PH1, because is an autonomous-cell disease in which the functional cells can not compensate the oxalate overproduction from the deficient ones, the percentage of corrected cells that should be achieved after therapy is thought to be high, in order to revert hyperoxaluric phenotype and correct the disease (Danpure, 2009). Preclinical proof of concept of gene therapy efficacy has been provided using adenoviral or adenoassociated viral vectors in PH1 mice (Salido et al., 2006; Salido et al., 2011). Despite this success, the non-integrating nature of AAV vectors challenges their application to paediatric patients, as the transgene would be diluted during liver growth and the immune response currently hampers effective re-administration. Moreover, the widespread pre-existing immunity against AAV capsid reduces the fraction of eligible patients. Since around 30% of PH1 patients have an infantile onset (Sas et al., 2019; Mandrile et al., 2014) it becomes crucial to develop a gene therapy strategy with the potential to persist life-long following a single administration to paediatric patients.

Lentiviral vectors (LV) are attractive vehicles for liver gene therapy, by virtue of their ability to stably integrate in the genome of target cells and the low prevalence of pre-existing immunity against vector components in humans. In the last years, the use of a LV harbouring FIX for the treatment of Haemophilia B has shown promising results in mice, dogs and non-human primates (Annoni et al., 2013; Cantore et al., 2015; Milani et al., 2019). In the present invention, LV based on the vector used for the treatment of Haemophilia B will be used. These LV are designed to target transgene expression to hepatocytes by a combination of transcriptional (synthetic liver-specific promoter) and post-transcriptional control (microRNAs 142 target sequences, specific for hematopoietic cells, responsible for the degradation of any mRNA from the vector that are ectopically expressed in antigen presenting cells in liver and spleen) (Annoni et al., 2013). In addition, the proposed LV includes an enhanced version of *AGXT* that confers a higher stability to the protein (Mesa-Torres et al., 2014).

### DESCRIPTION OF THE FIGURES

**Figure 1. Lentiviral vector constructs. A.** Reporter lentiviral vector: LV.ET.eGFP.142-3pT. The developed reporter lentiviral vector expresses an eGFP gene which codifies for Green Fluorescence Protein (GFP). Hepatocyte-specific expression of this transgene is achieved due to the presence of two different transcriptional control elements, a specific hepatic promoter (TTR) composed of a synthetic enhancer, a murine TTR enhancer and a part of the murine TTR promoter, and four target sequences of a hematopoietic specific miRNA-142-3 to block the transgene expression in Antigen Presenting Cells (APCs) and, therefore, the development of an immune response. **B**. Therapeutic lentiviral vector: LV.ET.AGXT-RHEAM.142-3pT. It was developed from the same lentiviral vector structure, as the reporter lentiviral vector, and expresses an improved version of the *AGXT* gene, AGXT-RHEAM, with five amino acid changes, which codifies for a more active form of AGT protein As well as in reporter LV, hepatocyte-specific expression of the therapeutic transgene is achieved due to the presence of two different transcriptional control elements, a specific hepatic promoter (TTR) and four repetitions of a target sequence of a hematopoietic specific miRNA-142-3.

**Figure 2. *In vivo* testing protocol of the gene therapy approach efficacy study.** To study the efficacy of the proposed strategy two kinds of experiments were performed. **A**. C57BL/6 adult male mice were intravenously injected with a single dose of reporter lentiviral vector and were sacrificed four weeks later. Afterwards, the number of genome copies of the lentiviral vector integrated, as well as the percentage of transduced cells were analysed in the target tissue, the liver, to determine the transduction efficacy. Moreover, a biodistribution study was performed to determine the security of the procedure, by analysis of vector copy number (VCN) in spleen, lungs, bone marrow, lymph nodes, thymus, brain, testicles, pancreas, and kidneys. B. For the analysis of the *AGXT*-expressing lentiviral vector therapeutic effect in a mouse model of PH1 the following protocol was performed. Adult *Agxt1*$^{-/-}$ male mice were intravenously injected with different doses of the therapeutic lentiviral vector. Moreover, three different control groups were included, two of them composed of *Agxt1*$^{-/-}$ mice non-injected or injected with the reporter lentiviral vector, and the other one composed of non-injected C57BL/6 wild-type mice. Three weeks after the treatment, all mice groups were subjected to an ethylene glycol challenge, in which they were supplemented with 0.5% ethylene glycol in drinking water, to induce overloading in oxalate production. During the challenge, urine oxalate concentration and weight, two different PH1 end-points, were measured. After the treatment, mice were sacrificed, and kidney damage was defined. After the sacrifice, the transduction efficacy of the therapeutic lentiviral vector was analyzed by VCN and *AGXT* expression in liver. In reporter lentiviral vector injected *Agxt1*$^{-/-}$ mice percentage of transduced cells was also analysed to complete the results obtained with the therapeutic lentiviral vector.

**Figure 3. Number of viral genomes integrated into the target tissue of lentiviral vector injected mice.** Integration of lentiviral vector in the liver, the target tissue, was analysed four weeks after the lentiviral vector injection by quantification of VCN by qPCR. Data from experiments performed in C57BL/6 mice as well as in *Agxt1*$^{-/-}$ are represented. Non-injected control mice (n=3) did not show lentiviral vector integration in any mouse strains. Regarding experiments performed in C57BL/6 mice, two different reporter lentiviral vector doses ($1\cdot10^8$ TU/mouse and $2.5\cdot10^8$ TU/mouse) (n=3) were tested. We observed 0.55 (0.34-0.56) lentiviral vector copies per diploid genome in the lower one, whereas in the high-dose 1.03 (0.94-1.56) lentiviral vector copies per diploid genome were obtained. Furthermore, *Agxt1*$^{-/-}$ mice were intravenously injected with two different doses of LV.ET.eGFP.142-3pT resulting in a VCN of 0.11 (0.09-0.49) in the liver of mice injected with $1.4\cdot10^8$ TU/mouse (n=4) and 0.84 (0.73-1.58) in mice injected with $5\cdot10^8$ TU/mouse (n=5); and with three different doses of LV.ET.AGXT-RHEAM.142-3pT ($2.5\cdot10^8$ TU/mouse (n=6); $5\cdot10^8$ TU/mouse (n=10) and $1\cdot10^9$ TU/mouse (n=6)) resulting in a VCN of 0.615 (0.39-1.46), 0.765 (0.45-1.19) and 1.165 (0.85-0.32) respectively.

**Figure 4. *AGXT* expression analysis in the liver of *Agxt1*$^{-/-}$ injected mice after the *in vivo* lentiviral vector treatment. Comparison with physiological mouse Agxt expression in C57BL/6 wild-type mice.** *AGXT* expression in therapeutic lentiviral vector injected *Agxt1*$^{-/-}$ mice was analysed by RT-qPCR. Representation of *AGXT* expression representation was referred to a housekeeping gene. Two different housekeeping genes, with different expression levels were used for this analysis (*msTbp,* low level of expression, and *msActb,* high level of expression) to determine differences between a physiological *Agxt* expression in a wild-type mouse (higher expression level) compared with *AGXT* expression induced by lentiviral vector integration after the *in vivo* gene therapy strategy (lower expression level). **A.** *AGXT* expression referred to *msTbp* expression in the liver of LV.ET.AGXT-RHEAM.142-3pT injected *Agxt1*$^{-/-}$ mice and non-injected wild-type mice. Therapeutic lentiviral injected mice received a dose of $2.5\cdot10^8$ TU/mouse (n=6); $5\cdot10^8$ TU/mouse (n=10) and $1\cdot10^9$ TU/mouse (n=6), respectively, resulting in an *AGXT* expression of 10.88 (5.62-16.79), 12.11 (4.38-14.48) and 19.88 (18.52-28.55), expressed as % of ms*Tbp* expression, which correlates with the received dose. Non-injected *Agxt1*$^{-/-}$ mice (n=8) did not show *AGXT* expression. Moreover, wild-type C57BL/6 mice (n=4) showed an *Agxt* expression of 8563 % of ms*Tbp* expression (6996-8939), significantly higher than the one achieved in *Agxt1*$^{-/-}$ treated mice. B. *AGXT* expression referred to *msActb* expression in the liver of LV.ET.AGXT-RHEAM.142-3pT injected *Agxt1*$^{-/-}$ mice and non-injected wild-type mice. *AGXT* expression, expressed as % of *msActb* housekeeping gene expression, was of 0.1041 (0.06007-0.2452) in *Agxt1*$^{-/-}$ mice which received a therapeutic lentiviral vector dose of $2.5 \cdot 10^8$ TU/mouse (n=6); 0.1857 (0.08878-0.2966) in mice injected with $5 \cdot 10^8$ TU/mouse (n=10) and 0.3088 (0.1707-0.4418) in mice injected with $1 \cdot 10^9$ TU/mouse (n=6). *Agxt* physiological expression referred to *msActb* was 340.5 % (141-374.5). Although the differences in *Agxt* expression levels depending on the housekeeping gene, differences among groups were maintained.

**Figure 5. Percentage of transduced cells in reporter lentiviral vector C57BL/6 and *Agxt1*$^{-/-}$ injected mice.** Percentage of eGFP expressing hepatocytes in liver sections of reporter lentiviral vector injected wild-type C57BL/6 and *Agxt1*$^{-/-}$ mice was defined by immunostaining and following image analysis by QuPath® software. **A-E.** Representative images of immunostaining for eGFP in liver sections of all mice groups. Green: eGFP; blue: nuclei stained with DAPI. **F-H.** Representative images of double immunostaining of eGFP and mouse *Albumin,* a hepatocyte-specific marker, in liver sections of C57BL/6 mice. Red: eGFP: green: mouse *Albumin*; blue: nuclei stained with DAPI. I. Percentage of transduced hepatocytes. Regarding experiments performed in C57BL/6 mice, two different reporter lentiviral vector doses ($1 \cdot 10^8$ TU/mouse (n=3) and $2.5 \cdot 10^8$ TU/mouse (n=3)) were tested. This corresponds to 1.751 (1.132-5.556) % positive hepatocytes and 12.92 (10.72-13.81) % positive hepatocytes respectively. Furthermore, in *Agxt1*$^{-/-}$ two different doses were tested ($1.4 \cdot 10^8$ TU/mouse (n=4) and $5 \cdot 10^8$ TU/mouse (n=5)) resulting in 1.777 (0.399-4.572) % eGFP positive hepatocytes and 5.5059 (2.109-8.754) % eGFP positive hepatocytes respectively.

**Figure 6. Biodistribution analysis.** To determine the safety of the proposed therapeutic strategy, the number of lentiviral vector integrated genomes was analysed in C57BL/6 wild type mice injected with two different doses of the reporter lentiviral vector. The number of LV genome copies integrated into different tissues of injected wild-type C57BL/6 mice was analysed by qPCR. Integration of reporter lentiviral vector genome was only observed in liver, the target tissue, and in lungs, spleen, and bone marrow. C57BL/6 mice injected with $1 \cdot 10^8$ TU/mouse (n=3) showed off-target transduction with a VCN value of 2.72 (2.33-6.2) in lungs, 0.21 (0.14-0.32) in spleen and 0.05 (0.04-0.09) in bone marrow; whereas mice injected with a higher dose of $2.5 \cdot 10^8$ TU/mouse (n=3) showed a VCN value of 4.94 (0.185-7.79) in lungs, 0.35 (0.25-0.55) in spleen and 0.13 (0.09-0.18) in bone marrow.

**Figure 7. PH1 end-points. Oxalate urine concentration during the ethylene glycol challenge.** Quantification of urine oxalate (μmol/24 h) at a basal time point and on days 3 and 7 of ethylene glycol challenge. All represented mice were subjected to a 7-days ethylene glycol challenge in which 0.5% of ethylene glycol was added into the drinking water. Two different negative control groups are represented, one composed of *Agxt1*$^{-/-}$ non-injected mice (n=8) and the other one composed of *Agxt1*$^{-/-}$ mice injected at two different doses of LV.ET.GFP.142-3pT (n=9) ($1.4 \cdot 10^8$ TU/mouse and $5 \cdot 10^8$ TU/mouse). It is also included a positive control group composed of wild-type C57BL/6 non-injected mice (n=5). Therapeutic lentiviral vector injected *Agxt1*$^{-/-}$ mice are divided into three different groups according to the dose received ($2.5 \cdot 10^8$ (n=6), $5 \cdot 10^8$ (n=10) or $10 \cdot 10^8$ (n=6) TU/mouse). Basal data shows a higher oxalate concentration in treated and non-treated *Agxt1*$^{-/-}$ mice compared with wild-type ones. During the ethylene glycol challenge an increase in urine oxalate levels was observed in all groups, however, this increase was higher in the case of untreated *Agxt1*$^{-/-}$ mice (non-injected and LV.ET.eGFP.142-3pT injected Agxt1$^{-/-}$ mice) compared with *Agxt1*$^{-/-}$ treated mice at different doses of LV.ET.AGXT-RHEAM.142-3pT ($2.5 \cdot 10^8$, $5 \cdot 10^8$ or $10 \cdot 10^8$ TU/mouse) and wild-type C57BL/6 mice group. This difference was much higher in the case of *Agxt1*$^{-/-}$ mice which received $5 \cdot 10^8$ TU/mouse of LV.ET.AGXT-RHEAM.142-3pT at day 7 of the ethylene glycol challenge compared with untreated groups. Moreover, at day 7 of ethylene glycol treatment non-significant differences were observed between *Agxt1*$^{-/-}$ treated with $5 \cdot 10^8$ TU/mouse of therapeutic LV and wild-type C57BL/6 mice.

**Figure 8. PH1 end-points: Weight evolution during ethylene glycol challenge.** Weight progress was analysed during the ethylene glycol challenge. Animal weight was measured at a basal time point before the challenge and on days 3 and 7 of the challenge. In this figure, the gain or loss in weight of each animal to the basal weight is represented. Two different negative control groups are represented, one composed of *Agxt1*$^{-/-}$ non-injected mice and the other one composed of *Agxt1*$^{-/-}$ mice injected at two different doses of LV.ET.GFP.142-3pT ($1.4 \cdot 10^8$ TU/mouse and $5 \cdot 10^8$ TU/mouse). It is also included a positive control group composed of wild-type C57BL/6 non-injected mice. Therapeutic lentiviral vector injected *Agxt1*$^{-/-}$ mice are divided into three different groups according to the dose received ($2.5 \cdot 10^8$, $5 \cdot 10^8$ or $10 \cdot 10^8$ TU/mouse). In the case of therapeutic lentiviral vector treated mice, there was not observed a decrease in body weight and an increase was observed on day 7 of the challenge whereas in the case of untreated mice (the non-injected or reporter lentiviral vector injected *Agxt1*$^{-/-}$ mice) they experienced a weight loss during the challenge. Additionally, non-significant differences were observed between wild-type C57BL/6 mice and *Agxt1*$^{-/-}$ mice injected with the therapeutic lentiviral vector at a dose of $5 \cdot 10^8$ TU/mouse or $10 \cdot 10^8$ TU/mouse, meanwhile significant differences were observed among these groups and non-treated mice.

**Figure 9. PH1 end-points. Kidney damage Score. Kidney damage development after ethylene glycol challenge.** To determine kidney damage in treated and non-treated mice subjected to ethylene glycol challenge, kidney histological samples were classified according to the "kidney damage score" as 0: no damage; 1: normal architecture, but the presence of some damage signs such as dilated ducts; 2: architecture partially affected and an increase in the presence of dilated ducts; 3: tissue architecture highly affected and the presence of calcium oxalate crystals can be observed, indicating a nephrocalcinosis stage. **A.** Representative images of haematoxylin-eosin kidney sections of each mice group. From each group, there is a representative image of samples with a kidney damage score of 0-1, which indicates none or mild kidney damage, and a representative image of samples with a kidney damage score of 2-3, which indicates moderate or severe kidney damage. The number of mice that developed that level of kidney damage to the total number of mice in each group is specified in each image. According to the different mice groups, two different negative control groups are represented, one composed of *Agxt1*$^{-/-}$ non-injected mice (n=8) and the other one composed of *Agxt1*$^{-/-}$ mice injected at two different doses of LV.ET.GFP.142-3pT ($1.4\cdot10^8$ TU/mouse and $5\cdot10^8$ TU/mouse) (n=9). It is also included a positive control group composed of wild-type C57BL/6 non-injected mice (n=5). Therapeutic lentiviral vector injected *Agxt1*$^{-/-}$ mice are divided into three different groups according to the dose received ($2.5\cdot10^8$ TU/mouse (n=6), $5\cdot10^8$ TU/mouse (n=10) or $10\cdot10^8$ TU/mouse (n=6)). B. Representation of kidney damage stage of each animal. Grey band represents the development of nephrocalcinosis. Among different treated *Agxt1*$^{-/-}$ mice, none of the mice injected with the middle dose of therapeutic lentiviral vector developed nephrocalcinosis.

**Figure 10. Transduction enhancers: Cyclosporin H increases reporter lentiviral vector transduction in HepG2 cell line.** HepG2 cells were transduced with different administration regimens of LV.ET.eGFP.142-3pT and cyclosporine H (CsH) combinations. Fold increase of the percentage of eGFP positive cells between the different CsH treated conditions compared to reporter lentiviral vector transduced cells without CsH is represented. In the first transduction protocol, HepG2 cells were subjected to different concentrations of CsH (4, 8 or, 16 $\mu$M) during lentiviral vector transduction. Cells were transduced with a lentiviral vector MOI of 0.3. Three technical replicates of each condition were performed. Basal transduction levels were 36.67% and in the different CsH treated samples a transduction percentage of 37.67%, 44.3% and, 51.5% in 4, 8 and, 16 $\mu$M CsH conditions were observed, which means a fold-increase of 1.03, 1.2 and, 1.4 respectively. In addition, another CsH administration regimen was tested. HepG2 cells were pre-treated with CsH for 16 hours before transduction, and CsH was added together with LV.ET.eGFP.142-3pT during lentiviral transduction. The only CsH concentration tested was 16 $\mu$M. In this CsH administration regimen, a fold-increase in eGFP positive cells of 2.35 to the control group was observed. Cells were transduced with a MOI of 0.3 of LV.ET.eGFP.142-3pT and the percentage of transduced cells achieved were 13.2% in the control group and 31.03% in the CsH treated cells. The differences observed in transduction levels at the same MOI are probably due to different viral stocks used.

**Figure 11. LV.ET.eGFP.142-3pT construct.**

**Figure 12. LV.ET.AGXT-RHEAM.142-3pT construct.**

**Figure 13. Comparison of LV.ET.eGFP.142-3pT transduction efficacy *in vitro* and *in vivo.*** For *in vitro* transduction efficacy determination, HepG2 cells were transduced with LV.ET.eGFP.142-3pT at different Multiplicity of Infection (MOI). Three days after transduction, the percentage of eGFP positive cells was determined by flow cytometry. Using MOIs of about 1, we achieved a transduction percentage of 50%, whereas higher MOIs achieved 100% transduction. For *in vivo* transduction efficacy determination, adult C57BL/6 (filled circle) or *Agxt1*$^{-/-}$ mice (open circle) were intravenously injected with LV.ET.eGFP.142-3pT at different doses ($1\cdot10^8$ TU/mouse, $1.4\cdot10^8$ TU/mouse, $2\cdot10^8$ TU/mouse and $5\cdot10^8$ TU/mouse). Corresponding MOIs were calculated only according to the estimated number of hepatocytes in the liver of these mice as previously described (Park *et al.,* 2000; Schmitt *et al.,* 2010). Therefore, the mice were injected with the following MOIs: 0.8, 1.1, 2, and 4. Percentage of transduced hepatocytes was determined 4 weeks later in liver histology samples.

## DESCRIPTION OF THE INVENTION

In a first aspect, the present invention provides a lentivirus vector (LV) comprising a nucleic acid, wherein the nucleic acid comprises a transcriptional unit, wherein the transcriptional unit is defined herein as comprising, from 5' to 3', an hepatocyte-specific promoter, preferably a ET promoter, a nucleotide sequence encoding optimized Alanine-Glyoxylate and Serine-Pyruvate Aminotransferase (hereinafter referred to as AGXT-RHEAM) protein, a woodchuck hepatitis virus posttranscriptional regulatory element (hereinafter referred to as WPRE), preferably a mutated WPRE, and at least one copy of at least one miRNA target sequence; wherein the hepatocyte-specific promoter, the WPRE and the at least one copy of at least one miRNA target sequence are operably linked to and regulate the expression of AGXT-RHEAM.

In a preferred embodiment of the first aspect or any of its embodiments, the lentiviral vector comprises a nucleic acid, wherein the nucleic acid comprises from 5' to 3' the following nucleotides:

a) a 5' long terminal repeat (LTR),
b) a primer binding site (PBS)
c) a psi packaging signal,
d) the Stem-loop 4 (SL4) region of wild-type HIV virus,
e) a Rev responsive element (RRE),
f) a DNA flap central polypurine tract (cPPT),
g) an engineered hepatocyte-specific promoter with at least 95%, preferably 98%, sequence identity over the full length of SEQ ID NO 16,
h) a nucleotide sequence encoding optimized Alanine-Glyoxylate and Serine-Pyruvate Aminotransferase (AGXT-RHEAM) protein with at least 95%, preferably 98%, sequence identity over the full length of SEQ ID NO 29,
i) a mutated optimized woodchuck hepatitis virus posttranscriptional regulatory element (WPRE) with at least 95%, preferably 98%, sequence identity over the full length of SEQ ID NO 21,
j) at least a copy of at least one miRNA target sequence, and
k) a 3' long terminal repeat (LTR),

wherein the long terminal repeat regions of a) and k) have been rendered substantially transcriptionally inactive by virtue of total or partial deletion in the U3 region of the LTR, and wherein g), i) and j) are operably linked to and regulate the expression of h). In a preferred embodiment of the first aspect or any of its embodiments, the sequence identity of g), h), i) to SEQ ID NOs 16, 29, and 21, respectively, is 100%.

In a preferred embodiment of the first aspect or any of its embodiments, the at least a copy of at least one miRNA target sequence of j) consists of four copies of the target sequence of miRNA-142-3 with at least 95%, preferably 98%, most preferably 100%, sequence identity over the full length of SEQ ID NO 22.

In a preferred embodiment of the first aspect or any of its embodiments, the nucleotide comprised in said lentiviral vector further comprises:

l) a DenvRF1 region located between d) and e), and
m) a DenvRF2 region located between e) and f).

In a preferred embodiment of the first aspect or any of its embodiments, the 5' long terminal repeat comprised in the nucleotide of said lentiviral vector has at least 95%, preferably 98%, most preferably 100%, sequence identity over the full length of SEQ ID NO 4.

In a preferred embodiment of the first aspect or any of its embodiments, the primer binding site comprised in the nucleotide of said lentiviral vector has at least 95%, preferably 98%, most preferably 100%, sequence identity over the full length of SEQ ID NO 9.

In a preferred embodiment of the first aspect or any of its embodiments the psi packaging signal comprised in the nucleotide of said lentiviral vector has at least 95%, preferably 98%, most preferably 100%, sequence identity over the full length of SEQ ID NO 10.

In a preferred embodiment of the first aspect or any of its embodiments, the Stem-loop 4 (SL4) comprised in nucleotide of said lentiviral vector has at least 95%, preferably 98%, most preferably 100%, sequence identity over the full length of SEQ ID NO 11.

In a preferred embodiment of the first aspect or any of its embodiments, the Rev responsive element (RRE) comprised in the nucleotide of said lentiviral vector has at least 95%, preferably 98%, most preferably 100%, sequence identity over the full length of SEQ ID NO 13.

In a preferred embodiment of the first aspect or any of its embodiments, the DNA flap central polypurine tract (cPPT) comprised in the nucleotide of said lentiviral vector has at least 95%, preferably 98%, most preferably 100%, sequence identity over the full length of SEQ ID NO 15.

In a preferred embodiment of the first aspect or any of its embodiments, the 3' long terminal repeat comprised in the nucleotide of said lentiviral vector has at least 95%, preferably 98%, most preferably 100%, sequence identity over the full length of SEQ ID NO 23.

In a preferred embodiment of the first aspect or any of its embodiments, the DenvRF1 comprised in the nucleotide of said lentiviral vector has at least 95%, preferably 98%, most preferably 100%, sequence identity over the full length of SEQ ID NO 12.

In a preferred embodiment of the first aspect or any of its embodiments, the DenvRF2 comprised in the nucleotide of said lentiviral vector has at least 95%, preferably 98%, most preferably 100%, sequence identity over the full length of SEQ ID NO 14.

In a preferred embodiment of the first aspect or any of its embodiments, the full-length nucleotide of said lentiviral has at least 95%, preferably 98%, most preferably 100% sequence identity with SEQ ID NO 30 (LV.ET.AGXT-RHEAM.142-3pT).

In a second aspect, the present invention relates to a host cell or a substantially pure population of cells comprising (e.g., transduced) the lentiviral vector as defined in the first aspect or in any of its embodiments. In an embodiment of the second aspect, the host cell or the substantially pure population of cells are hepatic cells.

In a third aspect, the present invention relates to an isolated nucleic acid comprising the nucleotide comprised in the lentiviral vector as defined in the first aspect or any of its embodiments.

In a fourth aspect, the present invention provides a pharmaceutical composition comprising the lentiviral as defined in the first aspect or in any of its embodiments, the host cell or the substantially pure population of cells according to the second aspect or any of its embodiments, and/or the nucleic acid as defined in the third aspect or any of its embodiments and a pharmaceutically acceptable carrier or diluent.

In a fifth aspect, the present invention provides the lentiviral vector according to the first aspect or any of its embodiment, the host cell or the substantially pure population of cells according to the second aspect or any of its embodiments, the isolated nucleic acid according to the third aspect or any of its embodiments, or the pharmaceutical composition of the fourth aspect or any of its embodiments for use as a medicament.

In a sixth aspect, the present invention provides the lentiviral vector according to the first aspect or any of its embodiment, the host cell or the substantially pure population of cells according to the second aspect or any of its embodiments, the isolated nucleic acid according to the third aspect or any of its embodiments, or the pharmaceutical composition of the fourth aspect or any of its embodiments for use in the treatment of Primary Hyperoxaluria, preferably Primary Hyperoxaluria type 1, the use comprising administering to the subject said lentiviral, said host cell or population of cells, or said composition.

## GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" is used herein to mean approximately, roughly, around, or in the regions of. When the term "about" is used in conjunction with a numerical range, it modifies that range by extending the boundaries above and below the numerical values set forth. In general, the term "about" is used herein to modify a numerical value above and below the stated value by a variance of 10 percent, up or down (higher or lower).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element.

A protein or nucleotide that "consists essentially of" a protein or nucleotide is a protein or nucleotide that has considerably the same amino acid or nucleotide sequence as the specified protein or nucleotide.

A protein or nucleotide that has "essentially the same amino acid or nucleotide sequence" as a protein or nucleotide, respectively, typically has more than 90% amino acid or nucleotide identity with this protein or nucleotide. Included in this definition are conservative amino acid substitutions.

The term "isolated" for the purposes of the present invention designates a biological material (cell, polypeptide, polynucleotide, or a fragment, variant, or derivative thereof) that has been removed from its original environment (the environment in which it is naturally present). For example, a polynucleotide present in the natural state in a plant or an animal is not isolated, however the same polynucleotide separated from the adjacent nucleic acids in which it is naturally present, is considered "isolated."

"Nucleic acids," "nucleic acid molecules," "oligonucleotide," and "polynucleotide" are used interchangeably and refer to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester analogs thereof, such as phosphorothioates and thioesters, in either single strandedform, or a double-stranded helix. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules (e.g., restriction fragments), plasmids, supercoiled DNA and chromosomes.

A "coding region" or "coding sequence" is a portion of polynucleotide which consists of codons translatable into amino acids.

The term "downstream" refers to a nucleotide sequence that is located 3' to a reference nucleotide sequence. In certain embodiments, downstream nucleotide sequences relate to sequences that follow the starting point of transcription.

The term "upstream" refers to a nucleotide sequence that is located 5' to a reference nucleotide sequence. In certain embodiments, upstream nucleotide sequences relate to sequences that are located on the 5' side of a coding region or starting point of transcription.

As used herein, the term "gene regulatory region" or "regulatory region" refers to nucleotide sequences located upstream (5' sequences), within, or downstream (3' sequences) of a coding region, and which influence the transcription, RNA processing, stability, or translation of the associated coding region. Regulatory regions can include promoters, translation leader sequences, introns, polyadenylation recognition sequences, RNA processing sites, effector binding sites and stem-loop structures. If a coding region is intended for expression in a eukaryotic cell, a polyadenylation signal and transcription termination sequence will usually be located 3' to the coding sequence.

The term "posttranscriptional regulatory element" refers to a DNA sequence that when transcribed creates a tertiary structure which enhances or inhibits the expression of a protein.

"Transcriptional control sequences" refer to DNA regulatory sequences, such as promoters, enhancers, terminators, and the like, that provide for the expression of a coding sequence in a host cell.

The term "promoter" refers to a DNA sequence to which RNA polymerase can bind to in order to initiate transcription. The sequence may further contain binding sites for various proteins that regulate transcription, such as transcription factors. The promoter sequence may be composed of different promoter fragments (either different or the same fragments) that are localized closely in the DNA sequence and may be separated by linkers or spacers. Such promoters are referred to as chimeric promoters.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term "therapeutically effective amount" refers to an amount of matter which has a therapeutic effect, and which is able to treat PH1.

The terms "individual", "patient" or "subject" are used interchangeably in the present application and are not meant to be limiting in any way. The "individual", "patient" or "subject" can be of any age, sex and physical condition.

As used herein, "pharmaceutically acceptable carrier" or "pharmaceutically acceptable diluent" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulphur containing reducing agents, such as urea, glutathione, thioctic acid, sodium thioglycolate, thioglycerol, [alpha]-monothioglycerol, and sodium thio sulphate; low molecular weight proteins, such as human serum albumin, bovine serum albumin, gelatin, or other immunoglobulins; and hydrophilic polymers, such as polyvinylpyrrolidone.

As used herein, the phrase "subject in need thereof includes subjects, such as mammalian subjects, that would benefit from administration of a nucleic acid molecule, a polypeptide, or vector provided herein, e.g., to improve hemostasis.

As used herein, the term "optimized," with regard to nucleotide sequences, refers to a polynucleotide sequence that encodes a polypeptide, wherein the polynucleotide sequence has been mutated to enhance a property of that polynucleotide sequence.

The term "lentivirus" (LV) refers to a genus of retroviruses that cause chronic and deadly diseases characterized by long incubation periods, in the human and other mammalian species. A "vector" is any vehicle which can be used to artificially carry foreign genetic material into a cell. Thus, a "lentiviral vector" refers to a recombinant lentivirus which carries a nucleic acid into a cell.

By "transcriptional unit" as used herein is meant the nucleotide sequence comprised in the LV vector of the present invention that comprises from 5' to 3' an hepatocyte-specific promoter, preferably the ET promoter, a nucleotide sequence encoding optimized Alanine-Glyoxylate and Serine-Pyruvate Aminotransferase (AGXT-RHEAM) protein, a woodchuck hepatitis virus posttranscriptional regulatory element (WPRE), preferably a mutated WPRE, and at least one copy of at least one miRNA target sequence; wherein the hepatocyte-specific promoter, the WPRE and the at least one copy of at least one miRNA target sequence are operably linked to and regulate the expression of AGXT-RHEAM.

## DESCRIPTION

Although lentiviral vectors represent an attractive tool for gene delivery, the efficiency of transduction is key to ensure that enough cells are genetically corrected in order to obtain the desired therapeutic effect. Unfortunately, it is common to find that the rate of transduction observed *in vitro* does not correspond to the transduction efficiency achieved *in vivo,* and this is especially accentuated when the vectors are not or cannot be delivered directly to the tissue where the genetic correction is desired (for instance, in the case of liver-specific diseases), but they are delivered by other routes, such as parenteral route, where the vector is incorporated into blood stream and is targeted by host's immune response. The authors of the present invention present in Figure 13 the transduction efficiency of the same lentiviral vector used *in vitro* (in HepG2 cells) and *in vivo* (C57BL/6 mice), demonstrating that there is a significant reduction in the percentage of transduced cells when the virus is in an *in vivo* context. Additionally, the transduction efficiency does not only vary from *in vitro*/*in vivo,* or from local delivery versus systemically delivery, but it also varies depending on the type of the lentivirus used and its ability to transduce the cells and to integrate into the host cell genome. Particularly, the ability to transduce the target cell is clearly influenced by the lentiviral backbone (Johnson et al., 2021, Mol Therapy). Often, small changes in the lentiviral backbone are translated into big changes in its transduction efficiency. In view of all of this, it can be asserted that it is difficult to determine whether a specific lentiviral vector comprising a gene of interest (transgene) would efficiently work *in vivo* for a specific disease.

Further, some diseases, such as hemophilia (e.g., hemophilia A), can be treated with lentiviral vectors even though their transduction efficiency *in vivo* does not even reach 10% of cells. This is because, in these cases, an *in vivo* transduction efficacy of as low as 5% can be enough to obtain a therapeutic effect that is sufficient to revert the phenotype of the disease. However, in the particular case of PH1, it is widely accepted that at least 40% of hepatic cells need to be corrected, that is, need to efficiently produce AGXT protein, in order to revert, at least partially, the PH1 phenotype. Castello et al. have previously disclosed that hepatocyte transplantation strategies are unlikely to be successful due to the high number of engrafted hepatocytes needed for correction of PH1, which is highly likely to be beyond the capacity of this procedure (Castello et al. 2015. doi:10.1038/gt.2015.107). Further, Jiang and colleagues also disclose that some authorities estimate that at least 75% of the overproducing mutant hepatocyte mass must be replaced with wild-type hepatocytes to achieve a clinical cure (Jiang et al. 2008. DOI: 10.1097/TP.0b013e31816de49e). However, the authors of the present invention have developed a lentiviral vector that is able to correct the deficiency in the expression of AGXT protein in hepatic cells causing a decrease in the oxalate accumulation and thus partially reverting the PH1 phenotype, as shown in Fig. 7-9. Surprisingly, this effect provided by the lentiviral vector described herein was achieved despite of the fact that, as shown in Fig. 5, said lentiviral vector was only able to transduce about 5% of the cells *in vivo.*

Thus, the present invention meets an important need in the art by providing lentiviral vectors comprising a codon optimized AGXT sequence that, upon transduction into a hepatic cell, increases the intracellular levels of AGXT protein and results in a great therapeutic efficacy to treat PH1. A schematic representation of the nucleic acid sequence comprised in the lentiviral vector provided herein is presented in Fig. 12.

Thus, in a **first aspect,** the present invention provides a lentivirus vector (LV) comprising a nucleic acid, wherein the nucleic acid comprises a transcriptional unit, wherein the transcriptional unit is defined herein as comprising, from 5' to 3', an hepatocyte-specific promoter, preferably a ET promoter, a nucleotide sequence encoding optimized Alanine-Glyoxylate and Serine-Pyruvate Aminotransferase (hereinafter referred to as AGXT-RHEAM) protein, a woodchuck hepatitis virus posttranscriptional regulatory element (hereinafter referred to as WPRE), preferably a mutated WPRE, and at least one copy of at least one miRNA target sequence; wherein the hepatocyte-specific promoter, the WPRE and the at least one copy of at least one miRNA target sequence are operably linked to and regulate the expression of AGXT-RHEAM. A coding sequence and a gene expression control sequence are said to be operably linked when they are linked in such a way as to place the expression or transcription and/or translation of the coding sequence under the influence or control of the gene expression control sequence. For example, the hepatocyte-specific promoter, the WPRE and the at least one copy of at least one miRNA target sequence are operably linked to nucleotide sequence encoding optimized AGXT-RHEAM protein so that the expression levels of AGXT are regulated by the promoter and WPRE.

In an embodiment, the nucleic acid comprised in the LV vector according to the first aspect further comprises one or more lentiviral backbone elements, which are defined as nucleic acids encoding for the necessary proteins for the LV to carry out its function (infect the cell and integrate into the viral genome to persist in the cell) and nucleic acids encoding for the necessary regulatory sequences for the LV to carry out its functions.

Thus, the LV vector of the first aspect comprises a nucleic acid, wherein the nucleic acid comprises 1) a transcriptional unit and 2) one or more LV vector backbone elements. It is noted that all the nucleic acid comprised in the transcriptional unit and in the LV vector backbone can be combined. Each of these two components are characterized in detail below:

1) The transcriptional unit

As defined above, by transcriptional unit is referred herein as to the nucleic acid sequence comprised in a LV vector of the present invention that comprises from 5' to 3' an hepatocyte-specific promoter, a nucleotide sequence encoding optimized AGXT-RHEAM protein, a WPRE element, and at least one copy of at least one miRNA target sequence; wherein the hepatocyte-specific promoter, the WPRE and the at least one copy of at least one miRNA target sequence are operably linked to and regulate the expression of AGXT-RHEAM.

• *Hepatocyte-specific promoter*

The hepatocyte-specific promoter leads the AGXT-RHEAM expression after lentiviral vector genome integration into the target cells.

In some embodiments, the nucleic acid sequence comprised in the lentiviral vector comprises at least one tissue specific promoter, i.e., a promoter that will regulate the expression of the optimized AGXT protein in a particular tissue or cell type. In some embodiments, a tissue specific promoter in the lentiviral vector selectively enhances expression of the optimized AGXT protein in a target liver cell. In some embodiments, the target liver cell is a hepatocyte or a precursor cell thereof. In some embodiments, the isolated nucleic acid molecule comprised in the lentiviral vector is stably integrated into the genome of the target cell or target tissue, for example, in the genome of a hepatocyte.

In some embodiments, the lentiviral vector comprises a hepatocyte-specific promoter for transgene expression. In an embodiment, the nucleotide comprising the hepatocyte-specific promoter is an engineered hepatocyte-specific promoter. In some embodiments, the tissue specific promoter that selectively enhances expression of the optimized AGXT protein in a target liver cell comprises a transthyretin promoter (TTRp). In an embodiment, the hepatocyte-specific promoter is a chimeric promoter. In some embodiments, the chimeric hepatocyte-specific promoter is the ET promoter, which consists of a synthetic enhancer, a transthyretin enhancer (hereinafter referred to as TTR enhancer), a transthyretin promoter (hereinafter referred to as TTRp), and a transthyretin 5' UT (hereinafter referred to as mTTR 5' UT).

In a preferred embodiment, the synthetic enhancer of the ET promoter comprises SEQ ID NO: 17 or a sequence which is at least 98% identical over the full length of SEQ ID NO: 17. Preferably the synthetic enhancer of the ET promoter comprises SEQ ID NO: 17 or a sequence which is at least 75%, 80%, 85%, 89%, 90%, 91%, 92%, 95%, 97%, 98%, 99%, or 100% identical over the full length of SEQ ID NO: 17. More preferably, the synthetic enhancer of the ET promoter is SEQ ID NO: 17 or a sequence which is at least 75%, 80%, 85%, 89%, 90%, 91%, 92%, 95%, 97%, 98%, 99%, or 100% identical over the full length of SEQ ID NO: 17.

In some embodiments, the nucleotide sequence of the synthetic enhancer of the ET promoter comprised in the lentiviral vector comprises, consists, or consists essentially of SEQ ID NO:17.

In a preferred embodiment, the TTR enhancer of the ET promoter is a murine TTR enhancer (mTTR enhancer) and comprises SEQ ID NO: 18 or a sequence which is at least 98% identical over the full length of SEQ ID NO: 18. Preferably the mTTR enhancer of the ET promoter comprises SEQ ID NO: 18 or a sequence which is at least 75%, 80%, 85%, 89%, 90%, 91%, 92%, 95%, 97%, 98%, 99%, or 100% identical over the full length of SEQ ID NO: 18. More preferably, the mTTR enhancer of the ET promoter is SEQ ID NO: 18 or a sequence which is at least 75%, 80%, 85%, 89%, 90%, 91%, 92%, 95%, 97%, 98%, 99%, or 100% identical over the full length of SEQ ID NO: 18.

In some embodiments, the nucleotide sequence of the mTTR enhancer of the ET promoter comprised in the lentiviral vector comprises, consists, or consists essentially of SEQ ID NO:18.

In a preferred embodiment, the transthyretin promoter (TTRp) of the ET promoter is a murine TTRp (mTTRp) and comprises SEQ ID NO: 19 or a sequence which is at least 98% identical over the full length of SEQ ID NO: 19.

Preferably the mTTRp of the ET promoter comprises SEQ ID NO: 19 or a sequence which is at least 75%, 80%, 85%, 89%, 90%, 91%, 92%, 95%, 97%, 98%, 99%, or 100% identical over the full length of SEQ ID NO: 19. More preferably, the mTTRp of the ET promoter is SEQ ID NO: 19 or a sequence which is at least 75%, 80%, 85%, 89%, 90%, 91%, 92%, 95%, 97%, 98%, 99%, or 100% identical over the full length of SEQ ID NO: 19.

In some embodiments, the nucleotide sequence of the mTTRp of the ET promoter comprised in the lentiviral vector comprises, consists, or consists essentially of SEQ ID NO:19.

In a preferred embodiment, the transthyretin 5' UT (TTR 5' UT) of the ET promoter is a murine TTR 5' UT (mTTR 5' UT) and comprises SEQ ID NO: 20 or a sequence which is at least 98% identical over the full length of SEQ ID NO: 20. Preferably the mTTR 5' UT of the ET promoter comprises SEQ ID NO: 20 or a sequence which is at least 75%, 80%, 85%, 89%, 90%, 91%, 92%, 95%, 97%, 98%, 99%, or 100% identical over the full length of SEQ ID NO: 20. More preferably, the mTTR 5' UT of the ET promoter is SEQ ID NO: 20 or a sequence which is at least 75%, 80%, 85%, 89%, 90%, 91%, 92%, 95%, 97%, 98%, 99%, or 100% identical over the full length of SEQ ID NO: 20.

In some embodiments, the nucleotide sequence of the mTTR 5' UT of the ET promoter comprised in the lentiviral vector comprises, consists, or consists essentially of SEQ ID NO:20.

The sequence identity between two sequences can be determined through conventional methods. For example, by using standard alignment logarithms known in the state of the art such as BLAST (Altschul et al., 1990. J Mol Biol. 215(3): 403-10). In a preferred embodiment, the sequence identity between two sequences is determined using BLAST.

In a preferred embodiment, the hepatocyte-specific promoter is the ET promoter which consists of a synthetic enhancer of SEQ ID NO: 17, an mTTR enhancer of SEQ ID NO: 18, a mTTRp of SEQ ID NO: 19, and a mTTR 5' UT of SEQ ID NO: 20, or a sequence which is at least 75%, 80%, 85%, 89%, 90%, 91%, 92%, 95%, 97%, 98%, 99%, or 100% identical over the full length of SEQ ID NO: 17, 18, 19, or 20, respectively.

In a preferred embodiment, the nucleotide sequence of the hepatocyte-specific ET promoter comprises SEQ ID NO: 16 or a sequence, which is at least 98% identical over the full length of SEQ ID NO: 16. Preferably the nucleotide sequence of the hepatocyte-specific ET promoter comprises SEQ ID NO: 16 or a sequence which is at least 75%, 80%, 85%, 89%, 90%, 91%, 92%, 95%, 97%, 98%, 99%, or 100% identical over the full length of SEQ ID NO: 16. More preferably, the nucleotide sequence of the hepatocyte-specific ET promoter is SEQ ID NO: 16 or a sequence which is at least 75%, 80%, 85%, 89%, 90%, 91%, 92%, 95%, 97%, 98%, 99%, or 100% identical over the full length of SEQ ID NO: 16.

In some embodiments, the nucleotide sequence comprised in the lentiviral vector and that is a liver-specific promoter that selectively enhances expression of the optimized AGXT protein in a target liver cell comprises, consists, or consists essentially of ET promoter of SEQ ID NO:16.

• *The nucleotide sequence encoding for the optimized Alanine-Glyoxylate and Serine-Pyruvate Aminotransferase (AGXT-RHEAM) protein.*

Lentiviral vectors provided herein comprise codon optimized polynucleotides encoding the optimized AGXT protein. In one embodiment, the codon optimized polynucleotides AGXT is operably linked to at least one, preferably two, expression control sequence, including a promoter and a posttranscriptional element. In some embodiments, the present invention provides a lentiviral vector comprising an isolated nucleic acid molecule comprising a nucleotide sequence encoding a polypeptide with similar activity to AGXT. In an embodiment, the nucleotide sequence comprised in the lentiviral vector and encoding for AGXT-RHEAM encodes for a signal peptide that locates the newly synthetized protein in the peroxisome of the cell.

In a preferred embodiment, the nucleotide sequence encoding for AGXT-RHEAM comprises SEQ ID NO: 29 or a sequence, which is at least 98% identical over the full length of SEQ ID NO: 29. Preferably the nucleotide sequence encoding for AGXT-RHEAM comprises SEQ ID NO: 29 or a sequence which is at least 75%, 80%, 85%, 89%, 90%, 91%, 92%, 95%, 97%, 98%, 99%, or 100% identical over the full length of SEQ ID NO: 29. More preferably, the nucleotide sequence encoding for AGXT-RHEAM is SEQ ID NO: 29 or a sequence which is at least 75%, 80%, 85%, 89%, 90%, 91%, 92%, 95%, 97%, 98%, 99%, or 100% identical over the full length of SEQ ID NO: 29.

In some embodiments, the nucleotide sequence comprised in the lentiviral vector and encoding for AGXT-RHEAM comprises, consists, or consists essentially of SEQ ID NO:29.

• *WHV (woodchuck hepatitis virus) post-transcriptional regulatory element (WPRE)*

Lentiviral vectors provided herein also comprise at least one posttranscriptional regulatory element operably linked to the nucleotide encoding for the optimized AGXT. In an embodiment, said posttranscriptional regulatory element is the WHV (woodchuck hepatitis virus) post-transcriptional regulatory element (WPRE). In an embodiment, the post-transcriptional regulatory element is mutated to increase the safety of the vector. In a preferred embodiment, the mutated WPRE contains a mutation in gene X frame.

In a preferred embodiment, the nucleotide sequence comprising the mutated WPRE comprises SEQ ID NO: 21 or a sequence which is at least 98% identical over the full length of SEQ ID NO: 21. Preferably the nucleotide sequence comprising the mutated WPRE comprises SEQ ID NO: 21 or a sequence which is at least 75%, 80%, 85%, 89%, 90%, 91%, 92%, 95%, 97%, 98%, 99%, or 100% identical over the full length of SEQ ID NO: 21. More preferably, the nucleotide sequence comprising the mutated WPRE is SEQ ID NO: 21 or a sequence which is at least 75%, 80%, 85%, 89%, 90%, 91%, 92%, 95%, 97%, 98%, 99%, or 100% identical over the full length of SEQ ID NO: 21.
In some embodiments, the nucleotide sequence WPRE comprised in the lentiviral vector comprises, consists, or consists essentially of SEQ ID NO:21.

• *The at least one copy of at least one miRNA target sequence.*

The present invention also provides at least one copy of at least one miRNA target sequence operably linked to the optimized AGXT-RHEAM nucleotide sequence. More than one copy of a miRNA target sequence may be included in the transcription unit to increase the effectiveness of the system. The one or more miRNA target sequences can be the same or different. In an embodiment, the transcription unit comprised in the lentiviral vector comprises 1, 2, 3, 4, 5, 6, 7 or 8 copies of the same or different miRNA target sequence.
In an embodiment, the target sequence is a miRNA-142-3 target. In some embodiments, the lentiviral vector comprises one or more target sequences for miRNA-142-3 to reduce immune response to the transgene product. In one embodiment, the lentiviral vector comprises1, 2, 3, preferably 4, 5, 6, 7 or 8 repetitions or copies of miRNA-142-3 target sequences. In some embodiments, incorporating one or more target sequences for miRNA-142-3 into the transcription unit of the lentivirus of the present invention allows for a desired transgene expression profile and/or a reduced immunogenicity. In particular, incorporating one or more target sequences for miRNA-142-3 may suppress transgene expression in intravascular and extravascular hematopoietic lineages, whereas transgene expression is maintained in non-hematopoietic cells. In certain embodiments, the complementary sequence of hematopoietic-specific microRNAs, such as miRNA-142-3, is incorporated into the 3' untranslated region of a lentiviral vector, making the transgene-encoding transcript susceptible to miRNA-mediated down-regulation.
The target sequence can be fully or partially complementary to the miRNA. The term "fully complementary" means that the target sequence has a nucleic acid sequence which is 100 % complementary to the sequence of the miRNA which recognizes it. In an embodiment, the nucleic acid sequence comprised in the transcription unit comprises four repetitions of the target sequence of miRNA-142-3.
In a preferred embodiment, the nucleotide sequence comprising the miRNA-142-3 comprises SEQ ID NO: 22 or a sequence which is at least 98% identical over the full length of SEQ ID NO: 22. Preferably the nucleotide sequence comprising the miRNA-142-3 comprises SEQ ID NO: 22 or a sequence which is at least 75%, 80%, 85%, 89%, 90%, 91%, 92%, 95%, 97%, 98%, 99%, or 100% identical over the full length of SEQ ID NO: 22. More preferably, the nucleotide sequence comprising the miRNA-142-3 is SEQ ID NO: 22 or a sequence which is at least 75%, 80%, 85%, 89%, 90%, 91%, 92%, 95%, 97%, 98%, 99%, or 100% identical over the full length of SEQ ID NO: 22.
In some embodiments, the nucleotide sequence of miRNA-142-3 comprised in the lentiviral vector comprises, consists, or consists essentially of SEQ ID NO: 22.
In some embodiments, the target sequence is an miR-181 target. In some embodiments, the target sequence is an miR-223 target. In some embodiments, the target sequence is an miR-122 target. In some embodiments, the target sequence is an miR-30b target.
It is to be understood that all the embodiments described above for the different elements of the transcription unit (the hepatocyte-specific promoter, the nucleotide sequence encoding optimized AGXT-RHEAM protein, the WPRE element, and the at least one copy of at least one miRNA target sequence) can be combined with each other. In a preferred embodiment, the transcription unit comprised in the nucleic acid of the lentiviral vector consists of:

a) an engineered hepatocyte-specific promoter with at least 95%, preferably 98%, most preferably 100% sequence identity over the full length of SEQ ID NO 16,
b) a nucleotide sequence encoding optimized Alanine-Glyoxylate and Serine-Pyruvate Aminotransferase (AGXT-RHEAM) protein with at least 95%, preferably 98%, most preferably 100% sequence identity over the full length of SEQ ID NO 29,
c) a mutated optimized woodchuck hepatitis virus posttranscriptional regulatory element (WPRE) with at least 95%, preferably 98%, most preferably 100% sequence identity over the full length of SEQ ID NO 21, and
d) at least a copy of at least one miRNA target sequence,

and wherein a), c) and d) are operably linked to and regulate the expression of b). In a further preferred embodiment, the d) at least a copy of at least one miRNA target sequence consists of four copies of the target sequence of miRNA-142-3, preferably with at least 95%, preferably 98%, most preferably 100% sequence identity over the full length of SEQ ID NO 22.

2) Lentiviral vector backbone elements

As defined above, the lentiviral vector backbone elements are defined as nucleic acids sequences needed for the LV to carry out its function (infect the cell and integrate into the viral genome to persist in the cell).

Lentiviruses include members of the bovine lentivirus group, equine lentivirus group, feline lentivirus group, ovinecaprine lentivirus group, and primate lentivirus group.

In some embodiments, the lentiviral vector provided herein is "third-generation" lentiviral vector. As used herein, the term "third-generation" lentiviral vector refers to a lentiviral packaging system that has the characteristics of a second-generation vector system, and that further lacks a functional tat gene, such as one from which the tat gene has been deleted or inactivated. Typically, the gene encoding rev is provided on a separate expression construct. As used herein, a "second-generation" lentiviral vector system refers to a lentiviral packaging system that lacks functional accessory genes, such as one from which the accessory genes vif, vpr, vpu, and nef have been deleted or inactivated. As used herein, "packaging system" refers to a set of viral constructs comprising genes that encode viral proteins involved in packaging a recombinant virus. Typically, the constructs of the packaging system will ultimately be incorporated into a packaging cell.

In some embodiments, the third-generation lentiviral vector provided herein is a self-inactivating lentiviral vector. In some embodiments, the lentiviral vector is a VSV.G pseudo type lentiviral vector.

In certain embodiments, the lentiviral vector is a vector of a recombinant lentivirus capable of infecting non-dividing cells. In certain embodiments, the lentiviral vector is a vector of a recombinant lentivirus capable of infecting liver cells (e.g., hepatocytes). The lentiviral genome and the proviral DNA typically have the three genes found in retroviruses: gag, pol and env, which are flanked by two long terminal repeat (LTR) sequences. The gag gene encodes the internal structural (matrix, capsid and nucleocapsid) proteins; the pol gene encodes the RNA-directed DNA polymerase (reverse transcriptase), a protease and an integrase; and the env gene encodes viral envelope glycoproteins. The 5' and 3' LTR's serve to promote transcription and polyadenylation of the virion RNA's. The LTR contains all other cis-acting sequences necessary for viral replication. In some embodiments the lentiviruses have additional genes including vif, vpr, tat, rev, vpu, net and vpx (in HIV-I, HIV-2 and/or SIV)

In certain embodiments, the lentiviral vector has the HIV virulence genes env, vif, vpr, vpu and nef deleted without compromising the ability of the vector to transduce non-dividing cells.

Adjacent to the 5' LTR are sequences necessary for reverse transcription of the genome (the tRNA primer binding site) and for efficient encapsidation of viral RNA into particles (the Psi site). The sequences necessary for encapsidation (or packaging of retroviral RNA into infectious virions) may be missing from the viral genome, the cis defect prevents encapsidation of genomic RNA. However, the resulting mutant remains capable of directing the synthesis of all virion proteins.

Thus, as defined above, the lentiviral vector provided herein comprises a nucleic acid, wherein the nucleic acid comprises a transcription unit. Further, in another embodiment, said nucleic acid comprised in the lentiviral vector comprises upstream, that is, at the 5' region of the transcriptional unit as defined above, and in order from 5' to 3', at least one of the following: a) a 5' long terminal repeat (5' LTR), b) a primer binding site (PBS), c) a psi packaging signal, d) the Stem-loop 4 (SL4) region of wild-type HIV virus, e) a Rev responsive element (RRE), and f) a DNA flap central polypurine tract (cPPT) or any combination thereof. In a preferred embodiment, said nucleic acid comprised in the lentiviral vector further comprises upstream, that is, at the 5' region of the transcriptional unit as defined above, l) a DenvRF1 region located between d) and e), and m) a DenvRF2 region located between e) and f), or any combination thereof. Each of these nucleotides are further defined below.

In another embodiment, said nucleic acid comprised in the lentiviral vector further comprises downstream, that is, at the 3' region of the transcriptional unit as defined above, a k) 3' long terminal repeat (LTR). In some embodiments, the lentiviral vector comprises a deletion of the U3 region of the 5' LTR. The deletion of the U3 region of the 5' LTR can be the complete deletion or a partial deletion.

It is to be understood that any combination possible the nucleotides a) to f) and j) to k) is comprised in the present invention and in combination with the transcription unit described in the section above. Each of the nucleotides a) to f) and j) to k) and their preferred embodiments are further defined below.

In a preferred embodiment, the nucleotide sequence comprising the 5' LTR comprises SEQ ID NO: 4 or a sequence which is at least 98% identical over the full length of SEQ ID NO: 4. Preferably the nucleotide sequence comprising the 5' LTR comprises SEQ ID NO: 4 or a sequence which is at least 75%, 80%, 85%, 89%, 90%, 91%, 92%, 95%, 97%, 98%, 99%, or 100% identical over the full length of SEQ ID NO: 4. More preferably, the nucleotide sequence comprising the 5' LTR is SEQ ID NO: 4 or a sequence which is at least 75%, 80%, 85%, 89%, 90%, 91%, 92%, 95%, 97%, 98%, 99%, or 100% identical over the full length of SEQ ID NO: 4.

In some embodiments, the nucleotide sequence of 5' LTR comprised in the lentiviral vector comprises, consists, or consists essentially of SEQ ID NO:4.

In an embodiment, the 5' LTR region comprises a CMV promoter, preferably a chimeric CMV promoter, and/or a RU5 element. In an embodiment, the chimeric CMV promoter comprises or consists of a CMV enhancer and a CMV promoter. In a preferred embodiment, the 5' LTR region comprises or consists of a CMV enhancer, a CMV promoter, a RU5 element, and/or any combination thereof.

In a preferred embodiment, the nucleotide sequence comprising the chimeric CMV promoter comprises SEQ ID NO: 5 or a sequence which is at least 98% identical over the full length of SEQ ID NO: 5. Preferably the nucleotide sequence comprising the Chimeric CMV promoter comprises SEQ ID NO: 5 or a sequence which is at least 75%, 80%, 85%, 89%, 90%, 91%, 92%, 95%, 97%, 98%, 99%, or 100% identical over the full length of SEQ ID NO: 5. More preferably, the nucleotide sequence comprising the chimeric CMV promoter is SEQ ID NO: 5 or a sequence which is at least 75%, 80%, 85%, 89%, 90%, 91%, 92%, 95%, 97%, 98%, 99%, or 100% identical over the full length of SEQ ID NO: 5.

In some embodiments, the nucleotide sequence of chimeric CMV promoter comprised in the lentiviral vector comprises, consists, or consists essentially of SEQ ID NO:5.

In a preferred embodiment, the nucleotide sequence comprising the CMV enhancer comprises SEQ ID NO: 6 or a sequence which is at least 98% identical over the full length of SEQ ID NO: 6. Preferably the nucleotide sequence comprising the CMV enhancer comprises SEQ ID NO: 6 or a sequence which is at least 75%, 80%, 85%, 89%, 90%, 91%, 92%, 95%, 97%, 98%, 99%, or 100% identical over the full length of SEQ ID NO: 6. More preferably, the nucleotide sequence comprising the CMV enhancer is SEQ ID NO: 6 or a sequence which is at least 75%, 80%, 85%, 89%, 90%, 91%, 92%, 95%, 97%, 98%, 99%, or 100% identical over the full length of SEQ ID NO: 6.

In some embodiments, the nucleotide sequence of CMV enhancer comprised in the lentiviral vector comprises, consists, or consists essentially of SEQ ID NO: 6.

In a preferred embodiment, the nucleotide sequence comprising the CMV promoter comprises SEQ ID NO: 7 or a sequence which is at least 98% identical over the full length of SEQ ID NO: 7. Preferably the nucleotide sequence comprising the CMV promoter comprises SEQ ID NO: 7 or a sequence which is at least 75%, 80%, 85%, 89%, 90%, 91%, 92%, 95%, 97%, 98%, 99%, or 100% identical over the full length of SEQ ID NO: 7. More preferably, the nucleotide sequence comprising the CMV promoter is SEQ ID NO: 7 or a sequence which is at least 75%, 80%, 85%, 89%, 90%, 91%, 92%, 95%, 97%, 98%, 99%, or 100% identical over the full length of SEQ ID NO: 7.

In some embodiments, the nucleotide sequence of CMV promoter comprised in the lentiviral vector comprises, consists, or consists essentially of SEQ ID NO:7.

In a preferred embodiment, the nucleotide sequence comprising the RU5 element comprises SEQ ID NO: 8 or a sequence which is at least 98% identical over the full length of SEQ ID NO: 8. Preferably the nucleotide sequence comprising the RU5 element comprises SEQ ID NO: 8 or a sequence which is at least 75%, 80%, 85%, 89%, 90%, 91%, 92%, 95%, 97%, 98%, 99%, or 100% identical over the full length of SEQ ID NO: 8. More preferably, the nucleotide sequence comprising the RU5 element is SEQ ID NO: 8 or a sequence which is at least 75%, 80%, 85%, 89%, 90%, 91%, 92%, 95%, 97%, 98%, 99%, or 100% identical over the full length of SEQ ID NO: 8.

In some embodiments, the nucleotide sequence of RU5 element comprised in the lentiviral vector comprises, consists, or consists essentially of SEQ ID NO: 8.

In an embodiment, adjacent to the 5' LTR are sequences necessary for reverse transcription of the genome, which are the tRNA primer binding site (hereinafter referred to as PBS or PBS SL23).

In a preferred embodiment, the nucleotide sequence comprising the PBS SL23 comprises SEQ ID NO: 9 or a sequence which is at least 98% identical over the full length of SEQ ID NO: 9. Preferably the nucleotide sequence comprising the PBS SL23 comprises SEQ ID NO: 9 or a sequence which is at least 75%, 80%, 85%, 89%, 90%, 91%, 92%, 95%, 97%, 98%, 99%, or 100% identical over the full length of SEQ ID NO: 9. More preferably, the nucleotide sequence comprising the PBS SL23 is SEQ ID NO: 9 or a sequence which is at least 75%, 80%, 85%, 89%, 90%, 91%, 92%, 95%, 97%, 98%, 99%, or 100% identical over the full length of SEQ ID NO: 9.

In some embodiments, the nucleotide sequence of the PBS SL23 comprised in the lentiviral vector comprises, consists, or consists essentially of SEQ ID NO: 9.

In some embodiments, the lentiviral vector provided herein comprises at least a psi ($\Psi$) packaging signal, also called the psi site, for efficient encapsulation of viral RNA into particles.

In a preferred embodiment, the nucleotide sequence comprising the psi packaging signal comprises SEQ ID NO: 10 or a sequence which is at least 98% identical over the full length of SEQ ID NO: 10. Preferably the nucleotide sequence comprising the psi packaging signal comprises SEQ ID NO: 10 or a sequence which is at least 75%, 80%, 85%, 89%, 90%, 91%, 92%, 95%, 97%, 98%, 99%, or 100% identical over the full length of SEQ ID NO: 10. More preferably, the nucleotide sequence comprising the psi packaging signal is SEQ ID NO: 10 or a sequence which is at least 75%, 80%, 85%, 89%, 90%, 91%, 92%, 95%, 97%, 98%, 99%, or 100% identical over the full length of SEQ ID NO: 10.

In some embodiments, the nucleotide sequence of the psi packaging signal comprised in the lentiviral vector comprises, consists, or consists essentially of SEQ ID NO: 10. In an embodiment, the nucleotide of the PSI packaging signal partially overlaps with the PBS SL23 and with the Stem-loop 4 (SL4) region of wild-type HIV virus, which is defined below.

In certain embodiments, the lentiviral vector provided herein further comprises one or more nucleotide sequences comprises a nucleotide sequence encoding for a gag protein, a DenvRE1, a Rev-response element (hereinafter referred to as RRE), a DenVRF2, a central polypurine track (hereinafter referred to as cPPT), and/or any combination thereof.

In some embodiments, the lentiviral vector provided herein comprises at least a Stem-loop 4 (SL4) region of wild-type HIV virus (hereinafter referred to as SL4mgag).

In a preferred embodiment, the nucleotide sequence comprising the SL4mgag comprises SEQ ID NO: 11 or a sequence which is at least 98% identical over the full length of SEQ ID NO: 11. Preferably the nucleotide sequence comprising the SL4mgag comprises SEQ ID NO: 11 or a sequence which is at least 75%, 80%, 85%, 89%, 90%, 91%, 92%, 95%, 97%, 98%, 99%, or 100% identical over the full length of SEQ ID NO: 11. More preferably, the nucleotide sequence comprising the SL4mgag is SEQ ID NO: 11 or a sequence which is at least 75%, 80%, 85%, 89%, 90%, 91%, 92%, 95%, 97%, 98%, 99%, or 100% identical over the full length of SEQ ID NO: 11.

In some embodiments, the nucleotide sequence of the SL4mgag comprised in the lentiviral vector comprises, consists, or consists essentially of SEQ ID NO: 11.

In some embodiments, the lentiviral vector provided herein comprises at least a DenvRF1 element.

In a preferred embodiment, the nucleotide sequence comprising the DenvRF1 comprises SEQ ID NO: 12 or a sequence which is at least 98% identical over the full length of SEQ ID NO: 12. Preferably the nucleotide sequence comprising the DenvRF1 comprises SEQ ID NO: 12 or a sequence which is at least 75%, 80%, 85%, 89%, 90%, 91%, 92%, 95%, 97%, 98%, 99%, or 100% identical over the full length of SEQ ID NO: 12. More preferably, the nucleotide sequence comprising the DenvRF1 is SEQ ID NO: 12 or a sequence which is at least 75%, 80%, 85%, 89%, 90%, 91%, 92%, 95%, 97%, 98%, 99%, or 100% identical over the full length of SEQ ID NO: 12.

In some embodiments, the nucleotide sequence of the DenvRF1 comprised in the lentiviral vector comprises, consists, or consists essentially of SEQ ID NO: 12. In an embodiment, the nucleotide of the DenvRF1 partially overlaps with the nucleotide of the SL4mgag defined above and with the nucleotide of the RRE, which is defined below.

In a preferred embodiment, the nucleotide sequence comprising the RRE comprises SEQ ID NO: 13 or a sequence which is at least 98% identical over the full length of SEQ ID NO: 13. Preferably the nucleotide sequence comprising the RRE comprises SEQ ID NO: 13 or a sequence which is at least 75%, 75%, 80%, 85%, 89%, 90%, 91%, 92%, 95%, 97%, 98%, 99%, or 100% identical over the full length of SEQ ID NO: 13. More preferably, the nucleotide sequence comprising the RRE is SEQ ID NO: 13 or a sequence which is at least 75%, 80%, 85%, 89%, 90%, 91%, 92%, 95%, 97%, 98%, 99%, or 100% identical over the full length of SEQ ID NO: 13.

In some embodiments, the nucleotide sequence of RRE comprised in the lentiviral vector comprises, consists, or consists essentially of SEQ ID NO:13.

In some embodiments, the lentiviral vector provided herein comprises at least a DenvRF2 element.

In a preferred embodiment, the nucleotide sequence comprising the DenvRF2 comprises SEQ ID NO: 14 or a sequence which is at least 98% identical over the full length of SEQ ID NO: 14. Preferably the nucleotide sequence comprising the DenvRF2 comprises SEQ ID NO: 14 or a sequence which is at least 75%, 80%, 85%, 89%, 90%, 91%, 92%, 95%, 97%, 98%, 99%, or 100% identical over the full length of SEQ ID NO: 14. More preferably, the nucleotide sequence comprising the DenvRF2 is SEQ ID NO: 14 or a sequence which is at least 75%, 80%, 85%, 89%, 90%, 91%, 92%, 95%, 97%, 98%, 99%, or 100% identical over the full length of SEQ ID NO: 14.

In some embodiments, the nucleotide sequence of the DenvRF2 comprised in the lentiviral vector comprises, consists, or consists essentially of SEQ ID NO: 14.

In a preferred embodiment, the nucleotide sequence comprising the cPPT comprises SEQ ID NO: 15 or a sequence which is at least 98% identical over the full length of SEQ ID NO: 15. Preferably the nucleotide sequence comprising the cPPT comprises SEQ ID NO: 15 or a sequence which is at least 75%, 80%, 85%, 89%, 90%, 91%, 92%, 95%, 97%, 98%, 99%, or 100% identical over the full length of SEQ ID NO: 15. More preferably, the nucleotide sequence comprising the cPPT is SEQ ID NO: 15 or a sequence which is at least 75%, 80%, 85%, 89%, 90%, 91%, 92%, 95%, 97%, 98%, 99%, or 100% identical over the full length of SEQ ID NO: 15.

In some embodiments, the nucleotide sequence of cPPT comprised in the lentiviral vector comprises, consists, or consists essentially of SEQ ID NO:15.

In some embodiments the lentiviral vector comprises a nucleic acid, wherein the nucleic acid comprises a 3' long terminal repeat (hereinafter referred to as 3' LTR). 3' LTR is involved in mRNAs polyadenylation during lentiviral vector production.

In a preferred embodiment, the 3' LTR comprises a deletion of the U3 region of the 3' LTR. The deletion of the U3 region of the 3' LTR can be the complete deletion or a partial deletion.

In a preferred embodiment, the nucleotide sequence comprising the 3' LTR comprises SEQ ID NO: 23 or a sequence which is at least 98% identical over the full length of SEQ ID NO: 23. Preferably the nucleotide sequence comprising the 3' LTR comprises SEQ ID NO: 23 or a sequence which is at least 75%, 80%, 85%, 89%, 90%, 91%, 92%, 95%, 97%, 98%, 99%, or 100% identical over the full length of SEQ ID NO: 23. More preferably, the nucleotide sequence comprising the 3' LTR is SEQ ID NO: 23 or a sequence which is at least 75%, 80%, 85%, 89%, 90%, 91%, 92%, 95%, 97%, 98%, 99%, or 100% identical over the full length of SEQ ID NO: 23.

In some embodiments, the nucleotide sequence of 3' LTR comprised in the lentiviral vector comprises, consists, or consists essentially of SEQ ID NO: 23.

In a preferred embodiment, the 3' LTR is composed of a truncated U3 element (hereinafter referred to as ΔU3) and the elements R and U5 (hereinafter referred to as RU5) from wild-type HIV-1, being a self-inactivating lentiviral vector.

In a preferred embodiment, the nucleotide sequence comprising the ΔU3 comprises SEQ ID NO: 24 or a sequence which is at least 98% identical over the full length of SEQ ID NO: 24. Preferably the nucleotide sequence comprising the ΔU3 comprises SEQ ID NO: 24 or a sequence which is at least 75%, 80%, 85%, 89%, 90%, 91%, 92%, 95%, 97%, 98%, 99%, or 100% identical over the full length of SEQ ID NO: 24. More preferably, the nucleotide sequence comprising the ΔU3 is SEQ ID NO: 24 or a sequence which is at least 75%, 80%, 85%, 89%, 90%, 91%, 92%, 95%, 97%, 98%, 99%, or 100% identical over the full length of SEQ ID NO: 24.

In some embodiments, the nucleotide sequence of ΔU3 comprised in the lentiviral vector comprises, consists, or consists essentially of SEQ ID NO: 24.

In a preferred embodiment, the nucleotide sequence comprising the RU5 comprises SEQ ID NO: 25 or a sequence which is at least 98% identical over the full length of SEQ ID NO: 25. Preferably the nucleotide sequence comprising the RU5 comprises SEQ ID NO: 25 or a sequence which is at least 75%, 80%, 85%, 89%, 90%, 91%, 92%, 95%, 97%, 98%, 99%, or 100% identical over the full length of SEQ ID NO: 25. More preferably, the nucleotide sequence comprising the RU5 is SEQ ID NO: 25 or a sequence which is at least 75%, 80%, 85%, 89%, 90%, 91%, 92%, 95%, 97%, 98%, 99%, or 100% identical over the full length of SEQ ID NO: 25.

In some embodiments, the nucleotide sequence of RU5 comprised in the lentiviral vector comprises, consists, or consists essentially of SEQ ID NO: 25.

In an embodiment, the RU5 nucleotide is located between the chimeric CMV promoter and the PBS SL123 nucleotide. In an embodiment, the PBS SL123 nucleotide is located between the 5' LTR nucleotide and the Psi packaging signal. In an embodiment, the Psi packaging signal nucleotide is located between the PBS SL123 nucleotide and the Stem-loop 4 (SL4) region of wild-type HIV virus nucleotide. In an embodiment, the DenvRF1 nucleotide is located between the SL4mgag nucleotide and the RRE nucleotide. In an embodiment, the DenvRF2 nucleotide is located between the RRE nucleotide and the cPPT nucleotide. In an embodiment, the cPPT nucleotide is located between the RRE nucleotide and the ET promoter nucleotide. In an embodiment, the at least one copy of at least one miRNA target sequence is located between the mutated WPRE nucleotide and the 3' LTR nucleotide.

In a further preferred embodiment of the first aspect or any of its embodiment, the lentiviral vector comprises a nucleic acid, wherein the nucleic acid comprises from 5' to 3' the following nucleotides:

a) a 5' long terminal repeat (LTR),
b) a primer binding site (PBS)
c) a psi packaging signal,
d) the Stem-loop 4 (SL4) region of wild-type HIV virus
e) a Rev responsive element (RRE),
f) a DNA flap central polypurine tract (cPPT),
g) an engineered hepatocyte-specific promoter with at least 95%, preferably 98%, sequence identity over the full length of SEQ ID NO 16,
h) a nucleotide sequence encoding optimized Alanine-Glyoxylate and Serine-Pyruvate Aminotransferase (AGXT-RHEAM) protein with at least 95%, preferably 98%, sequence identity over the full length of SEQ ID NO 29,
i) a mutated optimized woodchuck hepatitis virus posttranscriptional regulatory element (WPRE) with at least 95%, preferably 98%, sequence identity over the full length of SEQ ID NO 21,
j) at least a copy of at least one miRNA target sequence, and
k) a 3' long terminal repeat (LTR),

wherein the long terminal repeat regions of a) and k) have been rendered substantially transcriptionally inactive by virtue of total or partial deletion in the U3 region of the LTR, and wherein g), i) and j) are operably linked to and regulate the expression of h). In a preferred embodiment, the at least a copy of at least one miRNA target sequence consists of four repetitions of the target sequence of miRNA-142-3.

In some embodiments, the nucleotide comprised in said lentiviral vector further comprises:

l) a DenvRF1 region located between d) the Stem-loop 4 (SL4) region of wild-type HIV virus and e) a Rev responsive element (RRE), and
m) a DenvRF2 region located between e) a Rev responsive element (RRE) and f) a DNA flap central polypurine tract (cPPT).

In a most preferred embodiment of the first aspect or any of its embodiment, the lentiviral vector comprises a nucleic acid, wherein the nucleic acid comprises from 5' to 3' the following nucleotides:

a) a 5' long terminal repeat (LTR) with at least 95%, preferably 98%, more preferably 100%, sequence identity over the full length of SEQ ID NO: 5,
b) a primer binding site (PBS) with at least 95%, preferably 98%, more preferably 100%, sequence identity over the full length of SEQ ID NO: 9,
c) a psi packaging signal with at least 95%, preferably 98%, more preferably 100%, sequence identity over the full length of SEQ ID NO: 10,
d) a Stem-loop 4 (SL4) region of wild-type HIV virus with at least 95%, preferably 98%, more preferably 100%, sequence identity over the full length of SEQ ID NO: 11,
e) a Rev responsive element (RRE) with at least 95%, preferably 98%, more preferably 100%, sequence identity over the full length of SEQ ID NO: 13,
f) a DNA flap central polypurine tract (cPPT) with at least 95%, preferably 98%, more preferably 100%, sequence identity over the full length of SEQ ID NO: 15,
g) an engineered hepatocyte-specific promoter with at least 95%, preferably 98%, more preferably 100%, sequence identity over the full length of SEQ ID NO: 16,
h) a nucleotide sequence encoding optimized Alanine-Glyoxylate and Serine-Pyruvate Aminotransferase (AGXT-RHEAM) protein with at least 95%, preferably 98%, more preferably 100%, sequence identity over the full length of SEQ ID NO: 29,
i) a mutated optimized woodchuck hepatitis virus posttranscriptional regulatory element (WPRE) with at least 95%, preferably 98%, more preferably 100%, sequence identity over the full length of SEQ ID NO: 21,
j) at least one copy of at least one miRNA target sequence, and
k) a 3' long terminal repeat (LTR) with at least 95%, preferably 98%, more preferably 100%, sequence identity over the full length of SEQ ID NO: 3,

wherein the long terminal repeat regions of a) and k) have been rendered substantially transcriptionally inactive by virtue of total or partial deletion in the U3 region of the LTR, and wherein g), i) and j) are operably linked to and regulate the expression of h). In a preferred embodiment, the at least a copy of at least one miRNA target sequence consists of four repetitions of the target sequence of miRNA-142-3 with at least 95%, preferably 98%, more preferably 100%, sequence identity over the full length of SEQ ID NO: 22.

In some embodiments, the nucleotide comprised in said lentiviral vector further comprises:

l) a DenvRF1 region with at least 95%, preferably 98%, more preferably 100%, sequence identity over the full length of SEQ ID NO: 12 and located between d) the Stem-loop 4 (SL4) region of wild-type HIV virus and e) a Rev responsive element (RRE), and
m) a DenvRF2 region with at least 95%, preferably 98%, more preferably 100%, sequence identity over the full length of SEQ ID NO: 14 and located between e) a Rev responsive element (RRE) and f) a DNA flap central polypurine tract (cPPT),

In a further preferred embodiment of the first aspect, the lentiviral vector comprises a nucleic acid, wherein the nucleic acid comprises from 5' to 3' the following nucleotides:

a) a 5' long terminal repeat (LTR),
b) a primer binding site (PBS),
c) a psi packaging signal,
d) a Stem-loop 4 (SL4) region of wild-type HIV virus,
e) a Rev responsive element (RRE),
f) a DNA flap central polypurine tract (cPPT),
g) an engineered hepatocyte-specific promoter, with at least 95%, preferably 98%, more preferably 100%, sequence identity over the full length of SEQ ID NO: 16,
h) a nucleotide sequence encoding optimized Alanine-Glyoxylate and Serine-Pyruvate Aminotransferase (AGXT-RHEAM) protein with at least 95%, preferably 98%, more preferably 100%, sequence identity over the full length of SEQ ID NO: 29,
i) a mutated optimized woodchuck hepatitis virus posttranscriptional regulatory element (WPRE) with at least 95%, preferably 98%, more preferably 100%, sequence identity over the full length of SEQ ID NO: 21,

j) at least one copy of at least one miRNA target sequence,
k) a 3' long terminal repeat (LTR),
l) a DenvRF1 region located between d) and e), and
m) a DenvRF2 region located between e) and f), and

wherein the long terminal repeat regions of a) and k) have been rendered substantially transcriptionally inactive by virtue of total or partial deletion in the U3 region of the LTR, and wherein g), i) and j) are operably linked to and regulate the expression of h). In a preferred embodiment, the at least a copy of at least one miRNA target sequence consists of four repetitions of the target sequence of miRNA-142-3.

In a further preferred embodiment of the first aspect, the lentiviral vector comprises a nucleic acid, wherein the nucleic acid comprises from 5' to 3' the following nucleotides:

a) a 5' long terminal repeat (LTR) with at least 95%, preferably 98%, more preferably 100%, sequence identity over the full length of SEQ ID NO: 5,
b) a primer binding site (PBS) with at least 95%, preferably 98%, more preferably 100%, sequence identity over the full length of SEQ ID NO: 9,
c) a psi packaging signal with at least 95%, preferably 98%, more preferably 100%, sequence identity over the full length of SEQ ID NO: 10,
d) a Stem-loop 4 (SL4) region of wild-type HIV virus with at least 95%, preferably 98%, more preferably 100%, sequence identity over the full length of SEQ ID NO: 11,
e) a Rev responsive element (RRE) with at least 95%, preferably 98%, more preferably 100%, sequence identity over the full length of SEQ ID NO: 13,
f) a DNA flap central polypurine tract (cPPT) with at least 95%, preferably 98%, more preferably 100%, sequence identity over the full length of SEQ ID NO: 15,
g) an engineered hepatocyte-specific promoter with at least 95%, preferably 98%, more preferably 100%, sequence identity over the full length of SEQ ID NO: 16,
h) a nucleotide sequence encoding optimized Alanine-Glyoxylate and Serine-Pyruvate Aminotransferase (AGXT-RHEAM) protein with at least 95%, preferably 98%, more preferably 100%, sequence identity over the full length of SEQ ID NO: 29,
i) a mutated optimized woodchuck hepatitis virus posttranscriptional regulatory element (WPRE) with at least 95%, preferably 98%, more preferably 100%, sequence identity over the full length of SEQ ID NO: 21,
j) at least one copy of at least one miRNA target sequence, and
k) a 3' long terminal repeat (LTR) with at least 95%, preferably 98%, more preferably 100%, sequence identity over the full length of SEQ ID NO: 3,
l) a DenvRF1 region with at least 95%, preferably 98%, more preferably 100%, sequence identity over the full length of SEQ ID NO: 12 and located between d) and e), and
m) a DenvRF2 region with at least 95%, preferably 98%, more preferably 100%, sequence identity over the full length of SEQ ID NO: 14 and located between e) and f), and

wherein the long terminal repeat regions of a) and k) have been rendered substantially transcriptionally inactive by virtue of total or partial deletion in the U3 region of the LTR, and wherein g), i) and j) are operably linked to and regulate the expression of h). In a preferred embodiment, the at least a copy of at least one miRNA target sequence consists of four repetitions of the target sequence of miRNA-142-3 with at least 95%, preferably 98%, more preferably 100%, sequence identity over the full length of SEQ ID NO: 22.

In another embodiment, the lentiviral vector, wherein the lentiviral vector comprises a nucleic acid, wherein the nucleic acid comprises from 5' to 3' the following nucleotides:

a) a 5' long terminal repeat (LTR),
b) a primer binding site (PBS)
c) a psi packaging signal,
d) the Stem-loop 4 (SL4) region of wild-type HIV virus
e) a Rev responsive element (RRE),
f) a DNA flap central polypurine tract (cPPT),
g) an engineered hepatocyte-specific promoter with at least 95%, preferably 98%, sequence identity over the full length of SEQ ID NO 16,
h) a nucleotide sequence encoding optimized Alanine-Glyoxylate and Serine-Pyruvate Aminotransferase (AGXT-RHEAM) protein with at least 95%, preferably 98%, sequence identity over the full length of SEQ ID NO 29,
i) a mutated optimized woodchuck hepatitis virus posttranscriptional regulatory element (WPRE) with at least 95%, preferably 98%, sequence identity over the full length of SEQ ID NO 21,

j) four copies of the target sequence of miRNA-142-3 with at least 95%, preferably 98%, sequence identity over the full length of SEQ ID NO 22, and

k) a 3' long terminal repeat (LTR),

wherein the long terminal repeat regions of a) and k) have been rendered substantially transcriptionally inactive by virtue of total or partial deletion in the U3 region of the LTR, and wherein g), i) and j) are operably linked to and regulate the expression of h).

In some embodiments, the nucleotide comprised in said lentiviral vector further comprises:

l) a DenvRF1 region located between d) the Stem-loop 4 (SL4) region of wild-type HIV virus and e) a Rev responsive element (RRE), and

m) a DenvRF2 region located between e) a Rev responsive element (RRE) and f) a DNA flap central polypurine tract (cPPT).

In a preferred embodiment, the full-length nucleotide comprised in the lentiviral provided herein has at least 75%, 80%, 85%, 89%, 90%, 91%, 92%, 95%, 97%, 98%, 99%, or 100% sequence identity over the full length of SEQ ID NO 30 (LV.ET.AGXT-RHEAM.142-3pT). In another preferred embodiment, the full-length nucleotide comprised in the lentiviral provided comprises, consists, or consists essentially of SEQ ID NO: 30.

In an embodiment, the lipid coat of the viral particle can include membrane bound polypeptides that were previously present on the surface of the host cell.

In some embodiments, the lentiviral vector has a modified expression of one or more polypeptides on its surface that inhibit an immune response to the lentiviral vector following administration to a human subject. In some embodiments, the surface of the lentiviral vector comprises one or more CD47 molecules. CD47 is a "marker of self-protein, which is ubiquitously expressed on human cells. Surface expression of CD47 inhibits macrophage-induced phagocytosis of endogenous cells through the interaction of CD47 and macrophage expressed-SIRPa. Cells expressing high levels of CD47 are less likely to be targeted and destroyed by human macrophages in vivo. In some embodiments, the lentiviral vector comprises a high concentration of CD47 polypeptide molecules on its surface. In some embodiments, the lentiviral vector is produced in a cell line that has a high expression level of CD47. In certain embodiments, the lentiviral vector is produced in a cell line, wherein the cell line has high expression of CD47 on the cell membrane. In particular embodiments, the lentiviral vector is produced in a CD47high HEK 293T cell, wherein the HEK 293T is has high expression of CD47 on the cell membrane. In some embodiments, the HEK 293T cell is modified to have increased expression of CD47 relative to unmodified HEK 293T cells. In certain embodiments, the CD47 is human CD47. In an embodiment, the surface of the lentiviral vector lacks expression or has a reduced expression of one or more major histocompatibility complex of class I (MHCI). In certain embodiment, the MHCI is human MHCI.

In a **second aspect**, the present invention relates to a host cell (e.g., a hepatocyte) or a substantially pure population of cells comprising (e.g., transduced) the lentiviral vector as defined in the first aspect or in any of its embodiments. In an embodiment of the second aspect, the host cell or the substantially pure population of cells are hepatic cells.

In a **third aspect**, the present invention relates to an isolated nucleic acid comprising the nucleotide comprised in the lentiviral vector as defined in the first aspect or any of its embodiments.

Pharmaceutical compositions

In a **fourth aspect**, the present invention provides a pharmaceutical composition comprising the lentiviral as defined in the first aspect or in any of its embodiments, the host cell or the substantially pure population of cells according to the second aspect or any of its embodiments, and/or the nucleic acid as defined in the third aspect or any of its embodiments and a pharmaceutically acceptable carrier or diluent.

In some embodiments, the substantially pure population of cells according to the second aspect or any of its embodiments, or the pharmaceutical composition of the fourth aspect or any of its embodiments the composition for administration are contacted or transduced with the lentiviral vector according to the first aspect or any of its embodiments, either *in vivo, in vitro,* or *ex vivo.*

A pharmaceutical composition as described herein may also contain other substances. These substances include, but are not limited to, cryoprotectants, lyoprotectants, surfactants, bulking agents, anti-oxidants, and stabilizing agents. In some embodiments, the pharmaceutical composition may be lyophilized.

The term "*cryoprotectant*" as used herein, includes agents which provide stability to the lentiviral vector against freezing-induced stresses, by being preferentially excluded from the lentiviral vector's surface. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a pharmaceutical composition described herein. The term "*lyoprotectant*" as used herein, includes agents that provide stability to the lentiviral vector during the freeze-drying or dehydration process (primary and secondary freeze- drying cycles), by providing an amorphous glassy matrix and by binding with the lentiviral vector's surface through hydrogen bonding, replacing the water molecules that are removed during the drying process. This helps to minimize product degradation during the lyophilization cycle, and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulphate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a pharmaceutical composition is generally an amount that does not lead to an unacceptable amount of degradation of the strain when the pharmaceutical composition is lyophilized.

In some embodiments, a bulking agent is included in the pharmaceutical composition. The term "*bulking agent*" as used herein, includes agents that provide the structure of the freeze-dried product without interacting directly with the pharmaceutical product. In addition to providing a pharmaceutically elegant cake, bulking agents may also impart useful qualities in regard to modifying the collapse temperature, providing freeze-thaw protection, and enhancing the strain stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in formulations in an amount from 0.5% to 10%.

Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition. As used herein, "*pharmaceutically acceptable carrier*" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulphate; low molecular weight proteins, such as human serum albumin, bovine serum albumin, gelatin, or other immunoglobulins; and hydrophilic polymers, such as polyvinylpyrrolidone.

The pharmaceutical composition may be prepared for oral, sublingual, buccal, intravenous, intramuscular, subcutaneous, intraperitoneal, conjunctival, rectal, transdermal, intrathecal, topical and/or inhalation-mediated administration. In a preferred embodiment, the pharmaceutical composition may be a solution which is suitable for intravenous, intramuscular, conjunctival, transdermal, intraperitoneal and/or subcutaneous administration. In another embodiment, the pharmaceutical composition may be a solution which is suitable for sublingual, buccal and/or inhalation-mediated administration routes. In an alternative embodiment, the pharmaceutical composition may be a gel or solution which is suitable for intrathecal administration. In an alternative embodiment, the pharmaceutical composition may be an aerosol which is suitable for inhalation-mediated administration. In a preferred embodiment, the pharmaceutical composition may be prepared for intrathecal administration.

The pharmaceutical composition may further comprise common excipients and carriers which are known in the state of the art. For solid pharmaceutical compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the pharmaceutical composition may further comprise cryoprotectants, lyoprotectants, surfactants, bulking agents, anti-oxidants, stabilizing agents and pharmaceutically acceptable carriers. For aerosol administration, the pharmaceutical compositions are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides.

Therefore, a suitable pharmaceutical composition for injection can comprise a buffer (e.g. acetate, phosphate or citrate buffer), a surfactant (e.g. polysorbate), optionally a stabilizer agent (e.g. human albumin), etc. However, in other embodiments compatible with the teachings herein, the lentiviral vector can be delivered directly to the site of the adverse cellular population, that is the hepatocytes, thereby increasing the exposure of the diseased tissue to the therapeutic agent.

The lentiviral vector provided herein can optionally be administered in combination with other agents that are effective in treating the disorder or condition in need of treatment (e.g., prophylactic or therapeutic). The administration of lentiviral vectors provided herein in conjunction or combination with an adjunct therapy means the sequential, simultaneous, coextensive, concurrent, concomitant or contemporaneous administration or application of the therapy and the disclosed polypeptides. In an embodiment, the lentiviral vector is administered in combination with other agents that are able to induce hepatic cell proliferation, such as, but not limited to, triiodothyronine hormone, human-interleukin 6, and/or derivatives thereof. In another embodiment, the lentiviral vector is administered in combination with other agents that are able to inhibit the immune response against the lentiviral vector, such as, but not limited to, dexamethasone, ciclosporin A, rapamycin, and/or derivatives thereof. In another embodiment, the lentiviral vector is administered in combination with other agents that are able to increase the number of specific receptor in target cells for VSV-G pseudotyped lentivirus, such as, but not limited to, statins, iron quelators, and/or derivatives thereof. In another embodiment, the lentiviral vector is administered in combination with other agents that are able to block apoptosis in transduced hepatocytes, such as, but not limited to, antioxidants such as N-acetylcysteine, and/or derivatives thereof.

In a particular embodiment, the lentiviral vector according to the first aspect or any of its embodiments is administrated in combination with Cyclosporin H (CsH), which acts as a transduction enhancer. In a preferred embodiment, the CsH is administrated in a dose that ranges from 20-1, 15-1, 10-1, 5-1, or 5-2.5 mg/kg. In an embodiment, the CsH is administered before, at the same time, or after the lentivirus's administration. Doses intermediate in the above ranges are also intended to be within the scope of the invention. In some embodiments, the CsH is administered once. In other embodiments, the CsH is administered more than once, at least twice, at least three times, at least four times, at least five times, at least six times, at least seven times, at least eight times, at least nine times, or at least ten times. The repetitive administrations of CsH may be performed in the same day or separated in time. In an embodiment, intervals between single dosages of CsH can be daily, weekly, monthly or yearly.

Medial uses and Schedule, route and dose of administration

In a **fifth aspect**, the present invention provides the lentiviral vector according to the first aspect or any of its embodiment, the host cell or the substantially pure population of cells according to the second aspect or any of its embodiments, the isolated nucleic acid according to the third aspect or any of its embodiments, or the pharmaceutical composition of the fourth aspect or any of its embodiments for use as a medicament. In a **sixth aspect**, the present invention provides the lentiviral vector according to the first aspect or any of its embodiment, the host cell or the substantially pure population of cells according to the second aspect or any of its embodiments, the isolated nucleic acid according to the third aspect or any of its embodiments, or the pharmaceutical composition of the fourth aspect or any of its embodiments for use in the treatment of Primary Hyperoxaluria, preferably Primary Hyperoxaluria type 1, the use comprising administering to the subject said lentiviral, said host cell or population of cells, or said composition.

The present invention also provides methods of treating, preventing, or ameliorating the PH1 disorder in a subject in need thereof comprising administering to the subject a therapeutically effective amount of the lentiviral vector according to the first aspect or any of its embodiment, the host cell or the substantially pure population of cells according to the second aspect or any of its embodiments, the isolated nucleic acid of the third aspect or any of its embodiments, or the pharmaceutical composition of the fourth aspect or any of its embodiments.

In another embodiment, the administration of a lentiviral vector, the host cell or the substantially pure population of cells, or the composition provided herein does not induce an immune response in a subject or induces a very low immune response in said subject.

In some embodiments, the lentiviral vector, the host cell or the substantially pure population of cells, or the composition of the present invention are administered as a single dose or multiple doses. In some embodiments, the lentiviral vector, the host cell or the substantially pure population of cells, or the composition of the present invention dose are administered at once or divided into multiple sub-dose, e.g., two sub-doses, three sub-doses, four sub-doses, five sub-doses, six sub-doses, or more than six sub-doses. In some embodiments, more than one lentiviral vector is administered. Most preferably, the lentiviral vector, the host cell or the substantially pure population of cells, or the composition of the present invention are administered as a single dose.

In some embodiments, the dose of lentiviral vector of the present invention, the host cell or the substantially pure population of cells of the present invention or the pharmaceutical composition is administered repeated at least twice, at least three times, at least four times, at least five times, at least six times, at least seven times, at least eight times, at least nine times, or at least ten times.

The lentiviral vector of the present invention, the host cell or the substantially pure population of cells of the present invention or the pharmaceutical composition can be administered locally or systemically. In one embodiment, the route of administration of the lentiviral vectors is parenteral. The term parenteral as used herein includes intravenous, intraarterial, intraperitoneal, intramuscular, subcutaneous, rectal or vaginal administration. In an embodiment, said lentiviral vector can be administrated intravenously, subcutaneously, intramuscularly, or via any mucosal surface, e.g., orally, sublingually, buccally, sublingually, nasally, rectally, vaginally or via pulmonary route. The intravenous form of parenteral administration is preferred. In an embodiment, a form for administration would be a solution for injection, in particular for intravenous or intraarterial injection or drip.

Effective doses of the compositions provided herein, for the treatment of PH1 vary depending upon many different factors, including means of administration, target site, physiological state of the subject, whether the subject is a human being or an animal, other medications administered, and whether treatment is prophylactic or therapeutic. Usually, the subject is a human but non-human mammals including transgenic mammals can also be treated. Treatment dosages can be titrated using routine methods known to those of skill in the art to optimize safety and efficacy. In one embodiment, the subjects include, but are not limited to, individuals with PH1. In some embodiments, the subject is a pediatric subject, whereas in other aspects, the subject is an adult subject.

The lentiviral vector of the present invention, the host cell or the substantially pure population of cells of the present invention or the pharmaceutical composition can be administered as a single dose or as multiple doses, wherein the multiple doses can be administered continuously or at specific timed intervals. *In vitro or in vivo assays* can be employed to determine optimal dose ranges and/or schedules for administration. Additionally, effective doses can be extrapolated from dose-response curves obtained from animal models.

In a preferred embodiment, the lentiviral vector of the present invention, the host cell or the substantially pure population of cells of the present invention or the pharmaceutical composition is administered at a dose of at least 1 x $10^9$ vector genomes/Kg body weight, preferably 1 x $10^{10}$, 1 x $10^{11}$ or 1 x $10^{12}$ vector genomes/Kg body weight. More preferably, at least or about 4.6 x $10^{12}$ vector genomes/Kg body weight are administered.

In an embodiment, the dose of lentiviral is of at least 5 x $10^2$, 5 x $10^3$, 5 x $10^4$, 5 x $10^5$, 5 x $10^6$, 5 x $10^7$, 5 x $10^8$, 5 x $10^9$, 5 x $10^{10}$, 5 x $10^{11}$, 5 x $10^{12}$, 5 x $10^{13}$, 5 x $10^{14}$, 5 x $10^{15}$ transducing units/kg (TU/kg). In an embodiment, the dose of lentiviral is between 5 x $10^2$ to 5 x $10^3$; 5 x $10^3$ to 5 x $10^4$; 5 x $10^4$ to 5 x $10^5$; 5 x $10^5$ to 5 x $10^6$; 5 x $10^6$ to 5 x $10^7$; 5 x $10^8$ to 5 x $10^9$; 5 x $10^9$ to 5 x $10^{10}$; 5 x $10^{10}$ to 5 x $10^{11}$; 5 x $10^{11}$ to 5 x $10^{12}$ transducing units/kg (TU/kg). In an embodiment, the dose of lentiviral is about 1 x $10^2$, 1 x $10^3$, 1 x $10^4$, 1 x $10^5$, 1 x $10^6$, 1 x $10^7$, 1 x $10^8$, 1 x $10^9$, 1 x $10^{10}$, 1 x $10^{11}$, 1 x $10^{12}$, 1 x $10^{13}$, 1 x $10^{14}$, 1 x $10^{15}$ transducing units/kg (TU/kg) or about 5 x $10^2$, 5 x $10^3$, 5 x $10^4$, 5 x $10^5$, 5 x $10^6$, 5 x $10^7$, 5 x $10^8$, 5 x $10^9$, 5 x $10^{10}$, 5 x $10^{11}$, 5 x 1012, 5 x $10^{13}$, 5 x $10^{14}$, 5 x $10^{15}$ transducing units/kg (TU/kg), or about 7.5 x $10^2$, 7.5 x $10^3$, 7.5 x $10^4$, 7.5 x $10^5$, 7.5 x $10^6$, 7.5 x $10^7$, 7.5 x $10^8$, 7.5 x $10^9$, 7.5 x $10^{10}$, 7.5 x $10^{11}$, 7.5 x 1012, 7.5 x $10^{13}$, 7.5 x $10^{14}$, 7.5 x $10^{15}$ transducing units/kg (TU/kg).

Doses intermediate in the above ranges are also intended to be within the scope of the invention.

The lentiviral vector of the present invention, the host cell or the substantially pure population of cells of the present invention or the pharmaceutical composition provided herein can be administered on multiple occasions. Intervals between single dosages can be daily, weekly, monthly or yearly. Intervals can also be irregular depending on the development of the disease or the curative effects of said lentivirals.

The dosage and frequency of administration of the lentiviral vector of the present invention, the host cell or the substantially pure population of cells of the present invention or the pharmaceutical composition can vary depending on whether the treatment is prophylactic or therapeutic. In prophylactic applications, compositions containing the lentiviral vector provided herein are administered to a subject not already in the disease state to enhance the subject's resistance or minimize effects of disease. Such an amount is defined to be a "prophylactic effective dose." A relatively low dosage is administered at relatively infrequent intervals over a long period of time. Some subjects may continue to receive treatment for the rest of their lives.

## EXAMPLES

### EXAMPLE 1: Materials, methods and sequences

#### 1.1 Lentiviral vector construction.

Reporter lentiviral vector (LV) construct, which expresses an enhanced version of Green Fluorescent Protein (eGFP), was developed from a LV construct previously described by Naldini and Cantore's group (LV.ET.FIX.142-3pT) (Brown *et al.,* 2007). *eGFP* expressing LV was developed by classic cloning techniques. LV.ET.FIX.142-3pT was digested by Nhel and Sall restriction enzymes (New England Biolabs®) to remove FIX sequence. Target sequences of Nhel and Sall are in 5' and 3' ends of FIX gene, respectively. After digestion of the plasmid with both enzymes, LV structure was isolated by electrophoresis and band purification.

Furthermore, we amplified *eGFP* sequence from #241.pCCL.sin.PPT.TTR.GFP.Wpre plasmid by PCR. Primers were designed to add Nhel (SEQ ID NO: 1 Fw: 5'-ATTGGCTAGCATGGTGAGCAAGGGCGAGGA -3') and Sall (SEQ ID NO: 2 Rv: 5'-CATTGTCGACTTACTTGTACAGCTCGTCCA -3') restrictions enzymes target sequences at 5' and 3' ends respectively. Finally, both sequences were ligated and LV.ET.eGFP.142-3pT was obtained.

The expression cassette of LV.ET.eGFP.142-3pT contains an engineered hepatocyte-specific promoter (modified from Enhanced Transthyretin, GenBank accession number AY661265) and target sequences for the hematopoietic-lineage specific microRNA 142 (mature human microRNA 142 SEQ ID NO: 3 - miRNA-142-3p: UGUAGUGUUUCCUACUUUAUGGA), with the aim of avoiding off-target transgene expression in Antigen Presenting Cells and, therefore, the development of an immune response against the transduced cells.

**LV.ET.EGFP.142-3PT SEQUENCES:**

**5' LTR** (SEQ ID NO: 4): it is responsible for provirus transcription during lentiviral vector production. In this lentiviral vector construct 5' LTR is composed of a chimeric form of the CMV promoter and a part of wild-type HIV-1 5' LTR, RU5. The lack of U3 sequence of wild-type 5' LTR involves that this lentiviral vector construct is Tat-independent and, therefore, it will be produced in third generation.

TGGCCATTGCATACGTTGTATCCATATCATAATATGTACATTTATATTGGCTCATGTC
CAACATTACCGCCATGTTGACATTGATTATTGACTAGTTATTAATAGTAATCAATTAC
GGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGT

ATGTTCCCATAGTAACGCCAATAGGGACTTTCCATTGACGTCAATGGGTGGAGTATT
TACGGTAAACTGCCCACTTGGCAGTACATCAAGTGTATCATATGCCAAGTACGCCCC
CTATTGACGTCAATGACGGTAAATGGCCCGCCTGGCATTATGCCCAGTACATGACCT
TATGGGACTTTCCTACTTGGCAGTACATCTACGTATTAGTCATCGCTATTACCATGGT
GATGCGGTTTTGGCAGTACATCAATGGGCGTGGATAGCGGTTTGACTCACGGGGAT
TTCCAAGTCTCCACCCCATTGACGTCAATGGGAGTTTGTTTTGGCACCAAAATCAAC
GGGACTTTCCAAAATGTCGTAACAACTCCGCCCCATTGACGCAAATGGGCGGTAGG
CGTGTACGGTGGGAGGTCTATATAAGCAGAGCTCGTTTAGTGAACCGGGGTCTCTC
TGGTTAGACCAGATCTGAGCCTGGGAGCTCTCTGGCTAACTAGGGAACCCACTGCT
TAAGCCTCAATAAAGCTTGCCTTGAGTGCTTCAAGTAGTGTGTGCCCGTCTGTTGTG
TGACTCTGGTAACTAGAGATCCCTCAGACCCTTTTAGTCAGTGTGGAAAATCTCTAG
CAG

**Chimeric CMV promoter** (SEQ ID NO: 5): This sequence leads the transcription of provirus during the lentiviral vector production. It is composed of two different sequences, one with enhancer activity and the other one with promoter activity.

TGGCCATTGCATACGTTGTATCCATATCATAATATGTACATTTATATTGGCTCATGTC
CAACATTACCGCCATGTTGACATTGATTATTGACTAGTTATTAATAGTAATCAATTAC
GGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGT
ATGTTCCCATAGTAACGCCAATAGGGACTTTCCATTGACGTCAATGGGTGGAGTATT
TACGGTAAACTGCCCACTTGGCAGTACATCAAGTGTATCATATGCCAAGTACGCCCC
CTATTGACGTCAATGACGGTAAATGGCCCGCCTGGCATTATGCCCAGTACATGACCT
TATGGGACTTTCCTACTTGGCAGTACATCTACGTATTAGTCATCGCTATTACCATGGT
GATGCGGTTTTGGCAGTACATCAATGGGCGTGGATAGCGGTTTGACTCACGGGGAT
TTCCAAGTCTCCACCCCATTGACGTCAATGGGAGTTTGTTTTGGCACCAAAATCAAC
GGGACTTTCCAAAATGTCGTAACAACTCCGCCCCATTGACGCAAATGGGCGGTAGG
CGTGTACGGTGGGAGGTCTATATAAGCAGAGCTCGTTTAGTGAACC

◦ **CMV enhancer** (SEQ ID NO: 6):

GACATTGATTATTGACTAGTTATTAATAGTAATCAATTACGGGGTCATTAGTTCAT
AGCCCATATATGGAGTTCCGCGTTACATAACTTACGGTAAATGGCCCGCCTGGC
TGACCGCCCAACGACCCCCGCCCATTGACGTCAATAATGACGTATGTTCCCATA
GTAACGCCAATAGGGACTTTCCATTGACGTCAATGGGTGGAGTATTTACGGTAAA
CTGCCCACTTGGCAGTACATCAAGTGTATCATATGCCAAGTACGCCCCCTATTGA
CGTCAATGACGGTAAATGGCCCGCCTGGCATTATGCCCAGTACATGACCTTATG
GGACTTTCCTACTTGGCAGTACATCTACGTATTAGTCATCGCTATTACCATG

◦ **CMV promoter** (SEQ ID NO: 7):

GTGATGCGGTTTTGGCAGTACATCAATGGGCGTGGATAGCGGTTTGACTCACGG
GGATTTCCAAGTCTCCACCCCATTGACGTCAATGGGAGTTTGTTTTGGCACCAAA
ATCAACGGGACTTTCCAAAATGTCGTAACAACTCCGCCCCATTGACGCAAATGG
GCGGTAGGCGTGTACGGTGGGAGGTCTATATAAGCAGAGCT

**RU5** (SEQ ID NO: 8): Truncated 5' LTR without RU3. This sequence is necessary for retrotranscription and integration of proviral genome into transduced cells.

GGGTCTCTCTGGTTAGACCAGATCTGAGCCTGGGAGCTCTCTGGCTAACTAGGGAA
CCCACTGCTTAAGCCTCAATAAAGCTTGCCTTGAGTGCTTCAAGTAGTGTGTGCCCG
TCTGTTGTGTGACTCTGGTAACTAGAGATCCCTCAGACCCTTTTAGTCAGTGTGGAA
AATCTCTAGCAG

**PBS** SL23 SL123 (Primer Binding Site) (SEQ ID NO: 9): tRNA is merged to PBS SL23 element during reverse transcription of proviral genome after target cell transduction and before integration into the cell genome.

TGGCGCCCGAACAGGGACTTGAAAGCGAAAGGGAAACCAGAGGAGCTCTCTCGAC
GCAGGACTCGGCTTGCTGAAGCGCGCACGGCAAGAGGCGAGGGGCGGCGACTGG
TGAGTACGCCAAAAATTTTGACTAGCGGAGGCTAGAAGGAGAGAG

Ψ **(packaging signal)** (SEQ ID NO: 10): This element is responsible for dimerization and packaging of lentiviral vector particles.

CTCTCTCGACGCAGGACTCGGCTTGCTGAAGCGCGCACGGCAAGAGGCGAGGGGC
GGCGACTGGTGAGTACGCCAAAAATTTTGACTAGCGGAGGCTAGAAGGAGAGAGAT
GGGTGCGAGAGCGTC

**SL4mgag** (SEQ ID NO: 11): This element is a part of wild-type mgag HIV-1 gene which is involved in provirus packaging.

ATGGGTGCGAGAGCGTCAGTATTAAGCGGGGGAGAATTAGATCGCGATGGGAAAAA
ATTCGGTTAAGGCCAGGGGGAAAGAAAAAATATAAATTAAAACATATAGTATGGGCA
AGCAGGGAGCTAGAACGATTCGCAGTTAATCCTGGCCTGTTAGAAACATCAGAAGG
CTGTAGACAAATACTGGGACAGCTACAACCATCCCTTCAGACAGGATCAGAAGAACT
TAGATCATTATATAATACAGTAGCAACCCTCTATTGTGTGCATCAAAGGATAGAGATA
AAAGACACCAAGGAAGCTTTAGACAAGATAGAGGAAGAGCAAAACAAAAGTAAGAC
CACCGCACAGCAAGCGGCCGCTGAT

**DenvRF1** (SEQ ID NO: 12):

TCTTCAGACCTGGAGGAGGAGATATGAGGGACAATTGGAGAAGTGAATTATATAAAT
ATAAAGTAGTAAAAATTGAACCATTAGGAGTAGCACCCACCAAGGCAAAGAGAAGAG
TGGTGCAGAGAGAAAAAAGAGCAGTGGGAATA

RRE (Rev-response element) (SEQ ID NO: 13): Rev will merge to this sequence in lentiviral vector transcripts to help nuclear exportation of provirus during lentiviral vector production.

AGGAGCTTTGTTCCTTGGGTTCTTGGGAGCAGCAGGAAGCACTATGGGCGCAGCGT
CAATGACGCTGACGGTACAGGCCAGACAATTATTGTCTGGTATAGTGCAGCAGCAG
AACAATTTGCTGAGGGCTATTGAGGCGCAACAGCATCTGTTGCAACTCACAGTCTG
GGGCATCAAGCAGCTCCAGGCAAGAATCCTGGCTGTGGAAAGATACCTAAAGGATC
AACAGCTCCT

DenvRF2 (SEQ ID NO: 14):

GGGTTGCTCTGGAAAACTCATTTGCACCACTGCTGTGCCTTGGAATGCTAGTTGGA GTAATAAATCTCTGGAACAGATTTGGAATCACACGACCTGGATGGAGTGGGACAGA GAAATTAACAATTACACAAGCTTAATACACTCCTTAATTGAAGAATCGCAAAACCAGC AAGAAAAGAATGAACAAGAATTATTGGAATTAGATAAATGGGCAAGTTTGTGGAATT GGTTTAACATAACAAATTGGCTGTGGTATATAAAATTATTCATAATGATAGTAGGAGG CTTGGTAGGTTTAAGAATAGTTTTTGCTGTACTTTCTATAGTGAATAGAGTTAGGCAG GGATATTCACCATTATCGTTTCAGACCCACCTCCCAACCCCGAGGGGACCCGACAG GCCCGAAGGAATAGAAGAAGAAGGTGGAGAGAGAGACAGAGACAGATCCATTCGAT TAGTGAACGGATC

**cPPT** (central polypurin tract) (SEQ ID NO: 15): This element act as a primer for positive DNA chain transcription during reverse transcription of lentiviral vectors after transduction of target cells. It also increases proviral DNA nuclear import and, therefore, lentiviral vector transduction efficacy. Moreover, it increases lentiviral vector titles.

TTTTAAAAGAAAAGGGGGGATTGGGGGGTACAGTGCAGGGGAAAGAATAGTAGACA TAATAGCAACAGACATACAAACTAAAGAATTACAAAAACAAATTACAAAAATTCAAAAT TTT

**ET Promoter** (SEQ ID NO: 16): It is an engineered hepatocyte-specific promoter. This sequence leads the transgene expression after lentiviral vector genome integration into the target cells. It is a chimeric promoter composed of a synthetic enhancer, an mTTR enhancer, and an mTTR promoter (Enhanced Transthyretin, GenBank accession number AY661265).

CGCGAGTTAATAATTACCAGCGCGGGCCAAATAAATAATCCGCGAGGGGCAGGTGA CGTTTGCCCAGCGCGCGCTGGTAATTATTAACCTCGCGAATATTGATTCGAGGCCG CGATTGCCGCAATCGCGAGGGGCAGGTGACCTTTGCCCAGCGCGCGTTCGCCCCG CCCCGGACGGTATCGATAAGCTTAGGAGCTTGGGCTGCAGGTCGAGGGCACTGGG AGGATGTTGAGTAAGATGGAAAACTACTGATGACCCTTGCAGAGACAGAGTATTAGG ACATGTTTGAACAGGGGCCGGGCGATCAGCAGGTAGCTCTAGAGGATCCCCGTCT GTCTGCACATTTCGTAGAGCGAGTGTTCCGATACTCTAATCTCCCTAGGCAAGGTTC ATATTTGTGTAGGTTACTTATTCTCCTTTTGTTGACTAAGTCAATAATCAGAATCAGCA GGTTTGGAGTCAGCTTGGCAGGGATCAGCAGCCTGGGTTGGAAGGAGGGGGTATA AAAGCCCCTTCACCAGGAGAAGCCGTCACACAGATCCACAAGCTCCTG

**ET promoter components:**

◦ **Synthetic enhancer** (SEQ ID NO: 17):

CGCGAGTTAATAATTACCAGCGCGGGCCAAATAAATAATCCGCGAGGGG
CAGGTGACGTTTGCCCAGCGCGCTGGTAATTATTAACCTCGCGAATA
TTGATTCGAGGCCGCGATTGCCGCAATCGCGAGGGGCAGGTGACCTTT
GCCCAGCGCGCG

◦ **mTTR enhancer** (SEQ ID NO: 18):

CACTGGGAGGATGTTGAGTAAGATGGAAAACTACTGATGACCCTTGCAG
AGACAGAGTATTAGGACATGTTTGAACAGGGGCCGGGCGATCAGCAGGT
AG

◦ **mTTR promoter** (SEQ ID NO: 19):

GTCTGTCTGCACATTTCGTAGAGCGAGTGTTCCGATACTCTAATCTCCCT
AGGCAAGGTTCATATTTGTGTAGGTTACTTATTCTCCTTTTGTTGACTAAG

TCAATAATCAGAATCAGCAGGTTTGGAGTCAGCTTGGCAGGGATCAGCA
GCCTGGGTTGGAAGGAGGGGGTATAAAAGCCCCTTCACCAGGAGAAGC
CGTC

◦ **mTTR 5' UT** (SEQ ID NO: 20): ACACAGATCCACAAGCTCCTG

***eGFP* sequence** (SEQ ID NO: 51): This sequence codifies for an enhanced version of Green Fluorescent Protein gene.

TGGTGAGCAAGGGCGAGGAGCTGTTCACCGGGGTGGTGCCCATCCTGGTCGAGCT
GGACGGCGACGTAAACGGCCACAAGTTCAGCGTGTCCGGCGAGGGCGAGGGCGA
TGCCACCTACGGCAAGCTGACCCTGAAGTTCATCTGCACCACCGGCAAGCTGCCCG
TGCCCTGGCCCACCCTCGTGACCACCCTGACCTACGGCGTGCAGTGCTTCAGCCG
CTACCCCGACCACATGAAGCAGCACGACTTCTTCAAGTCCGCCATGCCCGAAGGCT
ACGTCCAGGAGCGCACCATCTTCTTCAAGGACGACGGCAACTACAAGACCCGCGCC
GAGGTGAAGTTCGAGGGCGACACCCTGGTGAACCGCATCGAGCTGAAGGGCATCG
ACTTCAAGGAGGACGGCAACATCCTGGGGCACAAGCTGGAGTACAACTACAACAGC
CACAACGTCTATATCATGGCCGACAAGCAGAAGAACGGCATCAAGGTGAACTTCAA
GATCCGCCACAACATCGAGGACGGCAGCGTGCAGCTCGCCGACCACTACCAGCAG
AACACCCCCATCGGCGACGGCCCCGTGCTGCTGCCCGACAACCACTACCTGAGCA
CCCAGTCCGCCCTGAGCAAAGACCCCAACGAGAAGCGCGATCACATGGTCCTGCT
GGAGTTCGTGACCGCCGCCGGGATCACTCTCGGCATGGACGAGCTGTACAAGTAA
A

**Mutated WPRE** (WHV (woodchuck hepatitis virus) post-transcriptional regulatory element) (SEQ ID NO: 21): It increases transgene expression in transduced cells due to improvements in polyadenylation, RNA export from the nuclei and protein synthesis. It contains a mutation in gene X frame to increase security.

CAACCTCTGGATTACAAAATTTGTGAAAGATTGACTGGTATTCTTAACTATGTTGCTC
CTTTTACGCTATGTGGATACGCTGCTTTAATGCCTTTGTATCATGCTATTGCTTCCCG
TATGGCTTTCATTTTCTCCTCCTTGTATAAATCCTGGTTGCTGTCTCTTTATGAGGAG
TTGTGGCCCGTTGTCAGGCAACGTGGCGTGGTGTGCACTGTGTTTGCTGACGCAAC
CCCCACTGGTTGGGGCATTGCCACCACCTGTCAGCTCCTTTCCGGGACTTTCGCTT
TCCCCCTCCCTATTGCCACGGCGGAACTCATCGCCGCCTGCCTTGCCCGCTGCTGG
ACAGGGGCTCGGCTGTTGGGCACTGACAATTCCGTGGTGTTGTCGGGGAAATCATC
GTCCTTTCCTTGGCTGCTCGCCTGTGTTGCCACCTGGATTCTGCGCGGGACGTCCT
TCTGCTACGTCCCTTCGGCCCTCAATCCAGCGGACCTTCCTTCCCGC

**142-3pT** (SEQ ID NO: 22): Target sequence of 142-3 micro-RNA, which is specifically expressed in hematopoietic cells. This element increases the expression specificity of the transgene in transduced cells being a transcriptional control element.
TCCATAAAGTAGGAAACACTACA

**3' LTR: DR3RU5** (SEQ ID NO: 23): 3' LTR is involved in mRNAs polyadenylation during lentiviral vector production. In this case, 3' LTR is composed of a truncated U3 element and the elements R and U5 from wild-type HIV-1, being a self-inactivating lentiviral vector. The integrated viral genome will contain a deletion in viral promoter region (U3), resulting in the transcriptional inactivation of potentially packable viral genomes in the transduced cells.

TGGAAGGGCTAATTCACTCCCAACGAAGACAAGATCTGCTTTTTGCTTGTACTGGGT
CTCTCTGGTTAGACCAGATCTGAGCCTGGGAGCTCTCTGGCTAACTAGGGAACCCA
CTGCTTAAGCCTCAATAAAGCTTGCCTTGAGTGCTTCAAGTAGTGTGTGCCCGTCTG
TTGTGTGACTCTGGTAACTAGAGATCCCTCAGACCCTTTTAGTCAGTGTGGAAAATC
TCTAGCA

◦ **ΔU3** (SEQ ID NO: 24):

TGGAAGGGCTAATTCACTCCCAACGAAGACAAGATCTGCTTTTTGCTTGT
ACT

◦ **RU5** (SEQ ID NO: 25):

GGGTCTCTCTGGTTAGACCAGATCTGAGCCTGGGAGCTCTCTGGCTAAC
TAGGGAACCCACTGCTTAAGCCTCAATAAAGCTTGCCTTGAGTGCTTCAA
GTAGTGTGTGCCCGTCTGTTGTGTGACTCTGGTAACTAGAGATCCCTCA
GACCCTTTTAGTCAGTGTGGAAAATCTCTAGCAG

**LV.ET.eGFP.142-3pT sequence** (SEQ ID NO: 26):

TGGCCATTGCATACGTTGTATCCATATCATAATATGTACATTTATATTGGCTCATGTC
CAACATTACCGCCATGTTGACATTGATTATTGACTAGTTATTAATAGTAATCAATTAC
GGGGTCATTAGTTCATAGCCCATATATGGAGTTCCGCGTTACATAACTTACGGTAAA
TGGCCCGCCTGGCTGACCGCCCAACGACCCCCGCCCATTGACGTCAATAATGACGT
ATGTTCCCATAGTAACGCCAATAGGGACTTTCCATTGACGTCAATGGGTGGAGTATT
TACGGTAAACTGCCCACTTGGCAGTACATCAAGTGTATCATATGCCAAGTACGCCCC
CTATTGACGTCAATGACGGTAAATGGCCCGCCTGGCATTATGCCCAGTACATGACCT
TATGGGACTTTCCTACTTGGCAGTACATCTACGTATTAGTCATCGCTATTACCATGGT

GATGCGGTTTTGGCAGTACATCAATGGGCGTGGATAGCGGTTTGACTCACGGGGAT
TTCCAAGTCTCCACCCCATTGACGTCAATGGGAGTTTGTTTTGGCACCAAAATCAAC
GGGACTTTCCAAAATGTCGTAACAACTCCGCCCCATTGACGCAAATGGGCGGTAGG
CGTGTACGGTGGGAGGTCTATATAAGCAGAGCTCGTTTAGTGAACCGGGGTCTCTC
TGGTTAGACCAGATCTGAGCCTGGGAGCTCTCTGGCTAACTAGGGAACCCACTGCT
TAAGCCTCAATAAAGCTTGCCTTGAGTGCTTCAAGTAGTGTGTGCCCGTCTGTTGTG
TGACTCTGGTAACTAGAGATCCCTCAGACCCTTTTAGTCAGTGTGGAAAATCTCTAG
CAGTGGCGCCCGAACAGGGACCTGAAAGCGAAAGGGAAACCAGAGCTCTCTCGAC
GCAGGACTCGGCTTGCTGAAGCGCGCACGGCAAGAGGCGAGGGGCGGCGACTGG
TGAGTACGCCAAAAATTTTGACTAGCGGAGGCTAGAAGGAGAGAGATGGGTGCGAG
AGCGTCAGTATTAAGCGGGGGAGAATTAGATCGCGATGGGAAAAAATTCGGTTAAG
GCCAGGGGGAAAGAAAAAATATAAATTAAAACATATAGTATGGGCAAGCAGGGAGC
TAGAACGATTCGCAGTTAATCCTGGCCTGTTAGAAACATCAGAAGGCTGTAGACAAA
TACTGGGACAGCTACAACCATCCCTTCAGACAGGATCAGAAGAACTTAGATCATTAT
ATAATACAGTAGCAACCCTCTATTGTGTGCATCAAAGGATAGAGATAAAAGACACCA
AGGAAGCTTTAGACAAGATAGAGGAAGAGCAAAACAAAAGTAAGACCACCGCACAG
CAAGCGGCCGCTGATCTTCAGACCTGGAGGAGGAGATATGAGGGACAATTGGAGA
AGTGAATTATATAAATATAAAGTAGTAAAAATTGAACCATTAGGAGTAGCACCCACCA
AGGCAAAGAGAAGAGTGGTGCAGAGAGAAAAAAGAGCAGTGGGAATAGGAGCTTT
GTTCCTTGGGTTCTTGGGAGCAGCAGGAAGCACTATGGGCGCAGCCTCAATGACGC
TGACGGTACAGGCCAGACAATTATTGTCTGGTATAGTGCAGCAGCAGAACAATTTGC
TGAGGGCTATTGAGGCGCAACAGCATCTGTTGCAACTCACAGTCTGGGGCATCAAG
CAGCTCCAGGCAAGAATCCTGGCTGTGGAAAGATACCTAAAGGATCAACAGCTCCT
GGGGATTTGGGGTTGCTCTGGAAAACTCATTTGCACCACTGCTGTGCCTTGGAATG
CTAGTTGGAGTAATAAATCTCTGGAACAGATTTGGAATCACACGACCTGGATGGAGT
GGGACAGAGAAATTAACAATTACACAAGCTTAATACACTCCTTAATTGAAGAATCGCA
AAACCAGCAAGAAAAGAATGAACAAGAATTATTGGAATTAGATAAATGGGCAAGTTT
GTGGAATTGGTTTAACATAACAAATTGGCTGTGGTATATAAAATTATTCATAATGATA
GTAGGAGGCTTGGTAGGTTTAAGAATAGTTTTTGCTGTACTTTCTATAGTGAATAGAG
TTAGGCAGGGATATTCACCATTATCGTTTCAGACCCACCTCCCAACCCCGAGGGGA
CCCGACAGGCCCGAAGGAATAGAAGAAGAAGGTGGAGAGAGAGACAGAGACAGAT
CCATTCGATTAGTGAACGGATCTCGACGGTATCGGTTAACTTTTAAAAGAAAAGGGG
GGATTGGGGGGTACAGTGCAGGGGAAAGAATAGTAGACATAATAGCAACAGACATA
CAAACTAAAGAATTACAAAAACAAATTACAAAAATTCAAAATTTTATCGATCACGAGA
CTAGCCTCGATCGAGGTCAATTCACGCGAGTTAATAATTACCAGCGCGGGCCAAAT
AAATAATCCGCGAGGGGCAGGTGACGTTTGCCCAGCGCGCGCTGGTAATTATTAAC
CTCGCGAATATTGATTCGAGGCCGCGATTGCCGCAATCGCGAGGGGCAGGTGACC

TTTGCCCAGCGCGCGTTCGCCCCGCCCCGGACGGTATCGATAAGCTTAGGAGCTT
GGGCTGCAGGTCGAGGGCACTGGGAGGATGTTGAGTAAGATGGAAAACTACTGAT
GACCCTTGCAGAGACAGAGTATTAGGACATGTTTGAACAGGGGCCGGGCGATCAGC
AGGTAGCTCTAGAGGATCCCCGTCTGTCTGCACATTTCGTAGAGCGAGTGTTCCGA
TACTCTAATCTCCCTAGGCAAGGTTCATATTTGTGTAGGTTACTTATTCTCCTTTTGTT
GACTAAGTCAATAATCAGAATCAGCAGGTTTGGAGTCAGCTTGGCAGGGATCAGCA
GCCTGGGTTGGAAGGAGGGGGTATAAAAGCCCCTTCACCAGGAGAAGCCGTCACA
CAGATCCACAAGCTCCTGCCACCATGGTGAGCAAGGGCGAGGAGCTGTTCACCGG
GGTGGTGCCCATCCTGGTCGAGCTGGACGGCGACGTAAACGGCCACAAGTTCAGC
GTGTCCGGCGAGGGCGAGGGCGATGCCACCTACGGCAAGCTGACCCTGAAGTTCA
TCTGCACCACCGGCAAGCTGCCCGTGCCCTGGCCCACCCTCGTGACCACCCTGAC
CTACGGCGTGCAGTGCTTCAGCCGCTACCCCGACCACATGAAGCAGCACGACTTCT
TCAAGTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTGGTGA
ACCGCATCGAGCTGAAGGGCATCGACTTCAAGGAGGACGGCAACATCCTGGGGCA
CAAGCTGGAGTACAACTACAACAGCCACAACGTCTATATCATGGCCGACAAGCAGA
AGAACGGCATCAAGGTGAACTTCAAGATCCGCCACAACATCGAGGACGGCAGCGTG
CAGCTCGCCGACCACTACCAGCAGAACACCCCCATCGGCGACGGCCCCGTGCTGC
TGCCCGACAACCACTACCTGAGCACCCAGTCCGCCCTGAGCAAAGACCCCAACGA
GAAGCGCGATCACATGGTCCTGCTGGAGTTCGTGACCGCCGCCGGGATCACTCTC
GGCATGGACGAGCTGTACAAGTAAAGCGGCCTCGACGGCCCGCGGAATTTCGAC
AATCAACCTCTGGATTACAAAATTTGTGAAAGATTGACTGGTATTCTTAACTATGTTG
CTCCTTTTACGCTATGTGGATACGCTGCTTTAATGCCTTTGTATCATGCTATTGCTTC
CCGTATGGCTTTCATTTTCTCCTCCTTGTATAAATCCTGGTTGCTGTCTCTTTATGAG
GAGTTGTGGCCCGTTGTCAGGCAACGTGGCGTGGTGTGCACTGTGTTTGCTGACGC
AACCCCCACTGGTTGGGGCATTGCCACCACCTGTCAGCTCCTTTCCGGGACTTTCG
CTTTCCCCCTCCCTATTGCCACGGCGGAACTCATCGCCGCCTGCCTTGCCCGCTGC
TGGACAGGGGCTCGGCTGTTGGGCACTGACAATTCCGTGGTGTTGTCGGGGAAGC
TGACGTCCTTTCCATGGCTGCTCGCCTGTGTTGCCACCTGGATTCTGCGCGGGACG
TCCTTCTGCTACGTCCCTTCGGCCCTCAATCCAGCGGACCTTCCTTCCCGCGGCCT
GCTGCCGGCTCTGCGGCCTCTTCCGCGTCTTCGCCTTCGCCCTCAGACGAGTCGG
ATCTCCCTTTGGGCCGCCTCCCCGCCTGGAATTCGAGCTCCACCGCGGTGGCGGC
CGCTCTAGAGTCGACTCCATAAAGTAGGAAACACTACACGATTCCATAAAGTAGGAA
ACACTACAACCGGTTCCATAAAGTAGGAAACACTACATCACTCCATAAAGTAGGAAA
CACTACACTCGAGGGGGGGCCCGGTACCTTTAAGACCAATGACTTACAAGGCAGCT
GTAGATCTTAGCCACTTTTTAAAAGAAAAGGGGGGACTGGAAGGGCTAATTCACTCC
CAACGAAGACAAGATCTGCTTTTTGCTTGTACTGGGTCTCTCTGGTTAGACCAGATC

TGAGCCTGGGAGCTCTCTGGCTAACTAGGGAACCCACTGCTTAAGCCTCAATAAAG
CTTGCCTTGAGTGCTTCAAGTAGTGTGTGCCCGTCTGTTGTGTGACTCTGGTAACTA
GAGATCCCTCAGACCCTTTTAGTCAGTGTGGAAAATCTCTAGCA

**LV.ET.eGFP.142-3pT map**: LV.ET.eGFP.142-3pT construct. Different elements previously described are represented in Figure 11.

At the same time, we developed a therapeutic LV expressing an improved version of *AGXT* gene (AGXT-RHEAM). We used the isolated structure of LV.ET.FIX.142-3pT, obtained as explained above. We amplified the AGXT-RHEAM sequence, kindly provided by Eduardo Salido's group (Mesa-Torres *et al.,* 2014) by PCR. Primers were designed to add NheI (SEQ ID NO: 27 Fw: 5' CTA GCT AGC ATG GCC TCT CAC AAG CTG CT 3') and SalI (SEQ ID NO: 28 Rv: 5' CAA GTC GAC TCA CAG CTT CTT CTT GGG GC 3') target sequences in 5' and 3' ends respectively, to use them in the cloning. Finally, LV.ET.FIX.142-3pT structure and AGXT-RHEAM sequence were ligated and LV.ET.AGXT-RHEAM.142-3pT vector was obtained.

**LV.ET.AGXT-RHEAM.142-3PT SEQUENCES:**

LV.ET.AGXT-RHEAM.142-3pT sequence contains the same elements as LV.EG.eGFP.142-3pT except *eGFP,* which has been replaced by AGXT-RHEAM coding sequence.

**AGXT-RHEAM** (SEQ ID NO: 29): This sequence, developed by Eduardo Salido's group (Mesa-Torres *et al.,* 2014), codifies for an improved version of *AGXT* gene with 5 amino acid changes to increase protein stability and half-life.

ATGGCCTCTCACAAGCTGCTGGTGACCCCCCCAAGGCCCTGCTCAAGCCCCTCTC
CATCCCCAAC**CGT**CTCCTGCTGGGGCCTGGTCCTTCCAACCTGCCTCCTCGCATCA
TGGCAGCCGGGGGGCTGCAGATGATCGGG**CAC**ATGAGCAAG**GAA**ATGTACCAGAT
CATGGACGAGATCAAGGAAGGCATCCAGTACGTGTTCCAGACCAGGAACCCACTCA
CACTGGTCATCTCCGGCTCGGGACACTGTGCCCTGGAGGCCGCCCTGGTCAATGT
GCTGGAGCCTGGGGACTCCTTCCTGGTTGGGGCCAATGGCATTTGGGGGCAGCGA
GCC**GCG**GACATCGGGGAGCGCATAGGAGCCCGAGTGCACCCGATGACCAAGGAC
CCCGGAGGCCACTACACACTGCAGGAGGTGGAGGAGGGCCTGGCCCAGCACAAG
CCAGTGCTGCTGTTCTTAACCCACGGGGAGTCGTCCACCGGCGTGCTGCAGCCCC
TTGATGGCTTCGGGGAACTCTGCCACAGGTACAAGTGCCTGCTCCTGGTGGATTCG
GTGGCATCCCTGGGCGGGACCCCCCTTTACATGGACCGGCAAGGCATCGACATCC
TGTACTCGGGCTCCCAGAAGGCCCTGAACGCCCCTCCAGGGACCTCGCTCATCTCC

TTCAGTGACAAGGCCAAAAAGAAGATGTACTCCCGCAAGACGAAGCCCTTCTCCTTC TACCTGGACATCAAGTGGCTGGCCAACTTCTGGGGCTGTGACGACCAGCCCAGGAT GTACCATCACACAATCCCCGTCATCAGCCTGTACAGCCTGAGAGAGAGCCTGGCCC TCATTGCGGAACAGGGCCTGGAGAACAGCTGGCGCCAGCACCGCGAGGCCGCGG CGTATCTGCATGGGCGCCTGCAGGCACTGGGGCTGCAGCTCTTCGTGAAGGACCC GGCGCTCCGGCTTCCCACAGTCACCACTGTGGCTGTACCCGCTGGCTATGACTGGA GAGACATCGTCAGCTACGTC**ATG**GACCACTTCGACATTGAGATCATGGGTGGCCTT GGGCCCTCCACGGGGAAGGTGCTGCGGATCGGCCTGCTGGGCTGCAATGCCACC CGCGAGAATGTGGACCGCGTGACGGAGGCCCTGAGGGCGGCCCTGCAGCACTGC CCCAAGAAGAAGCTGTGA

**LV.ET.AGXT-RHEAM.142-3pT** (SEQ ID NO: 30):

TGGCCATTGCATACGTTGTATCCATATCATAATATGTACATTTATATTGGCTCATGTC CAACATTACCGCCATGTTGACATTGATTATTGACTAGTTATTAATAGTAATCAATTAC GGGGTCATTAGTTCATAGCCCATATATGGAGTTCCGCGTTACATAACTTACGGTAAA TGGCCCGCCTGGCTGACCGCCCAACGACCCCCGCCCATTGACGTCAATAATGACGT ATGTTCCCATAGTAACGCCAATAGGGACTTTCCATTGACGTCAATGGGTGGAGTATT TACGGTAAACTGCCCACTTGGCAGTACATCAAGTGTATCATATGCCAAGTACGCCCC CTATTGACGTCAATGACGGTAAATGGCCCGCCTGGCATTATGCCCAGTACATGACCT TATGGGACTTTCCTACTTGGCAGTACATCTACGTATTAGTCATCGCTATTACCATGGT GATGCGGTTTTGGCAGTACATCAATGGGCGTGGATAGCGGTTTGACTCACGGGGAT TTCCAAGTCTCCACCCCATTGACGTCAATGGGAGTTTGTTTTGGCACCAAAATCAAC GGGACTTTCCAAAATGTCGTAACAACTCCGCCCCATTGACGCAAATGGGCGGTAGG CGTGTACGGTGGGAGGTCTATATAAGCAGAGCTCGTTTAGTGAACCGGGGTCTCTC TGGTTAGACCAGATCTGAGCCTGGGAGCTCTCTGGCTAACTAGGGAACCCACTGCT TAAGCCTCAATAAAGCTTGCCTTGAGTGCTTCAAGTAGTGTGTGCCCGTCTGTTGTG TGACTCTGGTAACTAGAGATCCCTCAGACCCTTTTAGTCAGTGTGGAAAATCTCTAG CAGTGGCGCCCGAACAGGGACTTGAAAGCGAAAGGGAAACCAGAGGAGCTCTCTC GACGCAGGACTCGGCTTGCTGAAGCGCGCACGGCAAGAGGCGAGGGGCGGCGAC TGGTGAGTACGCCAAAAATTTTGACTAGCGGAGGCTAGAAGGAGAGAGATGGGTGC GAGAGCGTCAGTATTAAGCGGGGGAGAATTAGATCGCGATGGGAAAAAATTCGGTT AAGGCCAGGGGGAAAGAAAAAATATAAATTAAAACATATAGTATGGGCAAGCAGGG AGCTAGAACGATTCGCAGTTAATCCTGGCCTGTTAGAAACATCAGAAGGCTGTAGAC AAATACTGGGACAGCTACAACCATCCCTTCAGACAGGATCAGAAGAACTTAGATCAT TATATAATACAGTAGCAACCCTCTATTGTGTGCATCAAAGGATAGAGATAAAAGACAC CAAGGAAGCTTTAGACAAGATAGAGGAAGAGCAAAACAAAAGTAAGACCACCGCAC

AGCAAGCGGCCGCTGATCTTCAGACCTGGAGGAGGAGATATGAGGGACAATTGGA GAAGTGAATTATATAAATATAAAGTAGTAAAAATTGAACCATTAGGAGTAGCACCCAC CAAGGCAAAGAGAAGAGTGGTGCAGAGAGAAAAAAGAGCAGTGGGAATAGGAGCT TTGTTCCTTGGGTTCTTGGGAGCAGCAGGAAGCACTATGGGCGCAGCGTCAATGAC GCTGACGGTACAGGCCAGACAATTATTGTCTGGTATAGTGCAGCAGCAGAACAATTT GCTGAGGGCTATTGAGGCGCAACAGCATCTGTTGCAACTCACAGTCTGGGGCATCA AGCAGCTCCAGGCAAGAATCCTGGCTGTGGAAAGATACCTAAAGGATCAACAGCTC CTGGGGATTTGGGGTTGCTCTGGAAAACTCATTTGCACCACTGCTGTGCCTTGGAAT GCTAGTTGGAGTAATAAATCTCTGGAACAGATTTGGAATCACACGACCTGGATGGAG TGGGACAGAGAAATTAACAATTACACAAGCTTAATACACTCCTTAATTGAAGAATCGC AAAACCAGCAAGAAAAGAATGAACAAGAATTATTGGAATTAGATAAATGGGCAAGTT TGTGGAATTGGTTTAACATAACAAATTGGCTGTGGTATATAAAATTATTCATAATGATA GTAGGAGGCTTGGTAGGTTTAAGAATAGTTTTTGCTGTACTTTCTATAGTGAATAGAG TTAGGCAGGGATATTCACCATTATCGTTTCAGACCCACCTCCCAACCCCGAGGGGA CCCGACAGGCCCGAAGGAATAGAAGAAGAAGGTGGAGAGAGAGACAGAGACAGAT CCATTCGATTAGTGAACGGATCTCGACGGTATCGGTTAACTTTTAAAAGAAAAGGGG GGATTGGGGGGTACAGTGCAGGGGAAAGAATAGTAGACATAATAGCAACAGACATA CAAACTAAAGAATTACAAAAACAAATTACAAAAATTCAAAATTTTATCGATCACGAGA CTAGCCTCGAGCACGCGAGTTAATAATTACCAGCGCGGGCCAAATAAATAATCCGC GAGGGGCAGGTGACGTTTGCCCAGCGCGCGCTGGTAATTATTAACCTCGCGAATAT TGATTCGAGGCCGCGATTGCCGCAATCGCGAGGGGCAGGTGACCTTTGCCCAGCG CGCGTTCGCCCCGCCCCGGACGGTATCGATAAGCTTAGGAGCTTGGGCTGCAGGT CGAGGGCACTGGGAGGATGTTGAGTAAGATGGAAAACTACTGATGACCCTTGCAGA GACAGAGTATTAGGACATGTTTGAACAGGGGCCGGGCGATCAGCAGGTAGCTCTAG AGGATCCCCGTCTGTCTGCACATTTCGTAGAGCGAGTGTTCCGATACTCTAATCTCC CTAGGCAAGGTTCATATTTGTGTAGGTTACTTATTCTCCTTTTGTTGACTAAGTCAAT AATCAGAATCAGCAGGTTTGGAGTCAGCTTGGCAGGGATCAGCAGCCTGGGTTGGA AGGAGGGGGTATAAAAGCCCCTTCACCAGGAGAAGCCGTCACACAGATCCACAAGC TCCTGGCTAGCTAGCATGGCCTCTCACAAGCTGCTGGTGACCCCCCCCAAGGCCCT GCTCAAGCCCCTCTCCATCCCCAACCGTCTCCTGCTGGGGCCTGGTCCTTCCAACC TGCCTCCTCGCATCATGGCAGCCGGGGGGCTGCAGATGATCGGGCACATGAGCAA GGAAATGTACCAGATCATGGACGAGATCAAGGAAGGCATCCAGTACGTGTTCCAGA CCAGGAACCCACTCACACTGGTCATCTCCGGCTCGGGACACTGTGCCCTGGAGGC CGCCCTGGTCAATGTGCTGGAGCCTGGGGACTCCTTCCTGGTTGGGGCCAATGGC ATTTGGGGGCAGCGAGCCGCGGACATCGGGGAGCGCATAGGAGCCCGAGTGCAC CCGATGACCAAGGACCCCGGAGGCCACTACACACTGCAGGAGGTGGAGGAGGGC CTGGCCCAGCACAAGCCAGTGCTGCTGTTCTTAACCCACGGGGAGTCGTCCACCG

GCGTGCTGCAGCCCCTTGATGGCTTCGGGGAACTCTGCCACAGGTACAAGTGCCT
GCTCCTGGTGGATTCGGTGGCATCCCTGGGCGGGACCCCCCTTTACATGGACCGG
CAAGGCATCGACATCCTGTACTCGGGCTCCCAGAAGGCCCTGAACGCCCCTCCAG
GGACCTCGCTCATCTCCTTCAGTGACAAGGCCAAAAAGAAGATGTACTCCCGCAAG
ACGAAGCCCTTCTCCTTCTACCTGGACATCAAGTGGCTGGCCAACTTCTGGGGCTG
TGACGACCAGCCCAGGATGTACCATCACACAATCCCCGTCATCAGCCTGTACAGCC
TGAGAGAGAGCCTGGCCCTCATTGCGGAACAGGGCCTGGAGAACAGCTGGCGCCA
GCACCGCGAGGCCGCGGCGTATCTGCATGGGCGCCTGCAGGCACTGGGGCTGCA
GCTCTTCGTGAAGGACCCGGCGCTCCGGCTTCCCACAGTCACCACTGTGGCTGTAC
CCGCTGGCTATGACTGGAGAGACATCGTCAGCTACGTCATGGACCACTTCGACATT
GAGATCATGGGTGGCCTTGGGCCCTCCACGGGGAAGGTGCTGCGGATCGGCCTGC
TGGGCTGCAATGCCACCCGCGAGAATGTGGACCGCGTGACGGAGGCCCTGAGGG
CGGCCCTGCAGCACTGCCCCAAGAAGAAGCTGTGAGTCGACAATCAACCTCTGGAT
TACAAAATTTGTGAAAGATTGACTGGTATTCTTAACTATGTTGCTCCTTTTACGCTAT
GTGGATACGCTGCTTTAATGCCTTTGTATCATGCTATTGCTTCCCGTATGGCTTTCAT
TTTCTCCTCCTTGTATAAATCCTGGTTGCTGTCTCTTTATGAGGAGTTGTGGCCCGTT
GTCAGGCAACGTGGCGTGGTGTGCACTGTGTTTGCTGACGCAACCCCCACTGGTTG
GGGCATTGCCACCACCTGTCAGCTCCTTTCCGGGACTTTCGCTTTCCCCCTCCCTAT
TGCCACGGCGGAACTCATCGCCGCCTGCCTTGCCCGCTGCTGGACAGGGGCTCGG
CTGTTGGGCACTGACAATTCCGTGGTGTTGTCGGGGAAATCATCGTCCTTTCCTTGG
CTGCTCGCCTGTGTTGCCACCTGGATTCTGCGCGGGACGTCCTTCTGCTACGTCCC
TTCGGCCCTCAATCCAGCGGACCTTCCTTCCCGCGGCCTGCTGCCGGCTCTAGATA
ATCCATAAAGTAGGAAACACTACACGATTCCATAAAGTAGGAAACACTACAACCGGT
TCCATAAAGTAGGAAACACTACATCACTCCATAAAGTAGGAAACACTACACCCGGTC
GAGCTCGGTACCTTTAAGACCAATGACTTACAAGGCAGCTGTAGATCTTAGCCACTT
TTTAAAAGAAAAGGGGGGACTGGAAGGGCTAATTCACTCCCAACGAAGACAAGATC
TGCTTTTTGCTTGTACTGGGTCTCTCTGGTTAGACCAGATCTGAGCCTGGGAGCTCT
CTGGCTAACTAGGGAACCCACTGCTTAAGCCTCAATAAAGCTTGCCTTGAGTGCTTC
AAGTAGTGTGTGCCCGTCTGTTGTGTGACTCTGGTAACTAGAGATCCCTCAGACCCT
TTTAGTCAGTGTGGAAAATCTCTAGCA

**LV.ET.AGXT-RHEAM.142-3pT map.** LV.ET.AGXT-RHEAM.142-3pT construct. Different elements previously described are represented in Figure 12.

### 1.2 LV production

Laboratory-grade VSV.G-pseudotyped third-generation SIN LVs were produced by calcium phosphate transient transfection into HEK293T cells. Ten million HEK293T cells were seeded per 150 $cm^2$ cell culture dish and 24 hours later cells were transfected with a solution containing a mix of LV genome transfer plasmid (LV.ET.AGXT-RHEAM.142-3pT or LV.ET.eGFP.142-3pT) and packaging plasmids pMDLg/pRRE,pRSV.Rev, and pMD2.VSV.G. The medium was changed 6 hours after transfection and supernatant was collected 2 days later. Usually, a second collection was performed 24 hours after the first one. The collected supernatants were clarified by filtration (0.22 μm PES) and concentrated by centrifugation at 20,000 g for 120 minutes at 16°C. LV pellets were resuspended in an appropriate volume of sodium chloride 0,9 % and preserved at -80°C.

### 1.3 LV titration

LV.ET.AGXT-RHEAM.142-3pT titration was performed in HepG2, a hepatic cell line, due to the specificity of the target tissue in this strategy. For LV titration, $2.10^5$ cells were seeded in 24 well plates. Twenty-four hours after the seed, cells were transduced with serial dilutions of the LV in culture media and the number of cells at day 0 was determined. Five days after transduction, cells were collected, and genomic DNA (gDNA) was extracted using NucleoSpin® Tissue Kit (Macherey Nagel®) following manufacturer's instructions.

The number of lentiviral vector genomes (VCN) integrated into transduced cells was determined by qPCR. To analyse the copies of the lentiviral vector genome we used primers (SEQ ID NO: 31 Fw: 5' CAGGACTCGGCTTGCTGAAG 3'; SEQ ID NO: 32 Rv: 5' TCCCCCGCTTAATACTGACG 3') and a probe (SEQ ID NO: 33 5'-CGCACGGCAAGAGGCGAGG-3') (Taqman®, Thermo Fisher Scientific) designed on the ψ sequence of the lentiviral vector. To determine the amount of endogenous DNA we used primers (SEQ ID NO: 34 Fw: 5' GCTGTCATCTCTTGTGGGCTG 3'; SEQ ID NO: 35 Rv: 5' ACTCATGGGAGCTGCTGGTTC 3') and a probe (SEQ ID NO: 36 5' CCTGTCATGCCCACACAAATCTCTCC 3') (Taqman®, Thermo Fisher Scientific) against the human albumin gene. We also used a standard curve to determine the number of copies of ψ LV specific sequence and the number of diploid genomes per sample, which allows us to calculate the number of LV copies per cell (VCN = number of copies of ψ/number of diploid genomes). Finally, based on this parameter we calculated LV title, defined as the number of transducing units per millilitre, with the following formula:

$$Title\ (TU/mL)\ = \frac{number\ of\ cells\ at\ day\ 0 \cdot VCN\ per\ diploid\ genome}{LV\ volume\ (mL)}$$

Standard curve to extrapolate qPCR data was performed using serial dilutions of a DNA fragment which contains ψ and human albumin sequence. All reactions were carried out in duplicate in 7500 Fast Real-Time PCR System (Applied Biosystems®).

For LV.ET.eGFP.142-3pT titration we also transduced HepG2 as well as above. However, at day 3 post-transduction the percentage of GFP-expressing positive cells was analysed by Flow Cytometry. This parameter was used to calculate LV title with the following formula:

$$Title\ (TU/ml)\ = \left(\frac{number\ of\ cells\ at\ day\ 0 \cdot \%\ of\ fluorescence}{100}\right) \times \left(\frac{Dilution\ factor}{LV\ volume\ (ml)}\right)$$

### 1.4 Experimental animals

*Agxt1*$^{-/-}$ mice (B6.129SvAgxt$^{tm1Ull}$) were developed by targeted mutagenesis in embryonic stem cells (Salido *et al.,* 2006). Wild type C57BL/6 mice were purchased from The Jackson Laboratory®.

### 1.5 Animal procedures

LV.ET.AGXT-RHEAM.142-3pT or LV.ET.GFP.142-3pT was administered in adult male mice (12-14 weeks old) by tail vein injection. According to the experiments performed in *Agxt1*$^{-/-}$ mice, they received a single dose of LV.ET.AGXT-RHEAM.142-3pT ($2.5.10^8$TU/mouse; $5.10^8$ TU/mouse; $1.10^9$ TU/mouse) or LV.ET.eGFP.142-3pT ($1.4\text{-}10^8$ TU/mouse; $5.10^8$ TU/mouse). LV was injected in a total volume of 200 μl/mouse. Wild-type C57BL/6 mice received a single dose of LV.ET.eGFP.142-3pT ($1.10^8$ TU/mouse; $2.5.10^8$ TU/mouse). LV was injected in a total volume of 200 μl/mouse.

Three weeks after LV injection, *Agxt1*$^{-/-}$ mice were subjected to an ethylene glycol challenge. It has been reported that overloading of oxalate is needed to develop clinical signs of Primary Hyperoxaluria Type 1 in this mouse model. For this reason, 0.5% of ethylene glycol (Ethylene glycol Reagent Plus®, Sigma-Aldrich, REF 102466), a precursor of glyoxylate metabolism, was administered in drinking water for 7 days. During the ethylene glycol challenge, mice were isolated in metabolic cages and 24 hours urine was collected. Moreover, animal body weight was monitored during the whole treatment.

Seven days after the beginning of the ethylene glycol challenge in the case of *Agxt1*$^{-/-}$ mice or 4 weeks after the LV injection in the case of wild-type C57BL/6 mice, they were sacrificed.

For necropsy mice were anesthetised with ketamine (2.5 mg/20 g mouse) + medetomidine (0.2 mg/20 g mouse). Once unconscious, mice were sacrificed by diaphragm disruption and, therefore, they were infused with 20 ml of PBS1X to remove blood from the tissues to facilitate the following analysis. In sacrificed animals, different tissues were collected (liver, spleen, lungs, bone marrow, lymph nodes, thymus, brain, testicles, pancreas, and kidneys).

All animal procedures in which *Agxt1*$^{-/-}$ mice were involved were performed at CIMA (Centro de Investigation Medica Aplicada) in Pamplona. All experimental procedures were approved by the Ethics Committee of the University of Navarra and the Institute of Public Health of Navarra according to European Council Guidelines. All the experiments using animal models complied with all relevant ethical regulations. All animal procedures in which C57BL/6 mice were involved were performed at CIEMAT (Centro de Investigaciones Energéticas y Medioambientales y Tecnológicas) in Madrid. All experimental procedures were approved in PROEX 165-18 by Dirección General de Medio Ambiente of Comunidad de Madrid.

### 1.6 Determination of VCN in LV injected mice

After animals' sacrifice, different tissues were collected (liver, spleen, lungs, bone marrow, lymph nodes, thymus, brain, testicles, pancreas, and kidneys) and preserved at-80°C. For the determination of LV genomes integrated into these tissues, genomic DNA was extracted with the NucleoSpin® Tissue Kit (Macherey Nagel) following the manufacturer's instructions.

The number of integrated lentiviral vector genomes (VCN) in the injected mice tissues was determined by qPCR. To analyse the number of lentiviral vector genomes we used primers (SEQ ID NO: 37 Fw: 5' CAGGACTCGGCTTGCTGAAG 3'; SEQ ID NO: 38 Rv: 5' TCCCCCGCTTAATACTGACG 3') and a probe (SEQ ID NO: 39 5'-CGCACGGCAAGAGGCGAGG-3') (Taqman®, Thermo Fisher Scientific) designed on the $\psi$ sequence of the lentiviral vector. To determine the amount of endogenous DNA we used primers (SEQ ID NO: 40 Fw: 5' AAAACGAGCAGTGACGTGAGC 3'; SEQ ID NO: 41 Rv: 5' TTCAGTCATGCTGCTAGCGC 3') and a probe (SEQ ID NO: 42 5'-TGCACGGAAGCGTCTCGTCTCAGTC-3') against the mouse *titin* gene. Once determined the number of LV copies and the number of diploid genomes per sample, VCN was calculated (VCN = number of copies of $\psi$/number of diploid genomes).

A standard curve was performed using serial dilutions of a DNA fragment that contains $\psi$ and the *titin* sequences. All reactions were carried out in duplicate in 7500 Fast Real-Time PCR System (Applied Biosystems®).

### 1.7 Determination of *AGXTexpression*

To analyse the functionality of therapeutic lentiviral vector in injected *Agxt1*$^{-/-}$ mice, we determined transgene expression in the liver of these mice. To define this parameter, RNA from the liver was extracted by Trizol protocol. Briefly, liver fragments were homogenized in Trizol, after which we added chloroform to separate the aqueous phase. RNA was precipitated with isopropanol and glycogene, cleaned with ethanol 70%, and finally resuspended in free nucleases water. To assure the lack of genomic DNA in RNA samples, they were digested with DNase using DNase Max® Kit (Qiagen) following the manufacturer's instructions. RNA samples were maintained at -80°C.

Afterward, RT-PCR technique was performed and cDNA was synthesized from RNA samples by RETROscript® Kit (Ambion, USA) following the manufacturer's instructions.

Transgene expression was determined by qPCR. We designed primers against the AGXT-RHEAM (SEQ ID NO: 43 Rv: 5' GTCTTGCGGGAGTACATCTT 3'; SEQ ID NO: 44 Fw: 5' CAAGGCATCGACATCCTGTA 3') and two housekeeping genes, mouse *Tbp* (SEQ ID NO: 45 Rv: 5' GATGGGAATTCCAGGAGTCA 3'; SEQ ID NO: 46 Fw: 5' GGGAGAATCATGGACCAGA 3') and mouse *Actb* (SEQ ID NO: 47 Rv: 5' CTA AGG CCA ACC GTG AAA AG 3'; SEQ ID NO: 48 Fw: 5'ACC AGA GGC ATA CAG GGA CA 3'). All reactions were carried out in duplicate in Fast Real-Time PCR System (Applied Biosystems®). Quantification of relative expression of the transgene was performed by $\Delta\Delta$Ct analysis (Schefe *et al.,* 2006) referenced to two housekeeping genes, mouse *Tbp* and mouse *$\beta$-actin,* separately.

We also analysed mouse *Agxt* physiological expression in the liver of non-injected C57BL/6 mice. RNA was extracted from liver samples of non-injected wild-type mice and RT-PCR was performed as explained above. To analyse gene expression, primers against mouse *Agxt* gene were designed (SEQ ID NO: 49 Rv: 5' GACAAAGCCAGTCTCCTTCTAC 3'; SEQ ID NO: 50 Fw: 5' GTGACAGGTGTGGTATGATGAA 3'). For the expression analysis, mouse *Tbp* and mouse *Actb* were also measured by RT-qPCR. All reactions were carried out in duplicate in Fast Real-Time PCR System (Applied Biosystems®).Quantification of relative expression of the transgene was performed by $\Delta\Delta$Ct analysis (Schefe *et al.,* 2006) referenced to two housekeeping genes, mouse *Tbp* and mouse *Actb,* separately.

### 1.8 Determination of the percentage of transduced cells by immunostaining.

To analyse the percentage of transduced cells in the target tissue we performed an immunostaining assay to detect eGFP positive cells in the liver of LV.ET.eGFP.142-3pT injected mice.

From paraffin sections, samples were deparaffinized by incubation in xylene and decreased ethanol dilutions. Afterward, samples were incubated in blocking solution (10% of donkey serum in PBS 1X) for one hour and overnight with Goat-aeGFP (ab6673 abcam®) 1:100 and Rabbit- amsALB (AHP1478) 1:500 in blocking solution. The following day, samples were incubated with secondary antibodies (Donkey-aGoat AF® 488 (A11055 invitrogen®), Donkey-αRabbit AF® 488 (A21206 invitrogen®), Donkey-aGoat AF® 594 (A11058 invitrogen®)) 1:1000 and DAPI 1:1000 in blocking solution for 1 hour. Subsequently, samples were mounted with Mowiol® and visualized in a Zeiss AxioImager microscope. Images of each sample were acquired.

Determination of eGFP positive cells in liver immunostaining samples was performed by QuPath® software. The percentage of eGFP expressing cells with respect to the total of hepatocytes in each image was determined. At least ten different fields per sample were analysed (400x magnification).

### 1.9 Urine oxalate determination

Twenty four-hour urine from mice was collected to measure the total amount of oxalate removed from the organism in this period. Individual 24-h urines were collected in 50 μl of 5 N HCl and their volume was measured. Urine samples were clarified with activated charcoal and oxalate was measured by enzymatic determination by oxalate oxidation using Oxalate Kit (Trinity Biotech, REF 591-D) following the manufacturer's instructions. To determine the concentration of oxalate (μmol) in each sample, a reference curve of known concentrations of oxalate was performed (Oxalate Standard 0.5 mmol/l REF 591-3 Trinity Biotech). Finally, oxalate quantification (μmol/24 h) was calculated using the oxalate concentration and the urine volume.

### 1.10 Kidney Damage determination: Kidney Damage Score

After animals' sacrifice, kidneys were fixed in formalin and embedded in paraffin. Then, 5μm sections were obtained and stained with haematoxylin-eosin. To determine the damage produced we developed a "Kidney Damage Score" in which the stage of each sample was classified into 4 different stages according to the features observed:

- 0: no damage
- 1: Normal architecture, but the presence of some damage signs such as dilated ducts.
- 2: The architecture is partially affected and there is an increase in the presence of dilated ducts.
- 3: Tissue architecture is highly affected, and we can observe the presence of calcium oxalate crystals, indicating a nephrocalcinosis stage.

### 1.11 Transduction enhancers: Cyclosporin H *in vitro* testing.

To increase *in vivo* transduction efficacy after the treatment with the developed lentiviral vector we tested the effect of a transduction enhancer, Cyclosporin H (CsH), during the *in vitro* transduction protocol. Two different regimens of CsH were tested. Due to the important relevance of the target tissue origin, HepG2 cells, a hepatic cell line, were used.

For the first CsH regimen, $2.10^5$ cells were seeded in a 24-well plate. One day later, cells were transduced with a culture media solution containing LV.ET.eGFP.142-3pT at a MOI of 0.3 and CsH. Different CsH concentrations were tested (4, 8, and 16 μM). Three days after transduction, the percentage of eGFP positive cells was analysed by Flow Cytometry. All conditions were performed as triple experimental replicates.

For the second CsH regimen, $2.10^5$ cells were seeded in a 24-well plate. One day later, culture media was changed and cells were pre-incubated with culture media supplemented with 16 μM CsH. 16 hours later, cells were transduced with a solution containing LV.ET.eGFP.142-3pT at an MOI of 0.3 and 16 μM CsH. Three days after transduction, the percentage of eGFP positive cells was analysed by Flow Cytometry. All conditions were performed as triple experimental replicates.

### 1.12 Statistical analysis

Significant differences between groups were determined by the Mann-Whitney test, a non-parametric test that is used to compare two independent groups without Gaussian distribution. Analyses were performed with Graphpad Prism® software. Significative values were considered when $p<0.05$.

**EXAMPLE 2: Lentiviral vector efficiently transduces the liver in C57BL/6 mice and *Agxt1$^{-/-}$* mouse model after intravenous injection.**

First, as a proof of concept to study the feasibility of the developed gene therapy tool and to perform a transduction percentage and biodistribution analysis, we carried out an *in vivo* experiment in which adult C57BL/6 male mice were injected with a single dose of LV.ET.GFP.142-3pT (Figure 1a, Figure 2a). We tested two different doses ($1.10^8$ TU/mouse; $2.5.10^8$ TU/mouse). We observed 0.55 (0.34-0.56) lentiviral vector copies per diploid genome in C57BL/6 injected mice with a dose of $1{\cdot}10^8$ TU/mouse, whereas in C57BL/6 mice injected with a dose of $2.5.10^8$ TU/mouse, 1.03 (0.94-1.56) lentiviral vector copies per diploid genome were obtained (Figure 3). Immunostaining analysis reveals that this corresponds to 1.751 (1.13-5.55) % eGFP positive hepatocytes and 12.92 (10.72-13.81) % eGFP positive hepatocytes in the low and high dose, respectively (Figure 5). Representative images of these mice can be observed in Figure 5 (A-C; F-H).

Next, in order to further characterize the *in vivo* transduction efficiency of the lentiviral vector LV.ET.GFP.142-3pT and compare it with the transduction efficiency of the same lentiviral vector *in vitro,* HepG2 cells were transduced with LV.ET.eGFP.142-3pT at different Multiplicity of Infection (MOI). Three days after transduction, the percentage of eGFP positive cells was determined by flow cytometry. Using MOIs of about 1, a transduction percentage of 50% was achieved, whereas higher MOIs achieved 100% transduction in vitro. For *in vivo* transduction efficacy determination, adult C57BL/6 (Fig. 13, filled circle) or *Agxt1$^{-/-}$* mice (Fi. 13, open circle) were intravenously injected with LV.ET.eGFP.142-3pT at different doses ($1.10^8$ TU/mouse, $1.4.10^8$ TU/mouse, $2.10^8$ TU/mouse and $5.10^8$ TU/mouse). Corresponding MOIs were calculated only according to the estimated number of hepatocytes in the liver of these mice as previously described (Park *et al.,* 2000; Schmitt *et al.,* 2010). Therefore, the mice were injected with the following MOIs: 0.8, 1.1, 2, and 4. Percentage of transduced hepatocytes was determined 4 weeks later in liver histology samples. The results depicted in Fig. 13 show that there is a significant reduction in the transduction efficiency of this lentiviral vector when it is used *in vivo* in comparison with *in vitro* experiments.

Therefore, to test the feasibility of an *in vivo* administration of a therapeutic lentiviral vector as a gene therapy strategy to treat Primary Hyperoxaluria Type 1, adult male *Agxt1$^{-/-}$*mice were intravenously injected with a single dose of LV.ET.AGXT-RHEAM.142-3pT lentiviral vector (Figure 1B). Three different lentiviral vector doses ($2.5.10^8$ TU/mouse; $5.10^8$ TU/mouse; $1.10^9$ TU/mouse) were tested. Three weeks after injection, mice were subjected to an ethylene glycol challenge for 7 days (Figure 2B) and then sacrificed.

The number of genome lentiviral vector copies integrated in the liver was determined at sacrifice. There was a dose-related efficacy in liver transduction. Liver VCN achieved in the three different groups was 0.615 (0.39-1.46) in *Agxt1$^{-/-}$* mice which received $2.5.10^8$ TU/mouse; 0.765 (0.45-1.19) in *Agxt1$^{-/-}$* mice which received $5.10^8$ TU/mouse and 1.165 (0.85-0.32) in *Agxt1$^{-/-}$* mice which received $10.10^8$TU/mouse (Figure 3). We also analysed this parameter in non-injected mice and, as expected, we did not detect any copy of the lentiviral vector genome in those animals.

As control groups, we also injected *Agxt1$^{-/-}$* mice with a reporter lentiviral vector. Two different LV doses were tested ($1.4.10^8$ TU/mouse; $5.10^8$ TU/mouse). We also observed a dose-related transduction efficacy of the liver. In low-dose injected mice we obtained a VCN in the liver of 0.11 (0.09-0.49); meanwhile, in the high-dose, we observed a liver VCN of 0.84 (0.73-1.58) (Figure 3). Furthermore, reporter LV transduction efficacy was comparable to the efficacy achieved with the therapeutic lentiviral vector in *Agxt1$^{-/-}$* mice.

Intending to define the percentage of transduced cells achieved after lentiviral vector injection, we also analysed the percentage of positive cells in liver sections of LV.ET.eGFP.142-3pT *Agxt1$^{-/-}$* injected mice (Figure 5). In *Agxt1$^{-/-}$* mice which received $1.4.10^8$ TU/mouse we observed 1.777 (0.399-4.572) % eGFP positive hepatocytes, whereas in *Agxt1$^{-/-}$* mice which received $5.10^8$ TU/mouse we observed 5.5059 (2.109-8.754) % eGFP positive hepatocytes. Representative images of these mice are provided in Figure 5 (D, E). Since reporter lentiviral vector and therapeutic lentiviral vector seems to function in a similar way, it was expected that in *Agxt1$^{-/-}$* mice which received $5.10^8$ TU/mouse of the therapeutic lentiviral vector we had achieved a transduction percentage similar to the observed in *Agxt1$^{-/-}$* mice which received the same dose of reporter lentiviral vector.

Differences in terms of transduction efficacy between *Agxt1$^{-/-}$* and wild-type C57BL/6 injected mice could be due to the genetic background of the mice or to the variability in title determination of LV lab-grade batches.

**EXAMPLE 3: Therapeutic lentiviral vector is efficiently expressed in treated *Agxt1$^{-/-}$* mice liver.**

Although we checked the presence of therapeutic lentiviral vector in the target tissue of therapeutic lentiviral vector *Agxt1$^{-/-}$* injected mice, we wanted to test if this construct was expressing efficiently. We analysed transgene expression in treated *Agxt1$^{-/-}$* mouse livers by RT-qPCR. As reference, two different housekeeping genes were used for this analysis (ms*Tbp* and *msActb)* to determine properly the differences between a physiological mouse *Agxt1* expression in C57BL/6 wild-type mice compared with the *AGXT* expression induced by lentiviral vector integration after the *in vivo* gene therapy strategy in *Agxt1$^{-/-}$* mice.

Therapeutic lentiviral injected mice which received a dose of $2.5.10^8$ TU/mouse showed an *AGXT* expression of 10.88 % (5.62-16.79) referred to ms*Tbp* expression and of 0.1041 % (0.06007-0.2452) referred to *msActb. Agxt1*$^{-/-}$ mice which received a dose of $5.10^8$ TU/mouse resulted in an *AGXT* expression of 12.11 % (4.38-14.48) referred to ms*Tbp* and of 0.1857 % (0.08878-0.2966) referred to *msActb. Agxt1*$^{-/-}$ therapeutic lentiviral vector injected mice with $1{\cdot}10^9$TU/mouse showed an *AGXT* expression of 19.88 % (18.52-28.55) referred to ms*Tbp* and of 0.3088 % (0.1707-0.4418) referred to ms*Actb* (Figure 4). Thus, differences in *AGXT* expression levels among the different therapeutic lentiviral vector *Agxt1*$^{-/-}$ injected mice correlated with the dose received. Non-injected *Agxt1*$^{-/-}$ mice did not show AGXT expression.

Moreover, to study the differences between the achieved *AGXT* expression after therapeutic lentiviral vector injection and the physiological mouse *Agxt1* expression, we analysed this expression in wild-type C57BL/6 mice. Wild type C57BL/6 mice showed a mouse *Agxt1* expression of 8563 (6996-8939) referred to ms*Tbp* and of 340.5 (141-374.5) referred to ms*Actb,* strikingly higher than the one achieved in *Agxt1*$^{-/-}$ treated mice.

**EXAMPLE 4: In vivo lentiviral vector injection into C57BL/6 mice has a reduced off-target transduction.**

To determine the security of the proposed therapeutic strategy, the number of lentiviral vector integrated genomes was analysed in C57BL/6 wild type mice injected with two different doses of the reporter lentiviral vector. Number of LV genome copies integrated in different tissues of injected wild-type C57BL/6 mice was analysed by qPCR. Integration of reporter lentiviral vector genome was only observed in liver, the target tissue, and in lungs, spleen and bone marrow, that could occur due to the route of administration used in this strategy. C57BL/6 mice injected with $1{\cdot}10^8$ TU/mouse showed a VCN of 2.72 (2.33-6.2) in lungs, 0.21 (0.14-0.32) in spleen, and 0.05 (0.04-0.09) in bone marrow. Mice injected with a higher dose of $2.5.10^8$ TU/mouse showed a VCN value of 4.94 (0.185-7.79) in lungs, 0.35 (0.25-0.55) in spleen and 0.13 (0.09-0.18) in bone marrow (Figure 6). Although the presence of integrated vector genomes in these tissues was observed, reporter gene expression was not observed by immunofluorescence analysis, indicating a reduction in the derived risk from off-target transduction. Further, no signs of tumor development were observed.

**EXAMPLE 5: Therapeutic lentiviral vector treated *Agxt1*$^{-/-}$ mice experience a reduction in urine oxalate levels.**

To get a closer phenotype to the human one, Primary Hyperoxaluria Type 1 pathological phenotype analysis in *Agxt1*$^{-/-}$ mouse model requires an ethylene glycol challenge. This challenge generates overloading in oxalate production, which is needed to analyse the pathological phenotype of these mice.

Over the seven days of ethylene glycol challenge, mice were isolated in periods of 24 hours and 24-hour urine was collected. Oxalate concentration in urine was measured at three different time points, before the beginning of the treatment (basal measurement) and at days 3 and 7 of the treatment.

To analyse the pathological phenotype, three different therapeutic lentiviral vector doses were used, $2.5.10^8$TU/mouse, $5.10^8$TU/mouse and $10.10^8$TU/mouse. In addition, we included three different control groups: two negative control groups composed of *Agxt1*$^{-/-}$ mice injected with PBS or with a reporter lentiviral vector (LV.ET.GFP.142-3pT), and a positive control group composed of C57BL/6 wild type mice. All mice groups were subjected to ethylene glycol challenge.

Before treatment, urine oxalate concentration was low in all the conditions. After the beginning of the ethylene glycol challenge an increase in oxalate urine concentration was observed in all the groups. However, the increase observed was higher in untreated *Agxt1*$^{-/-}$ group than in the groups treated with the therapeutic lentiviral vector. Moreover, the dispersion of the data related to this value, a specific feature of this mouse model, was higher in the untreated *Agxt1*$^{-/-}$ mice than in the LV treated *Agxt1*$^{-/-}$ mice. Urine oxalate concentration in *Agxt1*$^{-/-}$ mice which received $5.10^8$ TU/mouse of the therapeutic lentiviral vector was significantly lower at days 3 and 7 than urine oxalate concentration in untreated *Agxt1*$^{-/-}$ mice groups. Significantly, at day 7 there were no significant differences between the therapeutic LV injected *Agxt1*$^{-/-}$ mice with $5.10^8$ TU/mouse and the wildtype C57BL/6 group (Figure 7).

**EXAMPLE 6: Treated mice gain weight during ethylene glycol challenge.**

Another important parameter to follow the pathological phenotype in *Agxt1*$^{-/-}$ mice is the gain or loss of animals' weight during the ethylene glycol challenge. Regarding this parameter, we analysed the weight development in all the mice at the two referred time points and we compared it with the weight measurement at a basal point. We observed a reduction in animal body weight of untreated control groups. This reduction was not observed in the therapeutic LV treated group. Moreover, a gain of weight was achieved (Figure 8). On day 7 after ethylene glycol challenge, the increase in weight in therapeutic LV injected *Agxt1*$^{-/-}$ mice with $5.10^8$ TU/mouse and with $10.10^8$ TU/mouse was not significantly different to the C57BL/6 wild-type mice.

Example 5 and 6 showed that, although the AGT expression observed in the corrected cells is much lower than the observed in normal hepatocytes, the phenotype correction obtained in the treated animals is unexpectedly much higher. Thus, an unexpected phenotype reversion degree was found considering the hypothesis of "more than 40% cells should be corrected to achieve a therapeutic effect". In the most efficient group, percentage of transduction is not higher than 20% (see figure 5 with reporter LV).

**EXAMPLE 7: There is a reduction in nephrocalcinosis development in treated mice.**

One of the most important clinical signs of Primary Hyperoxaluria Type 1 patients is the development of nephrocalcinosis due to the calcium oxalate crystals formation in kidney parenchyma. To determine the damage in the animals' kidneys, we developed a kidney damage score, where 0 represented no damage, 1 represents a mild damage in kidney parenchyma, 2 represents moderate damage which can turn into stage 3 in which we can observe clear signs of nephrocalcinosis, to classify the damage generated because of the ethylene glycol treatment. We classified each animal regarding this score. Regarding this classification, there was a higher percentage of mice, which develop nephrocalcinosis in non-treated groups compared to the treated ones. In therapeutic LV injected *Agxt1*$^{-/-}$ mice with 5.10$^8$ TU/mouse we did not observe any mouse which develops nephrocalcinosis (Figure 9A).

If we simplify this score into none or mild kidney damage (score 0-1) and moderate or severe kidney damage (score 2-3), which represents mice in which kidney damage is progressing due to the ethylene glycol challenge, we could observe than in untreated *Agxt1*$^{-/-}$ mice, the kidney damage progression occurs in a quarter of the half of the cases, as well as it occurs in therapeutic lentiviral vector injected *Agxt1*$^{-/-}$ mice with doses of 2.5.10$^8$ TU/mouse or 10.10$^8$ TU/mouse. Meanwhile in therapeutic lentiviral vector injected *Agxt1*$^{-/-}$ mice with 5.10$^8$ TU/mouse only one out of ten treated mice developed kidney damage progression, and none of the mice injected with the middle dose (5.10$^8$) developed nephrocalcinosis (Figure 9B).

Table 1: Summary of values obtained in Fig. 9B

| | *Agxt1*$^{-/-}$ | | | | | C57BL/6 |
|---|---|---|---|---|---|---|
| LV.eGFP (x10$^8$ TU/mouse) | - | 1-5 | - | - | - | - |
| LV.AGXT (x10$^8$ TU/mouse) | - | - | 2.5 | 5 | 10 | - |
| Score 0-1 | 4/8 | 6/9 | 4/6 | 9/10 | 4/6 | 5/5 |
| Score 2-3 | 4/8 | 3/9 | 2/6 | 1/10 | 2/6 | 0/5 |

**EXAMPLE 8: The use of Cyclosporin H in lentiviral transduction protocol increase the transduction efficacy in a hepatic cell line *in vitro.***

In order to increase the amount of corrected hepatocytes without increasing amount of virus dose, that could be not effective as observed in the previous experiments, we looked for potential transduction enhancers to optimize LV transduction. Among others Cyclosporin H had been described to facilitate the *in vitro* LV transduction in hematopoietic stem cells (HSCs), in which IFITM3, the target protein of CsH, is highly expressed under basal condition. However, no data related to hepatocytes as target cells had been suggested or tested. With the objective of testing this possibility, hepatic HepG2 cells were transduced with two different administration regimens of LV.ET.eGFP.142-3pT and at different CsH concentrations.

In the first transduction protocol, HepG2 cells were subjected to different concentrations of CsH (4, 8, or 16 μM) during the lentiviral vector transduction protocol. Cells were transduced with a lentiviral vector MOI of 0.3. Three technical replicates of each condition were performed. Basal transduction levels were 36.67% and in the different CsH treated samples a transduction percentage of 37.67%, 44.3%, and 51.5% in 4, 8, and 16 μM CsH conditions were observed, which means a fold-increase of control samples of 1.03, 1.2, and 1.4 respectively (Figure 10).

In the second CsH administration regimen, HepG2 cells were subjected to 16 hours of CsH pre-treatment before transduction, in which CsH was also included. The only CsH concentration tested was 16 μM. In this CsH administration regimen, a fold-increase in eGFP positive cells of 2.35 to the control sample was observed (Figure 10). Cells were transduced with an MOI of 0.3 of LV.ET.eGFP.142-3pT and the percentage of transduced cells achieved were 13.2% in the control condition and 31.03% in the CsH treated cells.

The differences observed in transduction levels between the control conditions at the same MOI are due to a probable reduction in LV title due to thaw-freeze cycles. The two different administration regimens were independent experiments.

Thus, remarkably, addition of the CsH as transduction enhancer increases the transduction efficiency of the developed lentiviral vectors *(in vitro)* in a hepatic cell line such as HepG2 even though CsH target gene, IFITM3, is not supposed to be expressed in hepatic lineage.

**Claims**

1. A lentiviral vector, wherein the lentiviral vector comprises a nucleic acid, wherein the nucleic acid comprises from 5' to 3' the following nucleotides:

   a) a 5' long terminal repeat (LTR),
   b) a primer binding site (PBS)
   c) a psi packaging signal,
   d) the Stem-loop 4 (SL4) region of wild-type HIV virus
   e) a Rev responsive element (RRE),
   f) a DNA flap central polypurine tract (cPPT),
   g) an engineered hepatocyte-specific promoter with at least 95%, preferably 98%, sequence identity over the full length of SEQ ID NO 16,
   h) a nucleotide sequence encoding optimized Alanine-Glyoxylate and Serine-Pyruvate Aminotransferase (AGXT-RHEAM) protein with at least 95%, preferably 98%, sequence identity over the full length of SEQ ID NO 29,
   i) a mutated optimized woodchuck hepatitis virus posttranscriptional regulatory element (WPRE) with at least 95%, preferably 98%, sequence identity over the full length of SEQ ID NO 21,
   j) at least a copy of at least one miRNA target sequence, and
   k) a 3' long terminal repeat (LTR),

   wherein the long terminal repeat regions of a) and k) have been rendered substantially transcriptionally inactive by virtue of total or partial deletion in the U3 region of the LTR, and wherein g), i) and j) are operably linked to and regulate the expression of h).

2. The lentiviral vector according to claim 1, wherein the sequence identity of g), h), i) to SEQ ID NOs 16, 29, and 21, respectively, is 100%.

3. The lentiviral vector according to any of claims 1 and 2, wherein the at least a copy of at least one miRNA target sequence of j) consists of four copies of the target sequence of miRNA-142-3 with at least 95%, preferably 98%, sequence identity over the full length of SEQ ID NO 22.

4. The lentiviral vector according to any of claims 1 to 3, wherein the nucleotide comprised in said lentiviral vector further comprises:

l) a DenvRF1 region located between d) and e), and
m) a DenvRF2 region located between e) and f).

5. The lentiviral vector according to any of claims 1 to 4, wherein the full-length nucleotide of said lentiviral has at least 95%, preferably 98% sequence identity with SEQ ID NO 30 (LV.ET.AGXT-RHEAM.142-3pT).

6. The lentiviral vector according to claim 5, wherein the sequence identity with SEQ ID NO 30 is 100%.

7. A substantially pure population of cells transduced with the lentiviral vector as defined in any of claims 1 to 6.

8. A pharmaceutical composition comprising the lentiviral vector as defined in any of claims 1 to 6, or the substantially pure population of cells as defined in claim 7, and a pharmaceutically acceptable carrier or diluent.

9. The lentiviral vector according to any of claims 1 to 6, the substantially pure population of cells according to claim 7, or the pharmaceutical composition according to claim 8, for use as a medicament.

10. The lentiviral vector according to any of claims 1 to 6, the substantially pure population of cells according to claim 7, or the pharmaceutical composition according to claim 8, for use in the treatment of Primary Hyperoxaluria in a subject in need thereof, the method comprising administering to the subject said lentiviral vector, said population of cells, or said pharmaceutical composition.

11. The lentiviral vector, the substantially pure population of cells, or the pharmaceutical composition for use according to claim 10, wherein the Primary Hyperoxaluria is type 1.

**Figure 1**

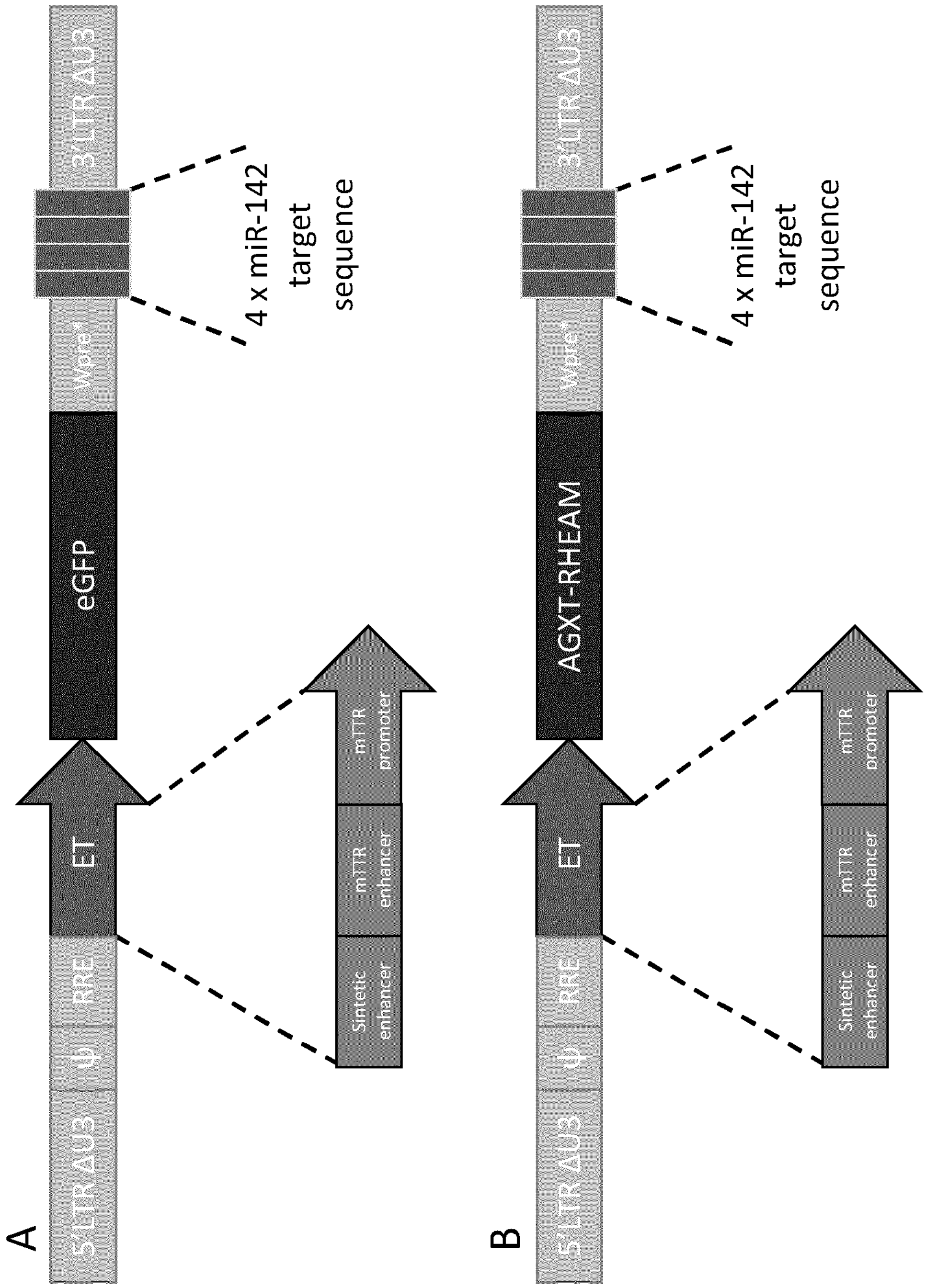
A
5'LTR ΔU3
ψ
RRE
ET
eGFP
Wpre*
3'LTR ΔU3
4 x miR-142
target
sequence
Sintetic
enhancer
mTTR
enhancer
mTTR
promoter
B
5'LTR ΔU3
ψ
RRE
ET
AGXT-RHEAM
Wpre*
3'LTR ΔU3
4 x miR-142
target
sequence
Sintetic
enhancer
mTTR
enhancer
mTTR
promoter

Figure 2

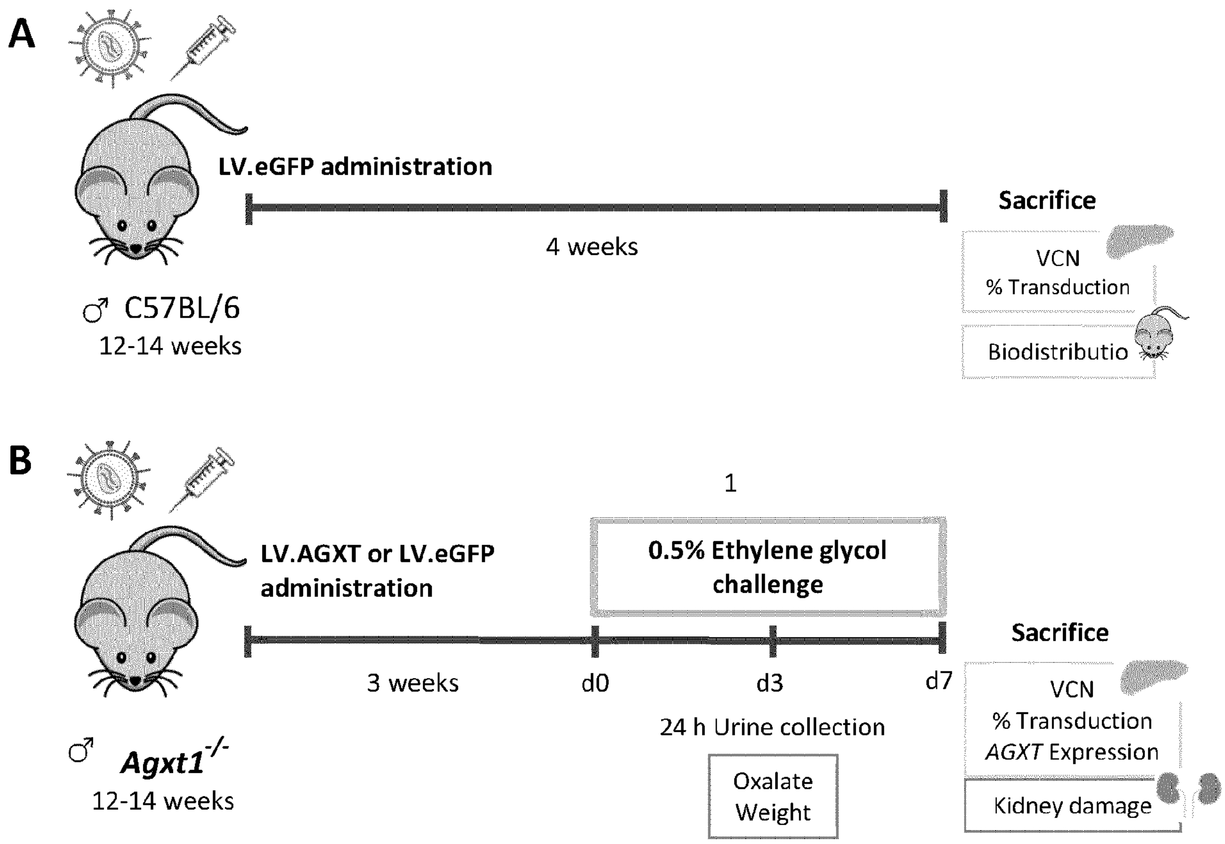


Figure 3

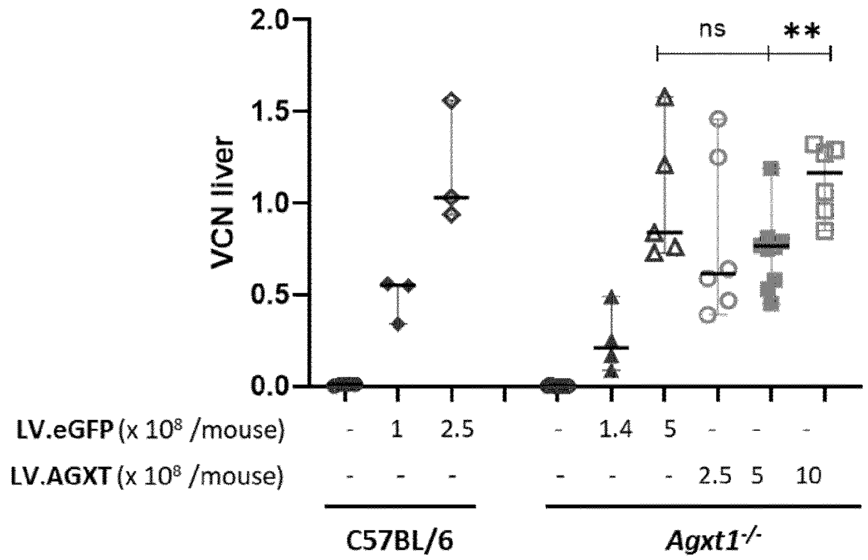

Figure 4

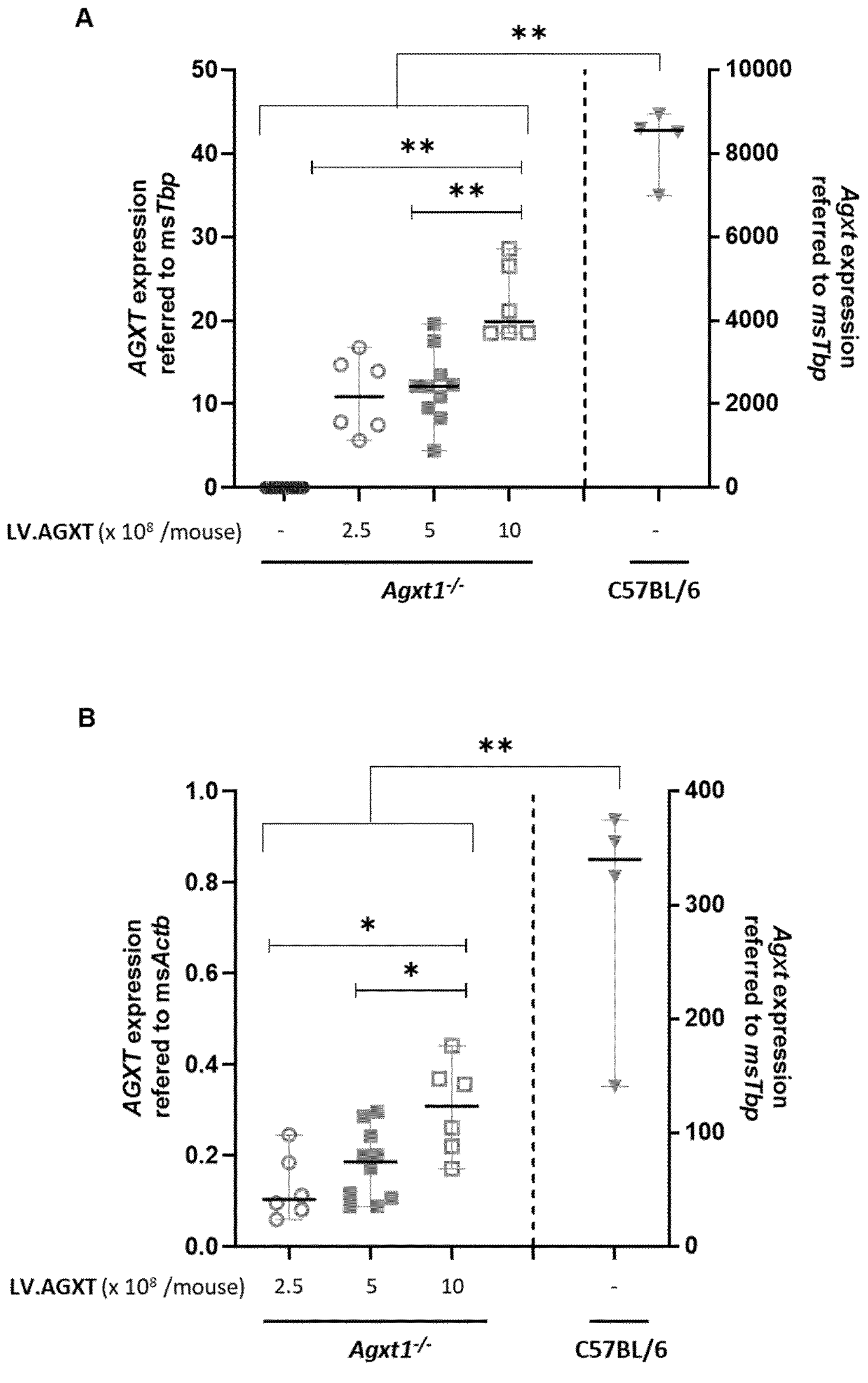
A
B
**
*
AGXT expression referred to msTbp
Agxt expression referred to msTbp
AGXT expression refered to msActb
0
10
20
30
40
50
0
2000
4000
6000
8000
10000
0.0
0.2
0.4
0.6
0.8
1.0
0
100
200
300
400
LV.AGXT (x 10⁸ /mouse)
-
2.5
5
10
Agxt1-/-
C57BL/6

Figure 5

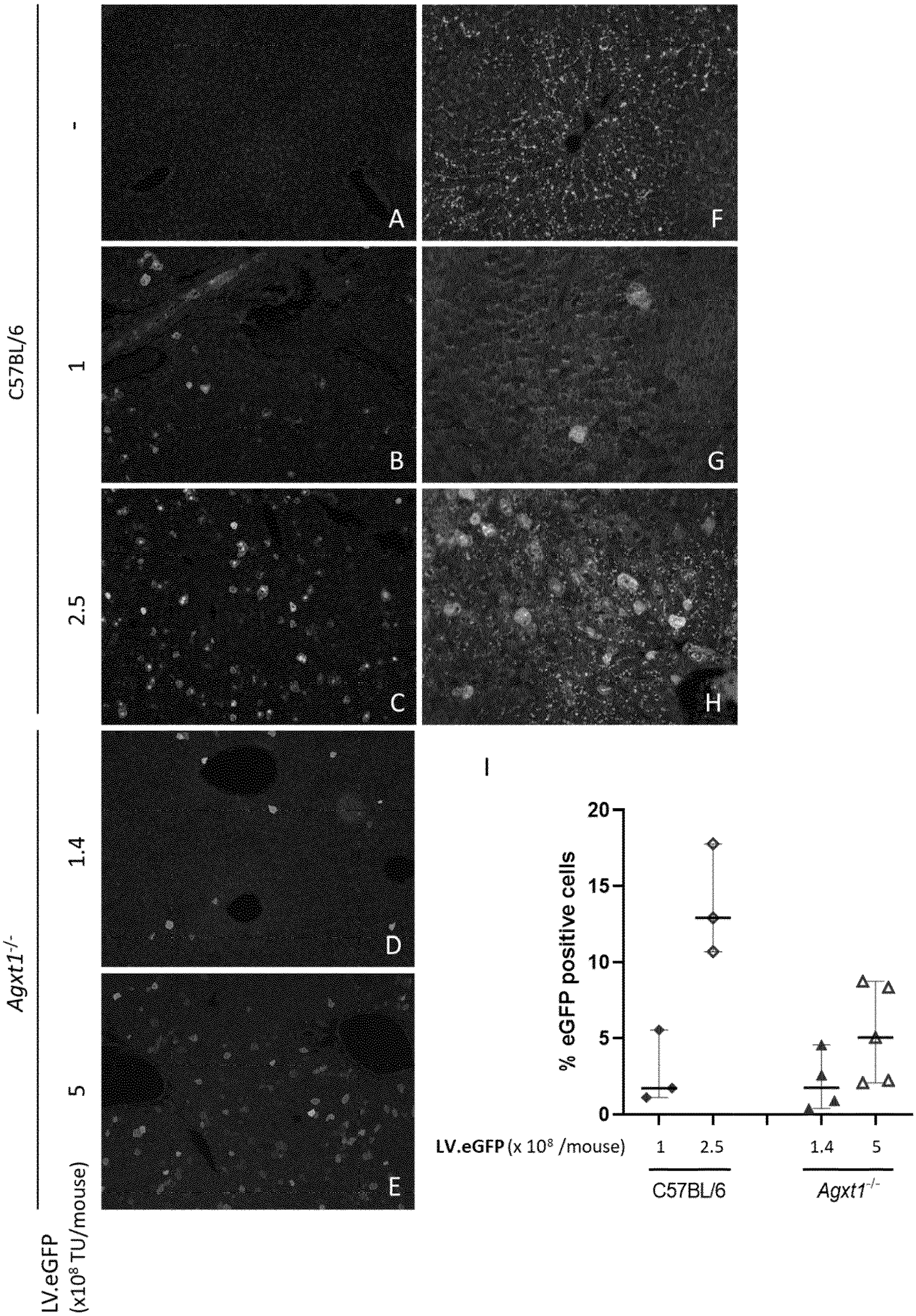
C57BL/6
Agxt1-/-
LV.eGFP (x10^8 TU/mouse)
-
1
2.5
1.4
5
A
B
C
D
E
F
G
H
I
% eGFP positive cells
20
15
10
5
0
LV.eGFP (x 10^8 /mouse) 1 2.5 1.4 5
C57BL/6
Agxt1-/-

Figure 6

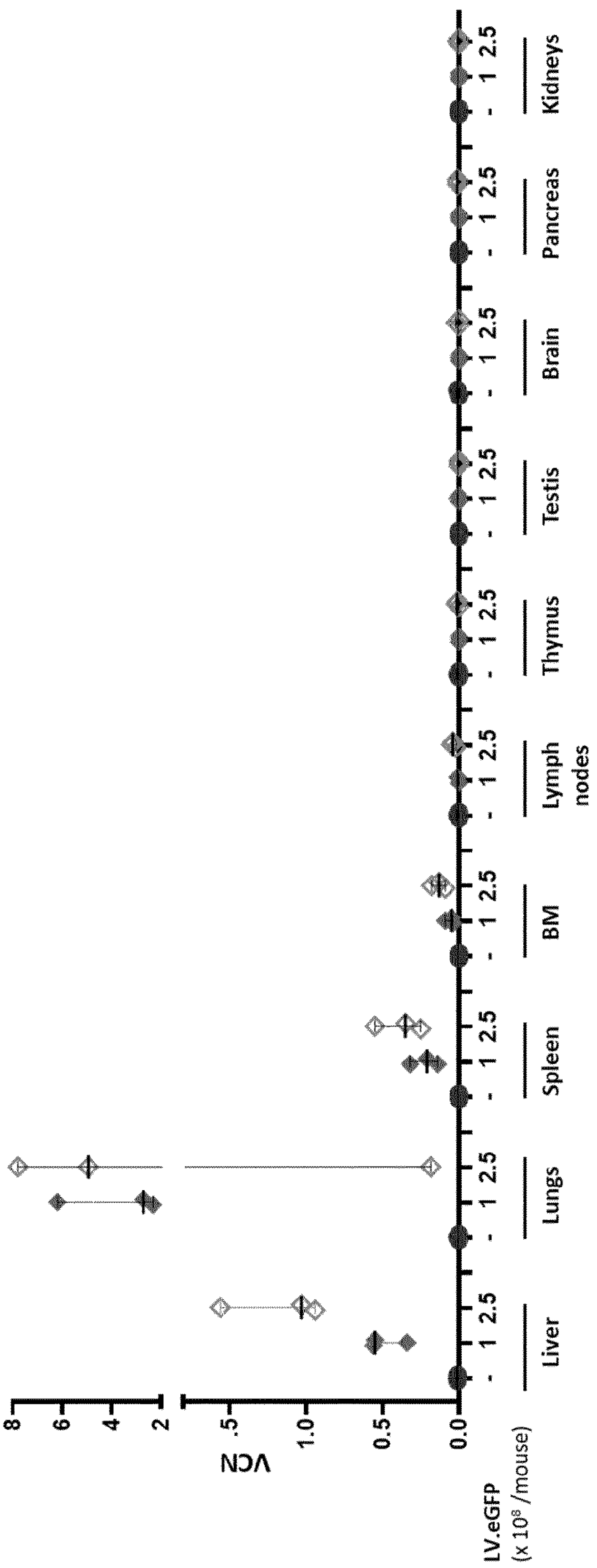

VCN
8
6
4
2
.5
1.0
0.5
0.0
LV.eGFP
(x 10^8 /mouse)
- 1 2.5
Liver
Lungs
Spleen
BM
Lymph nodes
Thymus
Testis
Brain
Pancreas
Kidneys

Figure 7

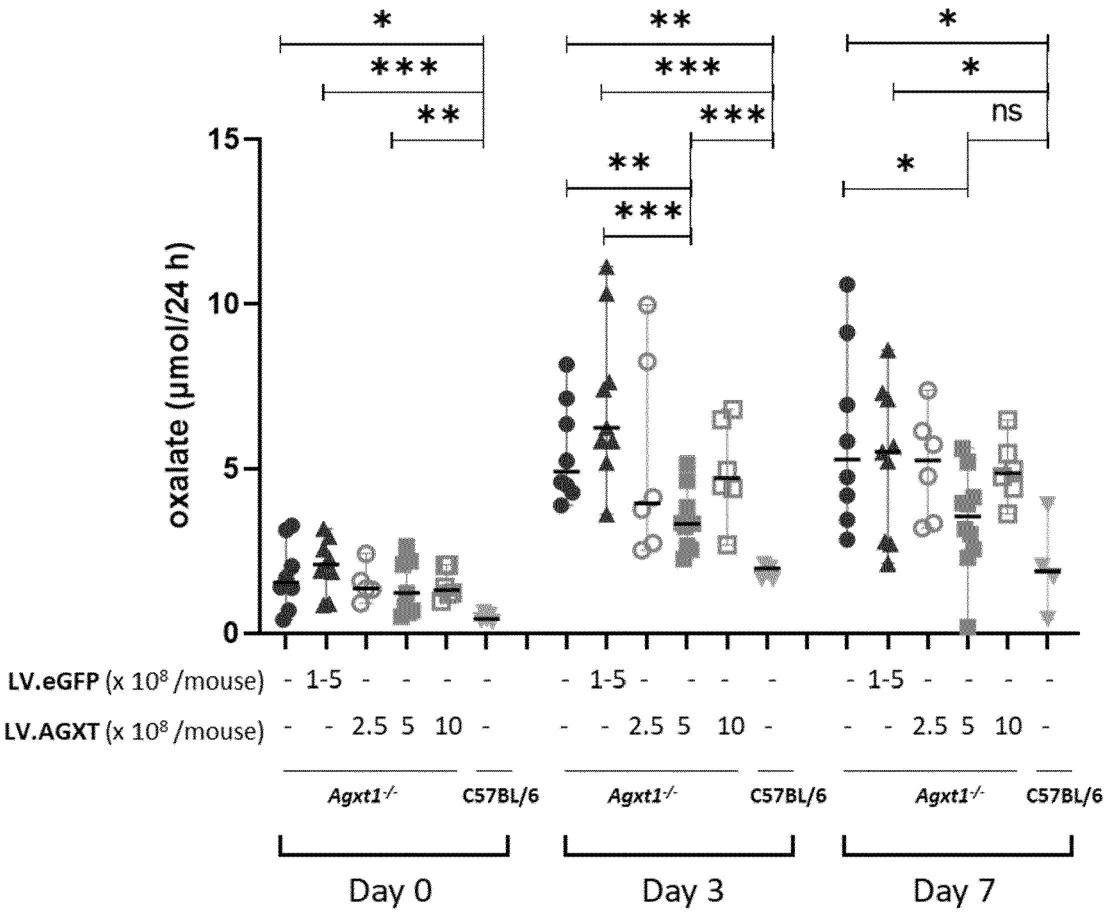
*
***
**
**
***
***
**
***
*
*
ns
*
15
10
5
0
oxalate (µmol/24 h)
LV.eGFP (x 10⁸ /mouse) - 1-5 - - - - - 1-5 - - - - - 1-5 - - - -
LV.AGXT (x 10⁸ /mouse) - - 2.5 5 10 - - - 2.5 5 10 - - - 2.5 5 10 -
Agxt1-/-
C57BL/6
Agxt1-/-
C57BL/6
Agxt1-/-
C57BL/6
Day 0
Day 3
Day 7

Figure 8

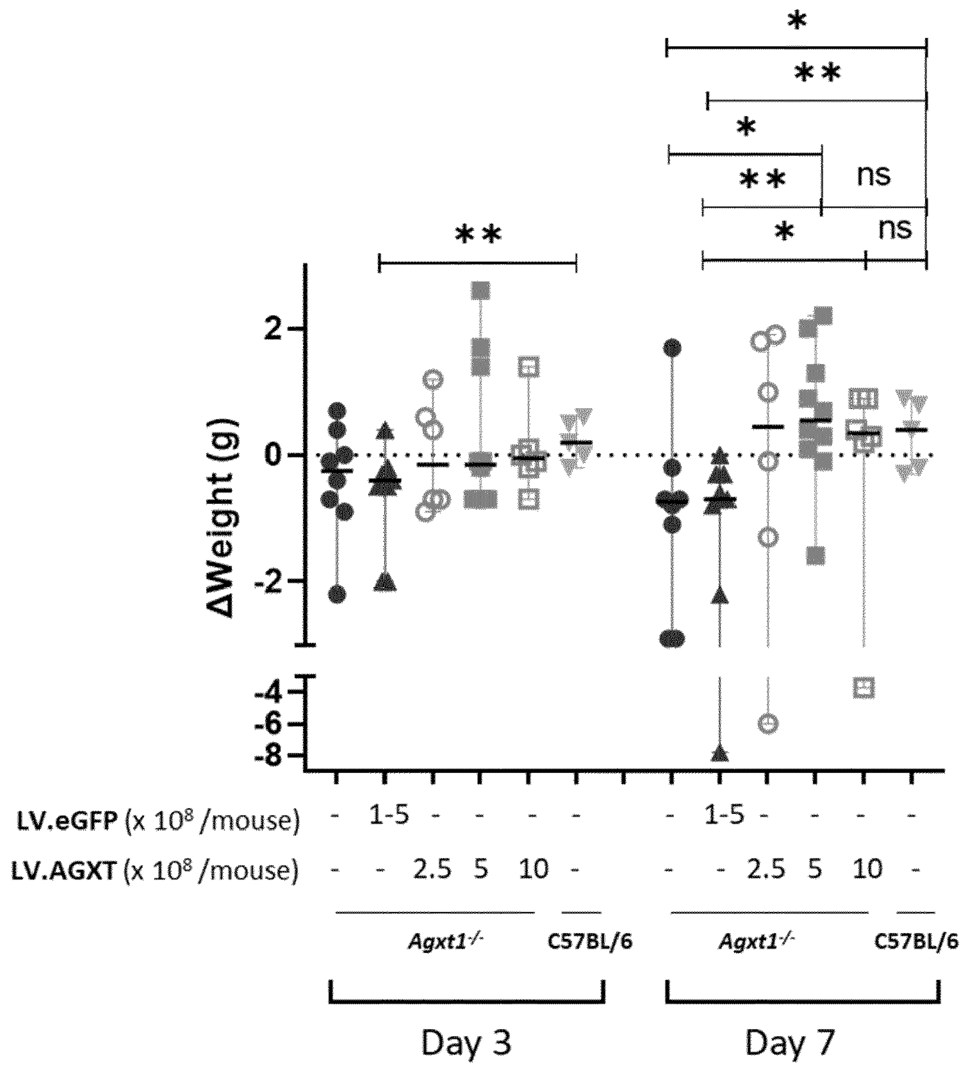
*
**
*
**
ns
*
ns
**
ΔWeight (g)
2
0
-2
-4
-6
-8
LV.eGFP (x 10⁸ /mouse) - 1-5 - - - - - 1-5 - - - -
LV.AGXT (x 10⁸ /mouse) - - 2.5 5 10 - - - 2.5 5 10 -
Agxt1-/-
C57BL/6
Agxt1-/-
C57BL/6
Day 3
Day 7

**Figure 9**

A

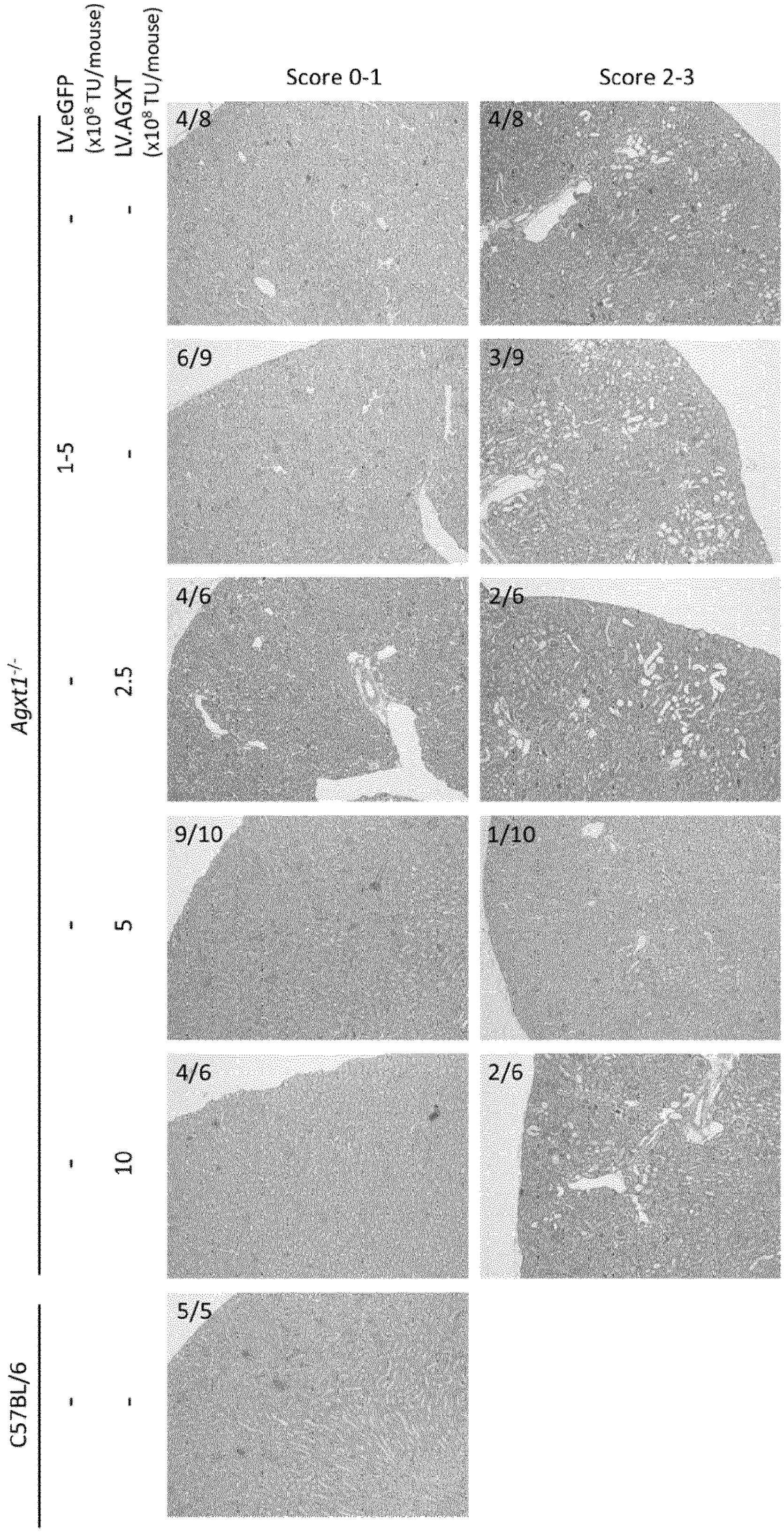

LV.eGFP (x10^8 TU/mouse)
LV.AGXT (x10^8 TU/mouse)
Score 0-1
Score 2-3
4/8
4/8
6/9
3/9
4/6
2/6
9/10
1/10
4/6
2/6
5/5
Agxt1-/-
C57BL/6
-
1-5
2.5
5
10

Figure 9 (cont.)

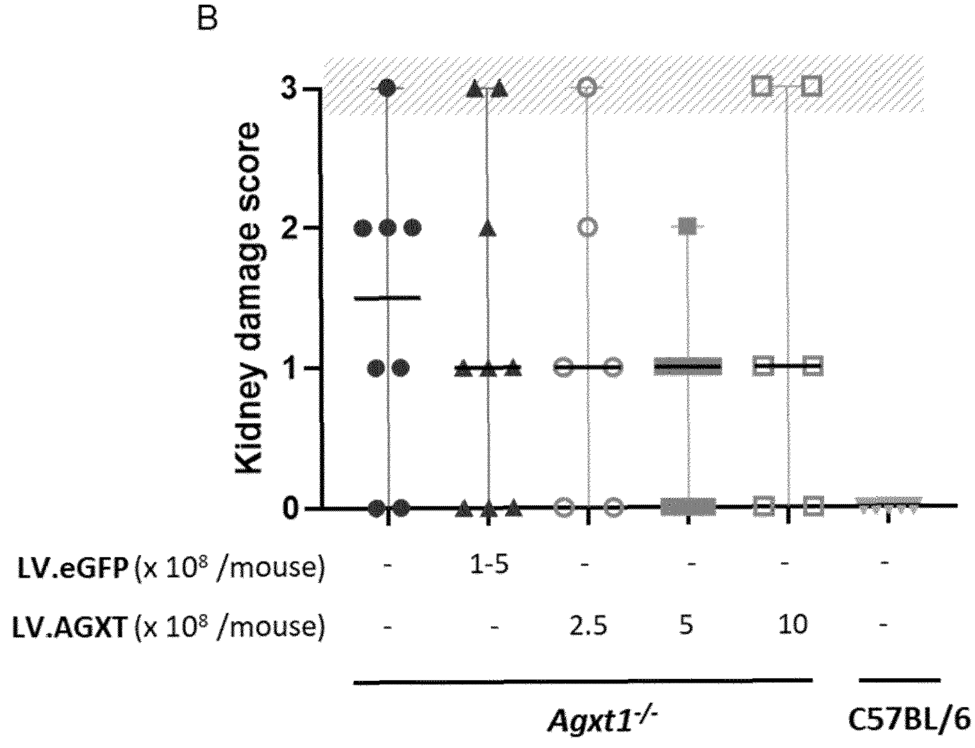

Figure 10

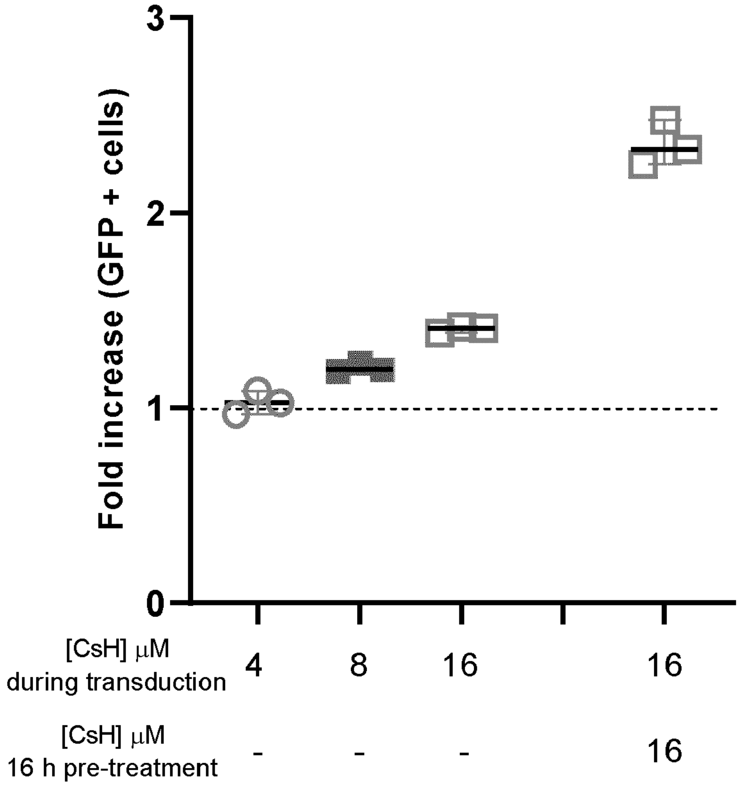

Fold increase (GFP + cells)
3
2
1
0
[CsH] µM
during transduction
4
8
16
16
[CsH] µM
16 h pre-treatment
-
-
-
16

Figure 11

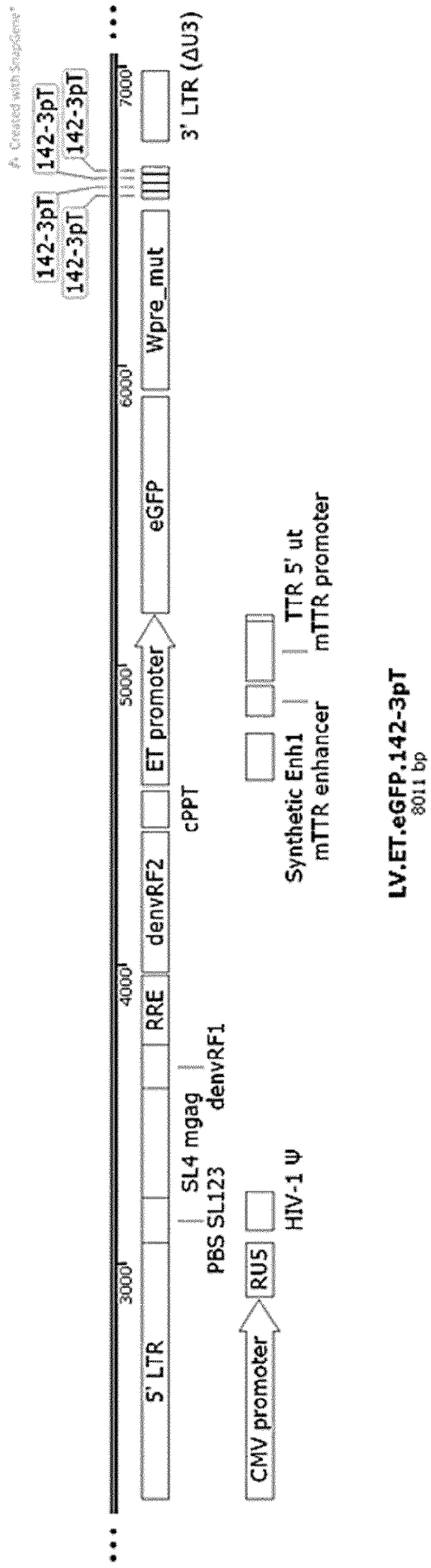
142-3pT
142-3pT
142-3pT
142-3pT
7000
3' LTR (ΔU3)
6000
Wpre_mut
eGFP
5000
ET promoter
cPPT
denvRF2
4000
RRE
denvRF1
SL4 mgag
PBS SL123
HIV-1 Ψ
3000
RU5
5' LTR
CMV promoter
TTR 5' ut
mTTR promoter
Synthetic Enh1
mTTR enhancer
LV.ET.eGFP.142-3pT
8011 bp

Figure 12

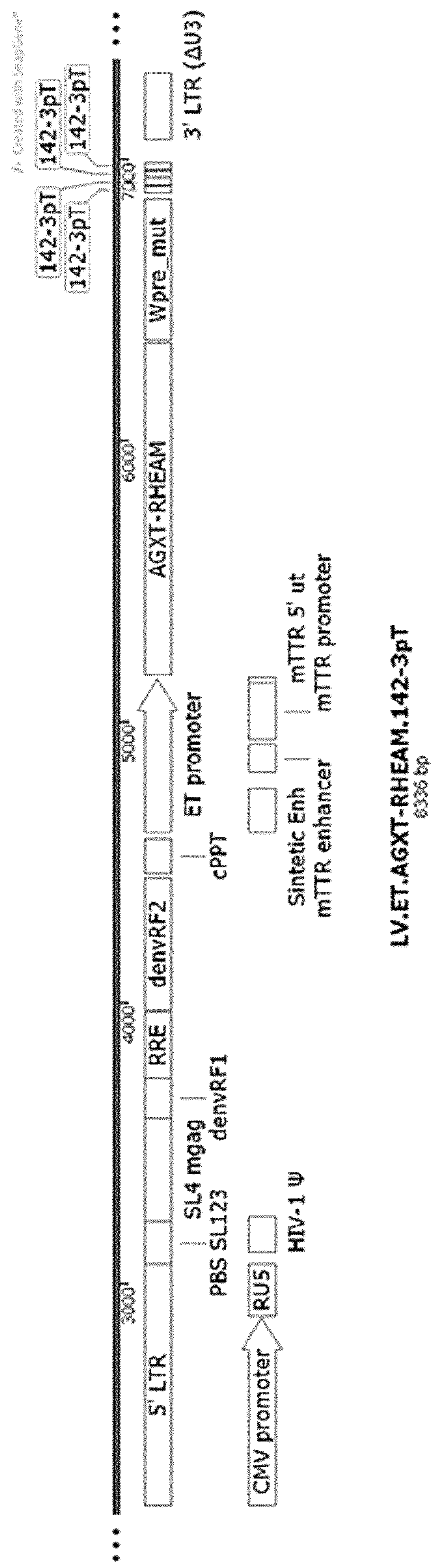
Created with SnapGene
142-3pT
142-3pT
142-3pT
142-3pT
3' LTR (ΔU3)
7000
Wpre_mut
6000
AGXT-RHEAM
5000
ET promoter
cPPT
denvRF2
4000
RRE
denvRF1
SL4 mgag
PBS SL123
HIV-1 Ψ
3000
RU5
5' LTR
CMV promoter
Sintetic Enh
mTTR enhancer
mTTR 5' ut
mTTR promoter
LV.ET.AGXT-RHEAM.142-3pT
6336 bp

Figure 13

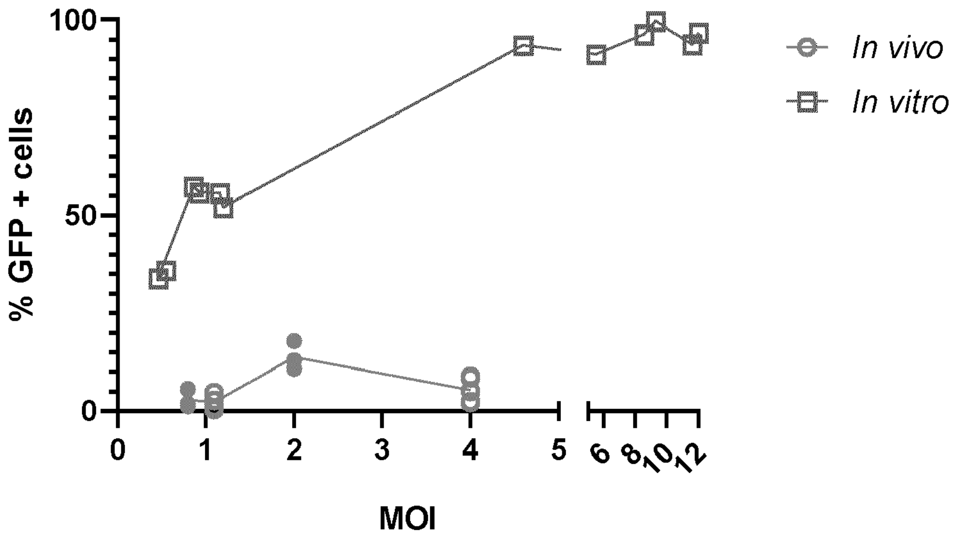

Europäisches Patentamt
European Patent Office
Office européen des brevets

# EUROPEAN SEARCH REPORT

Application Number
EP 21 38 2363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KUKREJA ANJLI ET AL: "Systemic Alanine Glyoxylate Aminotransferase mRNA Improves Glyoxylate Metabolism in a Mouse Model of Primary Hyperoxaluria Type 1", NUCLEIC ACID THERAPEUTICS, vol. 29, no. 2, 1 April 2019 (2019-04-01), pages 104-113, XP055849763, US ISSN: 2159-3337, DOI: 10.1089/nat.2018.0740 Retrieved from the Internet: URL:https://www.liebertpub.com/doi/pdf/10.1089/nat.2018.0740> * the whole document * ----- | 1-11 | INV. C12N15/867 A61K48/00 A61P1/16 C12N9/10 |
| A | ESTÈVE JULIE ET AL: "Generation of induced pluripotent stem cells-derived hepatocyte-like cells forex vivogene therapy of primary hyperoxaluria type 1", STEM CELL RESEARCH, vol. 38, 101467, 21 May 2019 (2019-05-21), XP085726998, ISSN: 1873-5061, DOI: 10.1016/J.SCR.2019.101467 * see the whole document, in particular sections 2.8, 3.2 and 3.3; figure 4 * ----- | 1-11 | |
| A | WO 2019/152692 A1 (BIOVERATIV THERAPEUTICS INC [US]) 8 August 2019 (2019-08-08) * figures 5,9 * ----- | 1-11 | TECHNICAL FIELDS SEARCHED (IPC) C12N A61K |
| A | EP 2 384 200 A1 (AMSTERDAM MOLECULAR THERAPEUTICS AMT IP B V [NL]) 9 November 2011 (2011-11-09) * the whole document * ----- | 1-11 | |
| | -/-- | | |

The present search report has been drawn up for all claims

1

| Place of search | Date of completion of the search | Examiner |
|---|---|---|
| Munich | 12 October 2021 | Brenz Verca, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 21 38 2363

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MESA-TORRES NOEL: "The consensus-based approach for gene/enzyme replacement therapies and crystallization strategies: the case of human alanine-glyoxylate aminotransferase", BIOCHEMICAL JOURNAL, vol. 462, no. Part 3, 15 September 2014 (2014-09-15), pages 453-463, XP009530546, DOI: 10.1042/BJ20140250 * the whole document * ----- | 1-11 | |
| | | | TECHNICAL FIELDS SEARCHED (IPC) |

1

The present search report has been drawn up for all claims

| Place of search | Date of completion of the search | Examiner |
|---|---|---|
| Munich | 12 October 2021 | Brenz Verca, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 38 2363

This annex lists the patent family members relating to the patent documents cited in the above-mentioned European search report. The members are as contained in the European Patent Office EDP file on
The European Patent Office is in no way liable for these particulars which are merely given for the purpose of information.

12-10-2021

| Patent document cited in search report | Publication date | Patent family member(s) | Publication date |
|---|---|---|---|
| WO 2019152692 A1 | 08-08-2019 | AU 2019215063 A1 | 03-09-2020 |
| | | BR 112020015228 A2 | 29-12-2020 |
| | | CA 3090136 A1 | 08-08-2019 |
| | | CN 111918674 A | 10-11-2020 |
| | | CO 2020010376 A2 | 29-01-2021 |
| | | EP 3746136 A1 | 09-12-2020 |
| | | JP 2021512126 A | 13-05-2021 |
| | | KR 20200118089 A | 14-10-2020 |
| | | SG 11202007114V A | 28-08-2020 |
| | | TW 201946929 A | 16-12-2019 |
| | | US 2021038744 A1 | 11-02-2021 |
| | | WO 2019152692 A1 | 08-08-2019 |
| EP 2384200 A1 | 09-11-2011 | CA 2750811 A1 | 05-08-2010 |
| | | EP 2384200 A1 | 09-11-2011 |
| | | ES 2429142 T3 | 13-11-2013 |
| | | JP 5728389 B2 | 03-06-2015 |
| | | JP 2012516325 A | 19-07-2012 |
| | | WO 2010087709 A1 | 05-08-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **JOHNSON ; 2021 et al.** *Mol Therapy*
- **ALTSCHUL et al.** *J Mol Biol.,* 1990, vol. 215 (3), 403-10
- Remington's Pharmaceutical Sciences. 1980

SEQUENCE LISTING

<110> Centro de Investigaciones Energéticas, Medioambientales y Tecnológicas, O.A., M.P. (CIEMAT)
Consorcio Centro de Investigación Biomédica en Red, M.P. (CIBER)
Fundación Instituto de Investigación Sanitaria Fundación Jiménez Díaz (FIIS-FJD).

<120> IN VIVO LENTIVIRAL GENE THERAPY FOR THE TREATMENT OF PRIMARY HYPEROXALURIA TYPE 1

<130> 905995

<160> 51

<170> BiSSAP 1.3.6

<210> 1
<211> 30
<212> DNA
<213> Artificial Sequence

<220>
<223> forward

<400> 1
attggctagc atggtgagca agggcgagga 30

<210> 2
<211> 30
<212> DNA
<213> Artificial Sequence

<220>
<223> Reverse

<400> 2
cattgtcgac ttacttgtac agctcgtcca 30

<210> 3
<211> 23
<212> RNA
<213> Artificial Sequence

<220>
<223> mature human microRNA 142-3p

<400> 3
uguaguguuu ccuacuuuau gga 23

<210> 4
<211> 856
<212> DNA
<213> Artificial Sequence

<220>
<223> 5′ LTR

<400> 4
tggccattgc atacgttgta tccatatcat aatatgtaca tttatattgg ctcatgtcca 60

acattaccgc catgttgaca ttgattattg actagttatt aatagtaatc aattacgggg 120

tcattagttc atagcccata tatggagttc cgcgttacat aacttacggt aaatggcccg 180

cctggctgac cgcccaacga cccccgccca ttgacgtcaa taatgacgta tgttcccata 240

gtaacgccaa tagggacttt ccattgacgt caatgggtgg agtatttacg gtaaactgcc 300

cacttggcag tacatcaagt gtatcatatg ccaagtacgc cccctattga cgtcaatgac 360

ggtaaatggc ccgcctggca ttatgcccag tacatgacct tatgggactt tcctacttgg 420

cagtacatct acgtattagt catcgctatt accatggtga tgcggttttg gcagtacatc 480

aatgggcgtg gatagcggtt tgactcacgg ggatttccaa gtctccaccc cattgacgtc 540

aatgggagtt tgttttggca ccaaaatcaa cgggactttc caaaatgtcg taacaactcc 600

gccccattga cgcaaatggg cggtaggcgt gtacggtggg aggtctatat aagcagagct 660

cgtttagtga accggggtct ctctggttag accagatctg agcctgggag ctctctggct 720

aactagggaa cccactgctt aagcctcaat aaagcttgcc ttgagtgctt caagtagtgt 780

gtgcccgtct gttgtgtgac tctggtaact agagatccct cagacccttt tagtcagtgt 840

ggaaaatctc tagcag 856

<210> 5
<211> 673
<212> DNA
<213> Artificial Sequence

<220>
<223> Chimeric CMV promoter

<400> 5
tggccattgc atacgttgta tccatatcat aatatgtaca tttatattgg ctcatgtcca 60

acattaccgc catgttgaca ttgattattg actagttatt aatagtaatc aattacgggg 120

tcattagttc atagcccata tatggagttc cgcgttacat aacttacggt aaatggcccg 180

cctggctgac cgcccaacga cccccgccca ttgacgtcaa taatgacgta tgttcccata 240

gtaacgccaa tagggacttt ccattgacgt caatgggtgg agtatttacg gtaaactgcc 300

cacttggcag tacatcaagt gtatcatatg ccaagtacgc cccctattga cgtcaatgac 360

ggtaaatggc ccgcctggca ttatgcccag tacatgacct tatgggactt tcctacttgg 420

cagtacatct acgtattagt catcgctatt accatggtga tgcggttttg gcagtacatc 480

aatgggcgtg gatagcggtt tgactcacgg ggatttccaa gtctccaccc cattgacgtc 540

aatgggagtt tgttttggca ccaaaatcaa cgggactttc caaaatgtcg taacaactcc 600

gccccattga cgcaaatggg cggtaggcgt gtacggtggg aggtctatat aagcagagct 660

cgtttagtga acc 673

<210> 6
<211> 380
<212> DNA
<213> Artificial Sequence

<220>
<223> CMV enhancer

<400> 6
gacattgatt attgactagt tattaatagt aatcaattac ggggtcatta gttcatagcc 60

catatatgga gttccgcgtt acataactta cggtaaatgg cccgcctggc tgaccgccca 120

acgacccccg cccattgacg tcaataatga cgtatgttcc catagtaacg ccaataggga 180

ctttccattg acgtcaatgg gtggagtatt tacggtaaac tgcccacttg gcagtacatc 240

aagtgtatca tatgccaagt acgcccccta ttgacgtcaa tgacggtaaa tggcccgcct 300

ggcattatgc ccagtacatg accttatggg actttcctac ttggcagtac atctacgtat 360

tagtcatcgc tattaccatg 380

<210> 7
<211> 204
<212> DNA
<213> Artificial Sequence

<220>
<223> CMV promoter

<400> 7
gtgatgcggt tttggcagta catcaatggg cgtggatagc ggtttgactc acggggattt 60

ccaagtctcc accccattga cgtcaatggg agtttgtttt ggcaccaaaa tcaacgggac 120

tttccaaaat gtcgtaacaa ctccgcccca ttgacgcaaa tgggcggtag gcgtgtacgg 180

tgggaggtct atataagcag agct 204

<210> 8
<211> 182
<212> DNA
<213> Artificial Sequence

<220>
<223> RU5

<400> 8
gggtctctct ggttagacca gatctgagcc tgggagctct ctggctaact agggaaccca 60

ctgcttaagc ctcaataaag cttgccttga gtgcttcaag tagtgtgtgc ccgtctgttg 120

tgtgactctg gtaactagag atccctcaga cccttttagt cagtgtggaa aatctctagc 180

ag 182

<210> 9
<211> 154
<212> DNA
<213> Artificial Sequence

<220>
<223> PBS SL23 SL123

<400> 9
tggcgcccga acagggactt gaaagcgaaa gggaaaccag aggagctctc tcgacgcagg 60

actcggcttg ctgaagcgcg cacggcaaga ggcgaggggc ggcgactggt gagtacgcca 120

aaaattttga ctagcggagg ctagaaggag agag 154

<210> 10
<211> 126
<212> DNA
<213> Artificial Sequence

<220>
<223> packaging signal

<400> 10
ctctctcgac gcaggactcg gcttgctgaa gcgcgcacgg caagaggcga ggggcggcga 60

ctggtgagta cgccaaaaat tttgactagc ggaggctaga aggagagaga tgggtgcgag 120

agcgtc 126

<210> 11

<211> 365
<212> DNA
<213> Artificial Sequence

<220>
<223> SL4mgag

<400> 11
atgggtgcga gagcgtcagt attaagcggg ggagaattag atcgcgatgg gaaaaaattc 60

ggttaaggcc agggggaaag aaaaaatata aattaaaaca tatagtatgg gcaagcaggg 120

agctagaacg attcgcagtt aatcctggcc tgttagaaac atcagaaggc tgtagacaaa 180

tactgggaca gctacaacca tcccttcaga caggatcaga agaacttaga tcattatata 240

atacagtagc aaccctctat tgtgtgcatc aaaggataga gataaaagac accaaggaag 300

ctttagacaa gatagaggaa gagcaaaaca aaagtaagac caccgcacag caagcggccg 360

ctgat 365

<210> 12
<211> 146
<212> DNA
<213> Artificial Sequence

<220>
<223> DenvRF1

<400> 12
tcttcagacc tggaggagga gatatgaggg acaattggag aagtgaatta tataaatata 60

aagtagtaaa aattgaacca ttaggagtag cacccaccaa ggcaaagaga agagtggtgc 120

agagagaaaa aagagcagtg ggaata 146

<210> 13
<211> 234
<212> DNA
<213> Artificial Sequence

<220>
<223> RRE

<400> 13
aggagctttg ttccttgggt tcttgggagc agcaggaagc actatgggcg cagcgtcaat 60

gacgctgacg gtacaggcca gacaattatt gtctggtata gtgcagcagc agaacaattt 120

gctgagggct attgaggcgc aacagcatct gttgcaactc acagtctggg gcatcaagca 180

gctccaggca agaatcctgg ctgtggaaag atacctaaag gatcaacagc tcct 234

<210> 14
<211> 468
<212> DNA
<213> Artificial Sequence

<220>
<223> DenvRF2

<400> 14
gggttgctct ggaaaactca tttgcaccac tgctgtgcct tggaatgcta gttggagtaa 60

taaatctctg gaacagattt ggaatcacac gacctggatg gagtgggaca gagaaattaa 120

caattacaca agcttaatac actccttaat tgaagaatcg caaaaccagc aagaaaagaa 180

tgaacaagaa ttattggaat tagataaatg ggcaagtttg tggaattggt ttaacataac 240

aaattggctg tggtatataa aattattcat aatgatagta ggaggcttgg taggtttaag 300

aatagttttt gctgtacttt ctatagtgaa tagagttagg cagggatatt caccattatc 360

gtttcagacc cacctcccaa ccccgagggg acccgacagg cccgaaggaa tagaagaaga 420

aggtggagag agagacagag acagatccat tcgattagtg aacggatc 468

<210> 15
<211> 118
<212> DNA
<213> Artificial Sequence

<220>
<223> cPPT

<400> 15
ttttaaaaga aaagggggga ttggggggta cagtgcaggg gaaagaatag tagacataat 60

agcaacagac atacaaacta aagaattaca aaaacaaatt acaaaaattc aaaatttt 118

<210> 16
<211> 553
<212> DNA
<213> Artificial Sequence

<220>
<223> ET Promoter

<400> 16
cgcgagttaa taattaccag cgcgggccaa ataaataatc cgcgaggggc aggtgacgtt 60

tgcccagcgc gcgctggtaa ttattaacct cgcgaatatt gattcgaggc cgcgattgcc 120

gcaatcgcga ggggcaggtg acctttgccc agcgcgcgtt cgccccgccc cggacggtat 180

cgataagctt aggagcttgg gctgcaggtc gagggcactg ggaggatgtt gagtaagatg 240

gaaaactact gatgaccctt gcagagacag agtattagga catgtttgaa caggggccgg 300

gcgatcagca ggtagctcta gaggatcccc gtctgtctgc acatttcgta gagcgagtgt 360

tccgatactc taatctccct aggcaaggtt catatttgtg taggttactt attctccttt 420

tgttgactaa gtcaataatc agaatcagca ggtttggagt cagcttggca gggatcagca 480

gcctgggttg gaaggagggg gtataaaagc cccttcacca ggagaagccg tcacacagat 540

ccacaagctc ctg 553

<210> 17
<211> 158
<212> DNA
<213> Artificial Sequence

<220>
<223> Synthetic enhancer

<400> 17
cgcgagttaa taattaccag cgcgggccaa ataaataatc cgcgaggggc aggtgacgtt 60

tgcccagcgc gcgctggtaa ttattaacct cgcgaatatt gattcgaggc cgcgattgcc 120

gcaatcgcga ggggcaggtg acctttgccc agcgcgcg 158

<210> 18
<211> 100
<212> DNA
<213> Artificial Sequence

<220>
<223> mTTR enhancer

<400> 18
cactgggagg atgttgagta agatggaaaa ctactgatga cccttgcaga gacagagtat 60

taggacatgt ttgaacaggg gccgggcgat cagcaggtag 100

<210> 19
<211> 202
<212> DNA
<213> Artificial Sequence

<220>
<223> mTTR promoter

<400> 19
gtctgtctgc acatttcgta gagcgagtgt tccgatactc taatctccct aggcaaggtt 60

catatttgtg taggttactt attctccttt tgttgactaa gtcaataatc agaatcagca 120

ggtttggagt cagcttggca gggatcagca gcctgggttg gaaggagggg gtataaaagc 180

cccttcacca ggagaagccg tc 202

<210> 20
<211> 21
<212> DNA
<213> Artificial Sequence

<220>
<223> mTTR 5' UT

<400> 20
acacagatcc acaagctcct g 21

<210> 21
<211> 501
<212> DNA
<213> Artificial Sequence

<220>
<223> Mutated WPRE

<400> 21
caacctctgg attacaaaat ttgtgaaaga ttgactggta ttcttaacta tgttgctcct 60

tttacgctat gtggatacgc tgctttaatg cctttgtatc atgctattgc ttcccgtatg 120

gctttcattt tctcctcctt gtataaatcc tggttgctgt ctctttatga ggagttgtgg 180

cccgttgtca ggcaacgtgg cgtggtgtgc actgtgtttg ctgacgcaac ccccactggt 240

tggggcattg ccaccacctg tcagctcctt tccgggactt tcgctttccc cctccctatt 300

gccacggcgg aactcatcgc cgcctgcctt gcccgctgct ggacaggggc tcggctgttg 360

ggcactgaca attccgtggt gttgtcgggg aaatcatcgt cctttccttg gctgctcgcc 420

tgtgttgcca cctggattct gcgcgggacg tccttctgct acgtcccttc ggccctcaat 480

ccagcggacc ttccttcccg c 501

<210> 22
<211> 23
<212> DNA
<213> Artificial Sequence

<220>
<223> miRNA-142-3pT

<400> 22
tccataaagt aggaaacact aca 23

<210> 23
<211> 234
<212> DNA
<213> Artificial Sequence

<220>
<223> 3’ LTR: DR3RU5

<400> 23
tggaagggct aattcactcc caacgaagac aagatctgct ttttgcttgt actgggtctc 60

tctggttaga ccagatctga gcctgggagc tctctggcta actagggaac ccactgctta 120

agcctcaata aagcttgcct tgagtgcttc aagtagtgtg tgcccgtctg ttgtgtgact 180

ctggtaacta gagatccctc agaccctttt agtcagtgtg gaaaatctct agca 234

<210> 24
<211> 53
<212> DNA
<213> Artificial Sequence

<220>
<223> ΔU3

<400> 24
tggaagggct aattcactcc caacgaagac aagatctgct ttttgcttgt act 53

<210> 25
<211> 182
<212> DNA
<213> Artificial Sequence

<220>
<223> RU5

<400> 25
gggtctctct ggttagacca gatctgagcc tgggagctct ctggctaact agggaaccca 60

ctgcttaagc ctcaataaag cttgccttga gtgcttcaag tagtgtgtgc ccgtctgttg 120

tgtgactctg gtaactagag atccctcaga cccttttagt cagtgtggaa aatctctagc 180

ag 182

<210> 26
<211> 4769
<212> DNA
<213> Artificial Sequence

<220>
<223> LV.ET.eGFP.142-3pT sequence

<400> 26
tggccattgc atacgttgta tccatatcat aatatgtaca tttatattgg ctcatgtcca 60

acattaccgc catgttgaca ttgattattg actagttatt aatagtaatc aattacgggg 120

tcattagttc atagcccata tatggagttc cgcgttacat aacttacggt aaatggcccg 180

cctggctgac cgcccaacga cccccgccca ttgacgtcaa taatgacgta tgttcccata 240

gtaacgccaa tagggacttt ccattgacgt caatgggtgg agtatttacg gtaaactgcc 300

cacttggcag tacatcaagt gtatcatatg ccaagtacgc cccctattga cgtcaatgac 360

ggtaaatggc ccgcctggca ttatgcccag tacatgacct tatgggactt tcctacttgg 420

cagtacatct acgtattagt catcgctatt accatggtga tgcggttttg gcagtacatc 480

aatgggcgtg gatagcggtt tgactcacgg ggatttccaa gtctccaccc cattgacgtc 540

aatgggagtt tgttttggca ccaaaatcaa cgggactttc caaaatgtcg taacaactcc 600

gccccattga cgcaaatggg cggtaggcgt gtacggtggg aggtctatat aagcagagct 660

cgtttagtga accggggtct ctctggttag accagatctg agcctgggag ctctctggct 720

aactagggaa cccactgctt aagcctcaat aaagcttgcc ttgagtgctt caagtagtgt 780

gtgcccgtct gttgtgtgac tctggtaact agagatccct cagacccttt tagtcagtgt 840

ggaaaatctc tagcagtggc gcccgaacag ggacctgaaa gcgaaaggga aaccagagct 900

ctctcgacgc aggactcggc ttgctgaagc gcgcacggca agaggcgagg ggcggcgact 960

ggtgagtacg ccaaaaattt tgactagcgg aggctagaag gagagagatg ggtgcgagag 1020

cgtcagtatt aagcggggga gaattagatc gcgatgggaa aaaattcggt taaggccagg 1080

gggaaagaaa aaatataaat taaaacatat agtatgggca agcagggagc tagaacgatt 1140

cgcagttaat cctggcctgt tagaaacatc agaaggctgt agacaaatac tgggacagct 1200

acaaccatcc cttcagacag gatcagaaga acttagatca ttatataata cagtagcaac 1260

cctctattgt gtgcatcaaa ggatagagat aaaagacacc aaggaagctt tagacaagat 1320

agaggaagag caaaacaaaa gtaagaccac cgcacagcaa gcggccgctg atcttcagac 1380

ctggaggagg agatatgagg gacaattgga gaagtgaatt atataaatat aaagtagtaa 1440

aaattgaacc attaggagta gcacccacca aggcaaagag aagagtggtg cagagagaaa 1500

aaagagcagt gggaatagga gctttgttcc ttgggttctt gggagcagca ggaagcacta 1560

tgggcgcagc ctcaatgacg ctgacggtac aggccagaca attattgtct ggtatagtgc 1620

agcagcagaa caatttgctg agggctattg aggcgcaaca gcatctgttg caactcacag 1680

tctggggcat caagcagctc caggcaagaa tcctggctgt ggaaagatac ctaaaggatc 1740

aacagctcct ggggatttgg ggttgctctg gaaaactcat ttgcaccact gctgtgcctt 1800

ggaatgctag ttggagtaat aaatctctgg aacagatttg gaatcacacg acctggatgg 1860

agtgggacag agaaattaac aattacacaa gcttaataca ctccttaatt gaagaatcgc 1920

aaaaccagca agaaaagaat gaacaagaat tattggaatt agataaatgg gcaagtttgt 1980

ggaattggtt taacataaca aattggctgt ggtatataaa attattcata atgatagtag 2040

gaggcttggt aggtttaaga atagtttttg ctgtactttc tatagtgaat agagttaggc 2100

agggatattc accattatcg tttcagaccc acctcccaac cccgagggga cccgacaggc 2160

ccgaaggaat agaagaagaa ggtggagaga gagacagaga cagatccatt cgattagtga 2220

acggatctcg acggtatcgg ttaactttta aaagaaaagg ggggattggg gggtacagtg 2280

caggggaaag aatagtagac ataatagcaa cagacataca aactaaagaa ttacaaaaac 2340

aaattacaaa aattcaaaat tttatcgatc acgagactag cctcgatcga ggtcaattca 2400

cgcgagttaa taattaccag cgcgggccaa ataaataatc cgcgaggggc aggtgacgtt 2460

tgcccagcgc gcgctggtaa ttattaacct cgcgaatatt gattcgaggc cgcgattgcc 2520

gcaatcgcga ggggcaggtg acctttgccc agcgcgcgtt cgccccgccc cggacggtat 2580

cgataagctt aggagcttgg gctgcaggtc gagggcactg ggaggatgtt gagtaagatg 2640

gaaaactact gatgaccctt gcagagacag agtattagga catgtttgaa caggggccgg 2700

gcgatcagca ggtagctcta gaggatcccc gtctgtctgc acatttcgta gagcgagtgt 2760

tccgatactc taatctccct aggcaaggtt catatttgtg taggttactt attctccttt 2820

tgttgactaa gtcaataatc agaatcagca ggtttggagt cagcttggca gggatcagca 2880

gcctgggttg gaaggagggg gtataaaagc cccttcacca ggagaagccg tcacacagat 2940

ccacaagctc ctgccaccat ggtgagcaag ggcgaggagc tgttcaccgg ggtggtgccc 3000

atcctggtcg agctggacgg cgacgtaaac ggccacaagt tcagcgtgtc cggcgagggc 3060

gagggcgatg ccacctacgg caagctgacc ctgaagttca tctgcaccac cggcaagctg 3120

cccgtgccct ggcccaccct cgtgaccacc ctgacctacg gcgtgcagtg cttcagccgc 3180

taccccgacc acatgaagca gcacgacttc ttcaagtccg ccatgcccga aggctacgtc 3240

caggagcgca ccatcttctt caaggacgac ggcaactaca agacccgcgc cgaggtgaag 3300

ttcgagggcg acaccctggt gaaccgcatc gagctgaagg gcatcgactt caaggaggac 3360

ggcaacatcc tggggcacaa gctggagtac aactacaaca gccacaacgt ctatatcatg 3420

gccgacaagc agaagaacgg catcaaggtg aacttcaaga tccgccacaa catcgaggac 3480

ggcagcgtgc agctcgccga ccactaccag cagaacaccc ccatcggcga cggccccgtg 3540

ctgctgcccg acaaccacta cctgagcacc cagtccgccc tgagcaaaga ccccaacgag 3600

aagcgcgatc acatggtcct gctggagttc gtgaccgccg ccgggatcac tctcggcatg 3660

gacgagctgt acaagtaaag cggcctcgac gggcccgcgg aatttcgaca atcaacctct 3720

ggattacaaa atttgtgaaa gattgactgg tattcttaac tatgttgctc cttttacgct 3780

atgtggatac gctgctttaa tgcctttgta tcatgctatt gcttcccgta tggctttcat 3840

tttctcctcc ttgtataaat cctggttgct gtctctttat gaggagttgt ggcccgttgt 3900

caggcaacgt ggcgtggtgt gcactgtgtt tgctgacgca acccccactg gttggggcat 3960

tgccaccacc tgtcagctcc tttccgggac tttcgctttc cccctcccta ttgccacggc 4020

ggaactcatc gccgcctgcc ttgcccgctg ctggacaggg gctcggctgt tgggcactga 4080

caattccgtg gtgttgtcgg ggaagctgac gtcctttcca tggctgctcg cctgtgttgc 4140

cacctggatt ctgcgcggga cgtccttctg ctacgtccct tcggccctca atccagcgga 4200

ccttccttcc cgcggcctgc tgccggctct gcggcctctt ccgcgtcttc gccttcgccc 4260

tcagacgagt cggatctccc tttgggccgc ctccccgcct ggaattcgag ctccaccgcg 4320

gtggcggccg ctctagagtc gactccataa agtaggaaac actacacgat tccataaagt 4380

aggaaacact acaaccggtt ccataaagta ggaaacacta catcactcca taaagtagga
4440

aacactacac tcgagggggg gcccggtacc tttaagacca atgacttaca aggcagctgt
4500

agatcttagc cactttttaa aagaaaaggg gggactggaa gggctaattc actcccaacg
4560

aagacaagat ctgctttttg cttgtactgg gtctctctgg ttagaccaga tctgagcctg
4620

ggagctctct ggctaactag ggaacccact gcttaagcct caataaagct tgccttgagt
4680

gcttcaagta gtgtgtgccc gtctgttgtg tgactctggt aactagagat ccctcagacc
4740

cttttagtca gtgtggaaaa tctctagca
4769

<210> 27
<211> 29
<212> DNA
<213> Artificial Sequence

<220>
<223> Forward

<400> 27
ctagctagca tggcctctca caagctgct 29

<210> 28
<211> 29
<212> DNA
<213> Artificial Sequence

<220>
<223> Reverse

<400> 28
caagtcgact cacagcttct tcttggggc 29

<210> 29
<211> 1179
<212> DNA
<213> Artificial Sequence

<220>
<223> AGXT-RHEAM

<400> 29

atggcctctc acaagctgct ggtgaccccc cccaaggccc tgctcaagcc cctctccatc 60

cccaaccgtc tcctgctggg gcctggtcct tccaacctgc ctcctcgcat catggcagcc 120

ggggggctgc agatgatcgg gcacatgagc aaggaaatgt accagatcat ggacgagatc 180

aaggaaggca tccagtacgt gttccagacc aggaacccac tcacactggt catctccggc 240

tcgggacact gtgccctgga ggccgccctg gtcaatgtgc tggagcctgg ggactccttc 300

ctggttgggg ccaatggcat ttgggggcag cgagccgcgg acatcgggga gcgcatagga 360

gcccgagtgc acccgatgac caaggacccc ggaggccact acacactgca ggaggtggag 420

gagggcctgg cccagcacaa gccagtgctg ctgttcttaa cccacgggga gtcgtccacc 480

ggcgtgctgc agccccttga tggcttcggg gaactctgcc acaggtacaa gtgcctgctc 540

ctggtggatt cggtggcatc cctgggcggg acccccctttt acatggaccg gcaaggcatc 600

gacatcctgt actcgggctc ccagaaggcc ctgaacgccc ctccagggac ctcgctcatc 660

tccttcagtg acaaggccaa aaagaagatg tactcccgca agacgaagcc cttctccttc 720

tacctggaca tcaagtggct ggccaacttc tggggctgtg acgaccagcc caggatgtac 780

catcacacaa tccccgtcat cagcctgtac agcctgagag agagcctggc cctcattgcg 840

gaacagggcc tggagaacag ctggcgccag caccgcgagg ccgcggcgta tctgcatggg 900

cgcctgcagg cactggggct gcagctcttc gtgaaggacc cggcgctccg gcttcccaca 960

gtcaccactg tggctgtacc cgctggctat gactggagag acatcgtcag ctacgtcatg 1020

gaccacttcg acattgagat catgggtggc cttgggccct ccacggggaa ggtgctgcgg 1080

atcggcctgc tgggctgcaa tgccacccgc gagaatgtgg accgcgtgac ggaggccctg 1140

agggcggccc tgcagcactg ccccaagaag aagctgtga 1179

<210> 30
<211> 5091
<212> DNA
<213> Artificial Sequence

<220>
<223> LV.ET.AGXT-RHEAM.142-3pT

<400> 30
tggccattgc atacgttgta tccatatcat aatatgtaca tttatattgg ctcatgtcca
60

acattaccgc catgttgaca ttgattattg actagttatt aatagtaatc aattacgggg
120

tcattagttc atagcccata tatggagttc cgcgttacat aacttacggt aaatggcccg 180

cctggctgac cgcccaacga cccccgccca ttgacgtcaa taatgacgta tgttcccata 240

gtaacgccaa tagggacttt ccattgacgt caatgggtgg agtatttacg gtaaactgcc 300

cacttggcag tacatcaagt gtatcatatg ccaagtacgc cccctattga cgtcaatgac 360

ggtaaatggc ccgcctggca ttatgcccag tacatgacct tatgggactt tcctacttgg 420

cagtacatct acgtattagt catcgctatt accatggtga tgcggttttg gcagtacatc 480

aatgggcgtg gatagcggtt tgactcacgg ggatttccaa gtctccaccc cattgacgtc 540

aatgggagtt tgttttggca ccaaaatcaa cgggactttc caaaatgtcg taacaactcc 600

gccccattga cgcaaatggg cggtaggcgt gtacggtggg aggtctatat aagcagagct 660

cgtttagtga accggggtct ctctggttag accagatctg agcctgggag ctctctggct 720

aactagggaa cccactgctt aagcctcaat aaagcttgcc ttgagtgctt caagtagtgt 780

gtgcccgtct gttgtgtgac tctggtaact agagatccct cagacccttt tagtcagtgt 840

ggaaaatctc tagcagtggc gcccgaacag ggacttgaaa gcgaaaggga aaccagagga 900

gctctctcga cgcaggactc ggcttgctga agcgcgcacg gcaagaggcg aggggcggcg 960

actggtgagt acgccaaaaa ttttgactag cggaggctag aaggagagag atgggtgcga 1020

gagcgtcagt attaagcggg ggagaattag atcgcgatgg gaaaaaattc ggttaaggcc 1080

agggggaaag aaaaaatata aattaaaaca tatagtatgg gcaagcaggg agctagaacg 1140

attcgcagtt aatcctggcc tgttagaaac atcagaaggc tgtagacaaa tactgggaca 1200

gctacaacca tcccttcaga caggatcaga agaacttaga tcattatata atacagtagc 1260

aaccctctat tgtgtgcatc aaaggataga gataaaagac accaaggaag ctttagacaa 1320

gatagaggaa gagcaaaaca aaagtaagac caccgcacag caagcggccg ctgatcttca 1380

gacctggagg aggagatatg agggacaatt ggagaagtga attatataaa tataaagtag 1440

taaaaattga accattagga gtagcaccca ccaaggcaaa gagaagagtg gtgcagagag 1500

aaaaaagagc agtgggaata ggagctttgt tccttgggtt cttgggagca gcaggaagca 1560

ctatgggcgc agcgtcaatg acgctgacgg tacaggccag acaattattg tctggtatag 1620

tgcagcagca gaacaatttg ctgagggcta ttgaggcgca acagcatctg ttgcaactca 1680

cagtctgggg catcaagcag ctccaggcaa gaatcctggc tgtggaaaga tacctaaagg 1740

atcaacagct cctggggatt tggggttgct ctggaaaact catttgcacc actgctgtgc 1800

cttggaatgc tagttggagt aataaatctc tggaacagat ttggaatcac acgacctgga 1860

tggagtggga cagagaaatt aacaattaca caagcttaat acactcctta attgaagaat 1920

cgcaaaacca gcaagaaaag aatgaacaag aattattgga attagataaa tgggcaagtt 1980

tgtggaattg gtttaacata acaaattggc tgtggtatat aaaattattc ataatgatag 2040

taggaggctt ggtaggttta agaatagttt ttgctgtact ttctatagtg aatagagtta 2100

ggcagggata ttcaccatta tcgtttcaga cccacctccc aaccccgagg ggacccgaca 2160

ggcccgaagg aatagaagaa gaaggtggag agagagacag agacagatcc attcgattag 2220

tgaacggatc tcgacggtat cggttaactt ttaaaagaaa aggggggatt ggggggtaca 2280

gtgcagggga aagaatagta gacataatag caacagacat acaaactaaa gaattacaaa 2340

aacaaattac aaaaattcaa aattttatcg atcacgagac tagcctcgag cacgcgagtt 2400

aataattacc agcgcgggcc aaataaataa tccgcgaggg gcaggtgacg tttgcccagc 2460

gcgcgctggt aattattaac ctcgcgaata ttgattcgag gccgcgattg ccgcaatcgc 2520

gaggggcagg tgacctttgc ccagcgcgcg ttcgccccgc cccggacggt atcgataagc 2580

ttaggagctt gggctgcagg tcgagggcac tgggaggatg ttgagtaaga tggaaaacta 2640

ctgatgaccc ttgcagagac agagtattag gacatgtttg aacaggggcc gggcgatcag 2700

caggtagctc tagaggatcc ccgtctgtct gcacatttcg tagagcgagt gttccgatac 2760

tctaatctcc ctaggcaagg ttcatatttg tgtaggttac ttattctcct tttgttgact 2820

aagtcaataa tcagaatcag caggtttgga gtcagcttgg cagggatcag cagcctgggt 2880

tggaaggagg gggtataaaa gccccttcac caggagaagc cgtcacacag atccacaagc 2940

tcctggctag ctagcatggc ctctcacaag ctgctggtga cccccccaa ggccctgctc 3000

aagcccctct ccatccccaa ccgtctcctg ctggggcctg gtccttccaa cctgcctcct 3060

cgcatcatgg cagccggggg gctgcagatg atcgggcaca tgagcaagga aatgtaccag 3120

atcatggacg agatcaagga aggcatccag tacgtgttcc agaccaggaa cccactcaca 3180

ctggtcatct ccggctcggg acactgtgcc ctggaggccg ccctggtcaa tgtgctggag 3240

cctggggact ccttcctggt tggggccaat ggcatttggg ggcagcgagc cgcggacatc 3300

ggggagcgca taggagcccg agtgcacccg atgaccaagg accccggagg ccactacaca 3360

ctgcaggagg tggaggaggg cctggcccag cacaagccag tgctgctgtt cttaacccac 3420

ggggagtcgt ccaccggcgt gctgcagccc cttgatggct tcggggaact ctgccacagg 3480

tacaagtgcc tgctcctggt ggattcggtg gcatccctgg gcgggacccc cctttacatg 3540

gaccggcaag gcatcgacat cctgtactcg ggctcccaga aggccctgaa cgcccctcca 3600

gggacctcgc tcatctcctt cagtgacaag gccaaaaaga agatgtactc ccgcaagacg 3660

aagcccttct ccttctacct ggacatcaag tggctggcca acttctgggg ctgtgacgac 3720

cagcccagga tgtaccatca cacaatcccc gtcatcagcc tgtacagcct gagagagagc 3780

ctggccctca ttgcggaaca gggcctggag aacagctggc gccagcaccg cgaggccgcg 3840

gcgtatctgc atgggcgcct gcaggcactg gggctgcagc tcttcgtgaa ggacccggcg 3900

ctccggcttc ccacagtcac cactgtggct gtacccgctg gctatgactg gagagacatc 3960

gtcagctacg tcatggacca cttcgacatt gagatcatgg gtggccttgg gccctccacg 4020

gggaaggtgc tgcggatcgg cctgctgggc tgcaatgcca cccgcgagaa tgtggaccgc 4080

gtgacggagg ccctgagggc ggccctgcag cactgcccca agaagaagct gtgagtcgac 4140

aatcaacctc tggattacaa aatttgtgaa agattgactg gtattcttaa ctatgttgct 4200

ccttttacgc tatgtggata cgctgcttta atgcctttgt atcatgctat tgcttcccgt 4260

atggctttca ttttctcctc cttgtataaa tcctggttgc tgtctcttta tgaggagttg 4320

tggcccgttg tcaggcaacg tggcgtggtg tgcactgtgt ttgctgacgc aacccccact 4380

ggttggggca ttgccaccac ctgtcagctc ctttccggga ctttcgcttt ccccctccct 4440

attgccacgg cggaactcat cgccgcctgc cttgcccgct gctggacagg ggctcggctg 4500

ttgggcactg acaattccgt ggtgttgtcg gggaaatcat cgtcctttcc ttggctgctc 4560

gcctgtgttg ccacctggat tctgcgcggg acgtccttct gctacgtccc ttcggccctc 4620

aatccagcgg accttccttc ccgcggcctg ctgccggctc tagataatcc ataaagtagg 4680

aaacactaca cgattccata aagtaggaaa cactacaacc ggttccataa agtaggaaac 4740

actacatcac tccataaagt aggaaacact acacccggtc gagctcggta cctttaagac 4800

caatgactta caaggcagct gtagatctta gccacttttt aaaagaaaag gggggactgg 4860

aagggctaat tcactcccaa cgaagacaag atctgctttt tgcttgtact gggtctctct 4920

ggttagacca gatctgagcc tgggagctct ctggctaact agggaaccca ctgcttaagc 4980

ctcaataaag cttgccttga gtgcttcaag tagtgtgtgc ccgtctgttg tgtgactctg 5040

gtaactagag atccctcaga cccttttagt cagtgtggaa aatctctagc a 5091

<210> 31
<211> 20
<212> DNA
<213> Artificial Sequence

<220>
<223> Forward

<400> 31
caggactcgg cttgctgaag 20

<210> 32
<211> 20
<212> DNA
<213> Artificial Sequence

<220>
<223> Reverse

<400> 32
tcccccgctt aatactgacg 20

<210> 33
<211> 19
<212> DNA
<213> Artificial Sequence

<220>
<223> probe

<400> 33

cgcacggcaa gaggcgagg 19

<210> 34
<211> 21
<212> DNA
<213> Artificial Sequence

<220>
<223> Forward

<400> 34
gctgtcatct cttgtgggct g 21

<210> 35
<211> 21
<212> DNA
<213> Artificial Sequence

<220>
<223> Reverse

<400> 35
actcatggga gctgctggtt c 21

<210> 36
<211> 26
<212> DNA
<213> Artificial Sequence

<220>
<223> probe

<400> 36
cctgtcatgc ccacacaaat ctctcc 26

<210> 37
<211> 20
<212> DNA
<213> Artificial Sequence

<220>
<223> Forward

<400> 37
caggactcgg cttgctgaag 20

<210> 38
<211> 20
<212> DNA

<213> Artificial Sequence

<220>
<223> Reverse

<400> 38
tcccccgctt aatactgacg 20

<210> 39
<211> 19
<212> DNA
<213> Artificial Sequence

<220>
<223> probe

<400> 39
cgcacggcaa gaggcgagg 19

<210> 40
<211> 21
<212> DNA
<213> Artificial Sequence

<220>
<223> forward

<400> 40
aaaacgagca gtgacgtgag c 21

<210> 41
<211> 20
<212> DNA
<213> Artificial Sequence

<220>
<223> Reverse

<400> 41
ttcagtcatg ctgctagcgc 20

<210> 42
<211> 25
<212> DNA
<213> Artificial Sequence

<220>
<223> probe

<400> 42
tgcacggaag cgtctcgtct cagtc 25

<210> 43
<211> 20
<212> DNA
<213> Artificial Sequence

<220>
<223> Reverse

<400> 43
gtcttgcggg agtacatctt 20

<210> 44
<211> 20
<212> DNA
<213> Artificial Sequence

<220>
<223> Forward

<400> 44
caaggcatcg acatcctgta 20

<210> 45
<211> 20
<212> DNA
<213> Artificial Sequence

<220>
<223> Reverse

<400> 45
gatgggaatt ccaggagtca 20

<210> 46
<211> 19
<212> DNA
<213> Artificial Sequence

<220>
<223> Forward

<400> 46
gggagaatca tggaccaga 19

<210> 47
<211> 20

<212> DNA
<213> Artificial Sequence

<220>
<223> Reverse

<400> 47
ctaaggccaa ccgtgaaaag 20

<210> 48
<211> 20
<212> DNA
<213> Artificial Sequence

<220>
<223> Forward

<400> 48
accagaggca tacagggaca 20

<210> 49
<211> 22
<212> DNA
<213> Artificial Sequence

<220>
<223> Reverse

<400> 49
gacaaagcca gtctccttct ac 22

<210> 50
<211> 22
<212> DNA
<213> Artificial Sequence

<220>
<223> Forward

<400> 50
gtgacaggtg tggtatgatg aa 22

<210> 51
<211> 720
<212> DNA
<213> Artificial Sequence

<220>
<223> eGFP sequence

```
<400> 51
tggtgagcaa gggcgaggag ctgttcaccg gggtggtgcc catcctggtc gagctggacg      60

gcgacgtaaa cggccacaag ttcagcgtgt ccggcgaggg cgagggcgat gccacctacg     120

gcaagctgac cctgaagttc atctgcacca ccggcaagct gcccgtgccc tggcccaccc     180

tcgtgaccac cctgacctac ggcgtgcagt gcttcagccg ctaccccgac cacatgaagc     240

agcacgactt cttcaagtcc gccatgcccg aaggctacgt ccaggagcgc accatcttct     300

tcaaggacga cggcaactac aagacccgcg ccgaggtgaa gttcgagggc gacaccctgg     360

tgaaccgcat cgagctgaag ggcatcgact tcaaggagga cggcaacatc ctggggcaca     420

agctggagta caactacaac agccacaacg tctatatcat ggccgacaag cagaagaacg     480

gcatcaaggt gaacttcaag atccgccaca acatcgagga cggcagcgtg cagctcgccg     540

accactacca gcagaacacc cccatcggcg acggccccgt gctgctgccc gacaaccact     600

acctgagcac ccagtccgcc ctgagcaaag accccaacga gaagcgcgat cacatggtcc     660

tgctggagtt cgtgaccgcc gccgggatca ctctcggcat ggacgagctg tacaagtaaa     720
```